# EUROPEAN PATENT APPLICATION

(11) **EP 4 019 638 A1**
(43) Date of publication of application: **29.06.2022**
(21) Application number: 20216525.4
(22) Date of filing: 22.12.2020
(51) Int. Cl.: C12N 15/82, A01H 4/00

(54) **PROMOTING REGENERATION AND TRANSFORMATION IN BETA VULGARIS**

(71) Applicant: KWS SAAT SE & Co. KGaA, 37574 Einbeck (DE); Stichting Wageningen Research, 6708 PB Wageningen (NL)
(72) Inventor: KONG, Jixiang, 37574 Einbeck (DE); MARTIN-ORTIGOSA, Susana, 37574 Einbeck (DE); PACHECO VILLALOBOS, David, 37574 Einbeck (DE); KASTNER, Christine, 37574 Einbeck (DE); LUDEWIG, Frank, 37574 Einbeck (DE)
(74) Representative: Heubeck, Christian

(57) **Abstract**

The present invention relates to the technical field of plant regeneration and induction of somatic embryogenesis, preferably in sugar beet and corn.

## Description

### TECHNICAL FIELD

The present invention relates to the technical field of plant regeneration and induction of somatic embryogenesis, preferably in sugar beet and corn.

### TECHNICAL BACKGROUND

The ability to regenerate whole plants via tissue culture is a well-described and much applied phenomenon. The most commonly used methods for plant regeneration from somatic cells involve culturing explants on medium containing growth regulators to induce callus formation, followed by organogenesis or embryogenesis from the dedifferentiated callus. Dissecting the phenomenon of *in vitro* regeneration at the molecular-genetic level has been difficult due to the length of the regeneration process and the many developmental events that take place as explants dedifferentiate and then redifferentiate new tissues and organs. Mutant screens and functional studies have been particularly helpful in identifying proteins that control developmental pathways.

Among the proteins controlling regeneration are those that induce somatic embryogenesis from competent somatic cells. The BABY BOOM (BBM) AP2/ERF domain protein is a seed and root-meristem expressed transcription factor that was identified as marker for embryo development in *Brassica napus* microspore-derived embryo cultures (Boutilier, Kim, et al. ("Ectopic expression of BABY BOOM triggers a conversion from vegetative to embryonic growth." The Plant Cell 14.8 (2002): 1737-1749.) The authors describe that in *in vitro* induction of embryo development from immature pollen grains of *Brassica napus* (microspore embryogenesis) *BABY BOOM* (*BBM*) showing similarity to the AP2/ERF family of transcription factors and expressed preferentially in developing embryos and seeds, is involved. Ectopic expression of *BBM* in Arabidopsis and Brassica led to the spontaneous formation of somatic embryos and cotyledon-like structures on transgenic seedlings. Ectopic *BBM* expression induced however additional pleiotropic phenotypes, including neoplastic growth and alterations in leaf and flower morphology.

Florez, Sergio L., et al. ("Enhanced somatic embryogenesis in Theobroma cacao using the homologous BABY BOOM transcription factor." BMC plant biology 15.1 (2015): 121.) describe the use of the transcription factor *Baby Boom* (*BBM*) derived from *Theobroma cacao* to promote the transition of somatic cacao cells from the vegetative to embryonic state. While transient ectopic expression of TcBBM provided only moderate enhancements in embryogenic potential, constitutive overexpression dramatically increased somatic embryogenesis proliferation but also appeared to inhibit subsequent development.

Srinivasan, Chinnathambi, et al. ("Heterologous expression of the BABY BOOM AP2/ERF transcription factor enhances the regeneration capacity of tobacco (Nicotiana tabacum L.)." Planta 225.2 (2007): 341.) examined the effect of ectopic *BBM* expression on the development and regenerative capacity of tobacco (*Nicotiana tabacum* L.) through heterologous expression of *Arabidopsis* and *B*. *napus BBM* genes. *35S::BBM* tobacco lines exhibited a number of phenotypes including callus formation, leaf rumpling, and sterility, but they did not undergo spontaneous somatic embryogenesis. *35S::BBM* plants with severe ectopic expression phenotypes could not be assessed for enhanced regeneration at the seedling stage due to complete male and female sterility of the primary transformants. Therefore fertile *BBM* ectopic expression lines with strong misexpression phenotypes were generated by expressing a steroid-inducible, post-translationally controlled BBM fusion protein (BBM:GR) under the control of a 35S promoter. These lines exhibited spontaneous shoot and root formation upon the application of DEX, while somatic embryogenesis could be induced from in-vitro germinated seedling hypocotyls cultured on media supplemented with cytokinin.

WO2011003850A1 describe a general method for providing fertile plants via induction of BBM during transformation. Recovery of fertile plants however requires phytohormones like auxins or cytokinins.

So far, induction of somatic embryogenesis through BBM transformation and have only been described for *Capsicum annuum, Nicotiana tabacum, Arabidopsis thaliana, Brassica napus, Theobroma cacao, Populus tomentosa,* but never for *Beta vulgaris* which belongs to one of the economically most relevant crops. Somatic embryogenesis based transformation was not possible before in *Beta vulgaris* and has a positive impact on the overall timeline for biotech processes in sugar beet.

### Summary of the invention

In the present invention it was found, that BBM is capable to promote somatic embryogenesis of *Beta vulgaris and* Zea *mays* (corn). Somatic embryogenesis is achieved in a hormone independent way resulting in fertile plants. The invention describes nucleotides and methods for the induction of somatic embryogenesis and plant regeneration in *Beta vulgaris* and Zea *mays.* The present invention leads to fertile plants without pleiotropic phenotypes, enabling transformation of recalcitrant genotypes, reduction of somaclonal variation and a high frequency of co-transformation. In general, the time needed for plant regeneration is significantly shortened with this method and the quality of the obtained plants is also clearly improved in comparison to other regeneration methods. The high quality of the obtained plants can be of specific value for root features e.g. weight, sucrose content as well as for leaf parameters like photosynthesis.

In particular, the invention is about a genotype independent callus-based regeneration method by using inducible or constitutive expression of BBM resulting in fertile plants. The transformation rate is dramatically increased. The regeneration of plants is hormone-independent supporting somatic embryogenesis and avoiding organogenesis (e.g. shoot formation without roots). The time needed for sugar beet transformation is significantly shortened with this invention.

Thus, according to a first aspect, the invention provides a method of promoting somatic embryogenesis or organogenesis of *Beta vulgaris,* comprising the following steps:
(A1)
   (a) inducing callus formation from at least one *Beta vulgaris* plant cell, and
   (b) introducing into the at least one plant cell to be used in step (a) or into the at least one cell of the callus obtained in step (a) an expression cassette comprising a coding nucleotide sequence selected from
      (i) a nucleotide sequence of SEQ ID NO: 1, or a sequence which is at least 80% identical to the sequence of SEQ ID NO: 1; and
      (ii) a nucleotide sequence encoding a polypeptide having the amino acid sequence of SEQ ID NO: 2, or a sequence which is at least 80% identical to the sequence of SEQ ID NO: 2,
      wherein the nucleotide sequence is operably linked to a heterologous constitutive regulatory element or a heterologous inducible regulatory element; or
(A2) providing an explant which is from a tissue of a *Beta vulgaris* plant and comprises at least one cell comprising an expression cassette comprising a coding nucleotide sequence selected from
   (i) a nucleotide sequence of SEQ ID NO: 1, or a sequence which is at least 80% identical to the sequence of SEQ ID NO: 1; and
   (ii) a nucleotide sequence encoding a polypeptide having the amino acid sequence of SEQ ID NO: 2, or an amino acid sequence which is at least 80% identical to the sequence of SEQ ID NO: 2,
   wherein the nucleotide sequence is operably linked to a heterologous constitutive regulatory element; or
(A3) providing an explant which is from a tissue of a *Beta vulgaris* plant and comprises at least one cell comprising an expression cassette comprising a coding nucleotide sequence selected from
   (i) a nucleotide sequence of SEQ ID NO: 1, or a sequence which is at least 80% identical to the sequence of SEQ ID NO: 1; and
   (ii) a nucleotide sequence encoding a polypeptide having the amino acid sequence of SEQ ID NO: 2, or an amino acid sequence which is at least 80% identical to the sequence of SEQ ID NO: 2,
   wherein the nucleotide sequence is operably linked to a heterologous inducible regulatory element; and
(B1) cultivating the callus obtained in step (A1) under conditions promoting growth of embryos and/or shoots out of the callus, wherein in the callus the polypeptide is expressed from the expression cassette constitutively or upon induction of the heterologous inducible expression system; or
(B2) cultivating the explant of step (A2) under conditions promoting growth of embryos out of the explant, wherein in the explant the polypeptide is expressed from the expression cassette constitutively; or
(B3) cultivating the explant of step (A3) under conditions promoting growth of shoots out of the explant, wherein in the explant the polypeptide is expressed from the expression cassette upon induction of the heterologous inducible expression system.

In the method of the present invention, *Beta vulgaris* cells or explants are used which have been modified so that they are able to express a polypeptide derived from *Brassica napus* BBM comprising the amino acid sequence of SEQ ID NO:2 or an amino acid sequence at least 80% identical thereto. For this purpose, either an expression cassette containing a respective coding nucleic acid sequence is inserted directly into the cells using the method of variant (A1) according to the invention. Alternatively, variants (A2) and (A3) use an explant, which is from a tissue of a *Beta vulgaris* plant comprising at least one cell comprising the expression cassette.

The plural *"plant cells"* as used herein must not be understood in that a minimum number of plant cells would be required. In principle, only one plant cell may be sufficient.

The coding nucleotide sequence comprises
(i) a nucleotide sequence of SEQ ID NO: 1, or a sequence which is at least 80%, preferably at least 85%, at least 90%, more preferably at least 95%, at least 98% or at least 99% identical to the sequence of SEQ ID NO: 1; or
(ii) a nucleotide sequence encoding a polypeptide having the amino acid sequence of SEQ ID NO: 2, or an amino acid sequence which is at least 80%, preferably at least 85%, at least 90%, more preferably at least 95%, at least 98% or at least 99% identical to the sequence of SEQ ID NO: 2.

The above polynucleotides and polypeptides are referred to as *"BnBBM-derived nucleic acids"* or *"BnBBM-derived polypeptides"* for simplicity. However, this does not mean that the compounds are obtained from BnBBM, but only that their sequences are derived from the sequence of BnBBM. The way in which the polynucleotides and polypeptides are obtained is basically not restricted.

For the purpose of this invention, the *"sequence identity"* of two related nucleotide or amino acid sequences, expressed as a percentage, refers to the number of positions in the two optimally aligned sequences, which have identical residues (x100) divided by the number of positions compared. A gap, i.e. a position in an alignment where a residue is present in one sequence but not in the other, is regarded as a position with non-identical residues. The alignment of the two sequences is performed by the Needleman and Wunsch algorithm (Needleman and Wunsch 1970). The computer-assisted sequence alignment above, can be conveniently performed using standard software program such as program NEEDLE as implemented in The European Molecular Biology Open Software Suite (EMBOSS), e.g. version 6.3.1.2 (Trends in Genetics 16 (6), 276 (2000)), with its default parameter, e.g. for proteins matrix = EBLOSUM62, gapopen = 10.0 and gapextend = 0.5.

The coding nucleotide sequence is operably linked to a heterologous constitutive regulatory element or a heterologous inducible regulatory element. The expression *"operably linked"* means that said elements of the chimeric gene are linked to one another in such a way that their function is coordinated and allows expression of the coding sequence, i.e. they are functionally linked. By way of example, a promoter is functionally linked to another nucleotide sequence when it is capable of ensuring transcription and ultimately expression of said other nucleotide sequence. Two proteins encoding nucleotide sequences are functionally or operably linked to each other if they are connected in such a way that a fusion protein of first and second protein or polypeptide can be formed.

The heterologous constitutive regulatory element is preferably a constitutive promoter. The heterologous inducible regulatory element can be an inducible promoter or an inducible expression system.

The term *"inducible promoter"* refers to promoters that selectively express a coding sequence or functional RNA in response to the presence of an endogenous or exogenous stimulus, for example by chemical compounds (chemical inducers) or in response to environmental, hormonal, chemical, and/or developmental signals. Inducible promoters include, for example, promoters induced by light, heat, stress, salt stress, osmotic stress, phytohormones, or chemicals such as ethanol, abscisic acid (ABA), jasmonate, salicylic acid, or safeners.

Promoters useful for targeted expression in trangenesis are reviewed in Potenza at al., 2004. Some abiotic stress promoters are the *Arabidospsis thaliana* or *Oryza sativa* DREB genes promoters (Dubouzet et al., 2003; Lee et al., 2004; Pellegrineschi at al., 2004); the *Oryza sativa* SISAP1, CDPK7 or WSI gene promoters (Mukhopadhyay et al., 2004; Saijo et al., 2000; Takahashi at al., 1994) the *A. thaliana* rd29 gene promoters (Yamaguchi-Shinozaki and Shinozaki 1993). Some plant heat inducible promoters may also be used hsp18.2 or hsp101 from *A. thaliana* (Yoshida at al., 1995; Young at al., 2005), hsp17.6 or hsp17.3 from *Glycine max* (Severin and Schoffl, 1990; Saidi at al., 2005). DNA microarrays have been used to identify stress regulated sequences (Rabbani at al., 2003; EP 1 452 596; WO 02/16655) The signalisation pathway of the response to stress includes abscisic acid signalisation so ABA-inducible promoters may also be powerful stress-inducible promoters, such as the *Horgum vulgare* A22 and hva1 promoters (Shen at al., 1993; Straub et al., 1994), Zea *maize* rab 17, DBF1 and DBF2 (Villardel et al., 1990; Kizis and Pages, 2002), *Arabidopsis thaliana* ABF3 (Genbank accession AK175851), and *Oryza sativa* rab21 (Mundy and Chua, 1988).

Some examples of couples of chemically inducible expression systems and chemical inducer used in plants are, the alcA promoter from *A. nidulans,* inducible by the Ethanol (Roslan at al., 2001) or the ecdysone receptor from *C. fumiferana,* inducible by an ecdysone agonist (Koo at al., 2004).

In another embodiment, expression of the polypeptide from the coding nucleotide sequence is indirectly induced by a chemical. As an illustration, one can use the GVG gene, which codes for a modified rat glucocorticoid responsive transcription factor that remains in the plant cytosol as a complex. On dexamethasone application, this complex dissociates such that the GVG protein enters the nucleus and binds to the target DNA sequences (UAS). Transcription from the UAS promoter allows the production of the polypeptide. This is considered as a dexamethasone inducible (although indirectly) promoter used to control the polypeptide expression (Aoyama and Chua (1997)). In this case, application of dexamethosone will induce both the expression of the polypeptide and its activity. Preferably, the inducible expression system is selected from an ecdysone or a dexamethasone based expression system.

A gene is said to be expressed when it leads to the formation of an expression product. An expression product denotes an intermediate or end product arising from the transcription and optionally translation of the nucleic acid, DNA or RNA, coding for such product, e. g. the second nucleic acid described herein. During the transcription process, a DNA sequence under control of regulatory regions, particularly the promoter, is transcribed into an RNA molecule. An RNA molecule may either itself form an expression product or be an intermediate product when it is capable of being translated into a peptide or protein. A gene is said to encode an RNA molecule as expression product when the RNA as the end product of the expression of the gene is, e.g., capable of interacting with another nucleic acid or protein. Examples of RNA expression products include inhibitory RNA such as e.g. sense RNA (co-suppression), antisense RNA, ribozymes, miRNA or siRNA, mRNA, rRNA and tRNA. A gene is said to encode a protein as expression product when the end product of the expression of the gene is a protein or peptide.

A nucleic acid (molecule) or nucleotide (sequence) or polynucleotide, as used herein, refers to both DNA and RNA. DNA also includes cDNA and genomic DNA. A nucleic acid molecule can be single- or double-stranded, and can be synthesized chemically or produced by biological expression *in vitro* or even *in vivo.*

It will be clear that whenever nucleotide sequences of RNA molecules are defined by reference to nucleotide sequence of corresponding DNA molecules, the thymine (T) in the nucleotide sequence should be replaced by uracil (U). Whether reference is made to RNA or DNA molecules will be clear from the context of the application.

As used herein *"comprising"* or the like is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structurally defined may comprise additional DNA regions etc.

Plant cells suitable for inducing callus tissue include embryonic plant cells and somatic plant cells. The way in which these plant cells are provided is not important for the method according to the present invention. Plant cells can be used either in isolated form or as part of a plant tissue. For example, embryonic or somatic plant cells can be provided from an explant isolated from a plant. Either the cells are isolated from the explant or the explant is directly used for the induction of callus tissue. Which part of a plant is eligible for obtaining an explant depends on the particular plant species. Generally, suitable plant cells can be obtained from hypocotyl, shoot, leaves, buds, flowers and roots of a plant. Preferably, an explant or a part thereof isolated from a plant is used in the method of the invention.

For inducing callus formation, the plant cells are incubated in a medium. In principle, any culture medium known in the art can be used, in particular a medium commonly used for inducing callus formation. Depending on the plant in question, the composition of the medium may vary. In principle, several types of basal salt mixtures can be added to the medium, but preferably, the medium comprises modified Murashige and Skoog (MS) medium, White's medium, or woody plant medium, most preferably MS medium. Previous studies indicate that callus induction is facilitated in the presence of appropriate amounts and concentrations of auxins and cytokinins alone or in combination with each other in MS medium. According to the invention, these components can also be added preferentially to the culture medium. Exemplary auxins include naphthalene acetic acid (NAA), indole-3-acetic acid (IAA) and indole-3-butyric acid (IBA). Exemplary cytokinins include 6-Benzylaminopurine (BAP) and 6-furfurylamino-purine (kinetin).

In step (A1)(b), an expression cassette comprising a coding nucleotide sequence is introduced into the at least one plant cell to be used in step (a) or into the at least one cell of the callus obtained in step (a). Step (b) of introducing into a plant cell the expression cassette comprising a coding nucleotide sequence may result in a stable integration thereof into the genome of the plant cell or a progeny cell thereof. Alternatively, introducing into a plant cell the expression cassette may result in a transient occurrence of the encoded polypeptide in the plant cell or in a progeny cell thereof. In terms of the invention, *"transient transformation"* means that the inserted sequence is not (stably) integrated into the genome of the plant cell. According to the invention, a stable integration into the genome is preferred.

Introducing the expression cassette can be carried out by any means. A number of methods is available to transfer nucleic acids of interest into plant cells. An exemplary vector mediated method is Agrobacterium-mediated transformation, as described, for example, by Lindsay & Gallois, 1990, Journal of Experimental Botany, and Kischenko et al., 2005, Cell Biology International for sugar beet, or by Ishida et al., 2007, ("Agrobacterium-mediated transformation of maize." Nature protocols, 2(7), 1614-1621) for corn. Other suitable techniques include particle bombardment and electroporation.

According to variants (A2) and (A3) of the method of the invention, an explant is used, which is from a tissue of a *Beta vulgaris* plant comprising at least one cell comprising the above described expression cassette. Preferably, the expression cassette is stably integrated into the genome of the at least one plant cell or in a progeny cell thereof. The explant can in principle be obtained in any way, for example as described above for variant (A1). Preferably, the explant is obtained from leaf tissue, hypocotyl tissue, floral tissue, shoot tissue, shoot apical meristem or root tip tissue.

In step (B1), (B2) or (B3), cultivation takes place under conditions in which the polypeptide is expressed. Cultivating is preferably carried out in a medium free of plant hormones. The term *"plant hormone"* is to be understood herein as a chemical that influences the growth and development of plant cells and tissues. Plant growth hormones comprise chemicals from the following five groups: auxins, cytokinins, gibberellins, abscisic acid (ABA) and ethylene. In addition to the five main groups, two other classes of chemical are often regarded as plant growth regulators: brassinosteroids and polyamines. For the induction of regeneration in plant tissues, a combination of one or more cytokinins and one or more auxins is usually employed. According to the invention, however, plant hormones are preferably not used in the culturing steps (B1), (B2), and (B3). It was found, that in the absence of plant hormones in the medium, negative side-effects on plant development (e.g. somaclonal variation) are reduced. Hence, the complexity of adjusting medium components to different genotypes is also reduced.

Additionally, it was found that the absence of plant hormones in steps (B1) and (B2) promotes the formation of somatic embryos instead of shoots. Thus, in a preferred embodiment, embryos are directly grown from the callus tissue in step (B1) or (B2). Accordingly, a tedious rooting procedure is avoided and hence the time to go to the green house is shortened.

The enhanced somatic embryo formation by using BnBBM in a hormone free transformation - leading to plant development without obvious phenotypes - allows fast validation of trait candidate genes especially for root traits and root tissue specific promoters. Furthermore, it can also be combined with co-delivery of other genetic elements and the application of the genome editing technology.

A remarkable time saving is the second improvement for plant transformation. In the described approach T0 plants can be directly used for different kind of analyses, whereas normally T1 plants are used in conventional transformation methods for such analyses. This is necessary since the conventional T0 plants produced via organogenesis have an obvious phenotype with not forming proper tap roots, the specific storage organ of sugar beet.

Due to the usage of the T0 plants in the described invention up to 2.5 years can be saved for the above-mentioned analyses processes.

Due to the increased regenerative capacities of plant material with inducible as well as constitutive expression of a BnBBM-derived polypeptide as described above, also the efficiency of co-delivery of other genetic elements (e.g. genetic engineering components) is enhanced. More transgenic or modified plants can be obtained and otherwise non-transformable genotypes can be transformed or edited. Thus, according to another aspect of the invention, the beneficial effect of the BnBBM-derived polypeptide as described above can be exploited in methods of producing transgenic plants as well as in methods for producing genetically modified/edited plants. It was found, that in recalcitrant plant species or plant genotypes, transformation efficiency can be improved by using the BnBBM-derived polypeptide as described above. The increased regenerative capacities of plant tissue with inducible as well as constitutive expression of the BnBBM-derived polypeptide leads also to a more efficient co-transformation rate which is for example important for the delivery of genome editing components. This has been successfully demonstrated by obtaining SDN 1 events. The regeneration of plants from modified plant cells that have been transformed or gene edited and possibly have a modified genome is significantly improved when a BnBBM-derived polypeptide as defined herein is present in the step of cultivating callus tissue. Further, the approach of the invention enables a remarkable time saving which is particularly pronounced when creating edited *Beta vulgaris* hybrids. Up to 6-7 years may be saved, since the editing event can be produced directly within the two parental lines of the final hybrid, instead of introducing the necessary modification in an amenable genotype for transformation first and afterwards bringing it to the parental lines of a specific hybrid via time consuming crossing and breeding steps.
According to this embodiment, the method further comprises a step of
(C1) introducing at least one nucleotide sequence of interest into the at least one plant cell or a predecessor thereof to be used in step (A1)(a), into at least one cell of the callus obtained in step (A1)(a) which itself or a progeny thereof is then to be used in step (A1)(b) or has been used in step (A1)(b), or into the at least one plant cell of step (A2) or (A3), and/or
(C2) modifying the genome of the at least one plant cell or a predecessor thereof to be used in step (A1)(a), of the at least one cell of the callus obtained in step (A1)(a) which itself or a progeny thereof is then to be used in step (A1)(b) or has been used in step (A1)(b), or into the at least one plant cell of step (A2) or (A3) by introducing into said cell a single stranded DNA break (SSB) inducing enzyme or a double stranded DNA break (DSB) inducing enzyme which preferably recognizes a predetermined site in the genome of said cell, and optionally a repair nucleic acid molecule, or a single stranded DNA break (SSB) inducing enzyme which preferably recognizes a predetermined site in the genome of said cell and is fused to a base editor enzyme,
   wherein the modification of said genome is selected from
   I. a replacement of at least one nucleotide;
   II. a deletion of at least one nucleotide;
   III. an insertion of at least one nucleotide; or
   IV. any combination of I. - III.

Step (C1) of introducing the at least one nucleotide sequence of interest can be performed using any suitable method commonly known in the art. A number of methods is available to transfer nucleic acids of interest into plant cells. An exemplary vector mediated method is Agrobacterium-mediated transformation, as described, for example, by Lindsay & Gallois, 1990, Journal of Experimental Botany, and Kischenko et al., 2005, Cell Biology International for sugar beet, or by Ishida et al., 2007, ("Agrobacterium-mediated transformation of maize." Nature protocols, 2(7), 1614-1621) for corn. Other suitable techniques include particle bombardment and electroporation.

The nucleotide sequence of interest according to the invention may be a DNA or RNA sequence, e.g. mRNA, siRNA, miRNA etc. More particularly, the nucleotide sequence of interest encodes at least one phenotypic trait. Preferably, the phenotypic trait conferred by the DNA or RNA can be selected from the group consisting of resistance/tolerance to biotic stress, including pathogen resistance/tolerance, wherein the pathogen can be a virus, bacterial, fungal or animal pathogen, resistance/tolerance to abiotic stress including chilling resistance/tolerance, drought stress resistance/tolerance, osmotic resistance/tolerance, heat stress resistance/tolerance, cold or frost stress resistance/tolerance, oxidative stress resistance/tolerance, heavy metal stress resistance/tolerance, salt stress or water logging resistance/tolerance, lodging resistance/tolerance, shattering resistance/tolerance, or resistance/tolerance against one or more herbicides like glyphosate, glufosinate, 2,4-D, Dicamba, ALS inhibitors et cetera. The at least one phenotypic trait of interest can also be selected from the group consisting of the modification of a further agronomic trait of interest including yield increase, flowering time modification, seed color modification, endosperm composition modification, nutritional content modification or metabolic engineering of a pathway of interest.

According to one embodiment of the invention, step (C1) of introducing the at least one nucleotide sequence of interest yields in transient transformation of the plant cell. In another embodiment, a stable transformation is effected, wherein the nucleotide sequence of interest in step (C1) is inserted into the genome of the plant cell.

In step (C2), modifying the genome of the plant cell can be accomplished by means of a single stranded DNA break (SSB) or double stranded DNA break (DSB) inducing enzyme or a base editor enzyme which preferably recognizes a predetermined site in the genome of said cell.

As used herein, a *"double-stranded DNA break inducing enzyme"* or *"DSBI enzyme"* is an enzyme capable of inducing a double-stranded DNA break at a particular nucleotide sequence, called the *"recognition site".* Accordingly, a *"single-stranded DNA or RNA break inducing enzyme"* or *"SSBI enzyme"* is an enzyme capable of inducing a single-stranded DNA or RNA break at a particular nucleotide sequence.

In order to enable a break at a predetermined target site, the enzymes preferably include a binding domain and a cleavage domain. Particular enzymes capable of inducing double or single-stranded breaks are nucleases as well as variants thereof, no longer comprising a nuclease function but rather operating as recognition molecules in combination with another enzyme. In recent years, many suitable nucleases, especially tailored endonucleases have been developed comprising meganucleases, zinc finger nucleases, TALE nucleases, Argonaute nucleases, derived, for example, from *Natronobacterium gregoryi,* and CRISPR nucleases, comprising, for example, Cas, Cpf1, CasX or CasY nucleases as part of the Clustered Regularly Interspaced Short Palindromic Repeats (CRISPR) system. Thus, in a preferred aspect of the invention, the DSB or SSB inducing enzyme is selected from CRISPR systems like CRISPR/Cas9, CRISPR/Cpf1, CRISPR/CasX, CRISPR/CasY, CRISPR/Csm1 or CRISPR/MAD7, preferably a CRISPR/Cas9 endonuclease, a CRISPR/MAD7 endonuclease or a CRISPR/Cpf1 endonuclease, a zinc finger nuclease (ZFN), a homing endonuclease, a meganuclease and a TAL effector nuclease.

Rare-cleaving endonucleases are enzymes that have a recognition site of preferably about 14 to 70 consecutive nucleotides, and therefore have a very low frequency of cleaving, even in larger genomes such as most plant genomes. Homing endonucleases, also called meganucleases, constitute a family of such rare-cleaving endonucleases. They may be encoded by introns, independent genes or intervening sequences, and present striking structural and functional properties that distinguish them from the more classical restriction enzymes, usually from bacterial restriction-modification Type II systems. Their recognition sites have a general asymmetry which contrast to the characteristic dyad symmetry of most restriction enzyme recognition sites. Several homing endonucleases encoded by introns or inteins have been shown to promote the homing of their respective genetic elements into allelic intronless or inteinless sites. By making a site-specific double strand break in the intronless or inteinless alleles, these nucleases create recombinogenic ends, which engage in a gene conversion process that duplicates the coding sequence and leads to the insertion of an intron or an intervening sequence at the DNA level. A list of other rare cleaving meganucleases and their respective recognition sites is provided in Table I of WO 03/004659 (pages 17 to 20) (incorporated herein by reference).

Furthermore, methods are available to design custom-tailored rare-cleaving endonucleases that recognize basically any target nucleotide sequence of choice. Briefly, chimeric restriction enzymes can be prepared using hybrids between a zinc-finger domain designed to recognize a specific nucleotide sequence and the nonspecific DNA-cleavage domain from a natural restriction enzyme, such as Fokl. Such methods have been described e.g. in WO 03/080809, WO 94/18313 or WO 95/09233 and in Isalan et al. (2001). A rapid, generally applicable method to engineer zinc fingers illustrated by targeting the HIV-1 promoter. Nature biotechnology, 19(7), 656; Liu et al. (1997). Design of polydactyl zinc-finger proteins for unique addressing within complex genomes. Proceedings of the National Academy of Sciences, 94(11), 5525-5530.).

Another example of custom-designed endonucleases includes the TALE nucleases (TALENs), which are based on transcription activator-like effectors (TALEs) from the bacterial genus *Xanthomonas* fused to the catalytic domain of a nuclease (e.g. Fokl or a variant thereof). The DNA binding specificity of these TALEs is defined by repeat-variable di-residues (RVDs) of tandem-arranged 34/35-amino acid repeat units, such that one RVD specifically recognizes one nucleotide in the target DNA. The repeat units can be assembled to recognize basically any target sequences and fused to a catalytic domain of a nuclease create sequence specific endonucleases (see e.g. Boch et al. (2009). Breaking the code of DNA binding specificity of TAL-type III effectors. Science, 326(5959), 1509-1512; Moscou & Bogdanove (2009). A simple cipher governs DNA recognition by TAL effectors. Science, 326(5959), 1501-1501; and WO 2010/079430, WO 2011/072246, WO 2011/154393, WO 2011/146121, WO 2012/001527, WO 2012/093833, WO 2012/104729, WO 2012/138927, WO 2012/138939). WO 2012/138927 further describes monomeric (compact) TALENs and TALEs with various catalytic domains and combinations thereof.

Recently, a new type of customizable endonuclease system has been described; the so-called CRISPR/Cas system. A CRISPR system in its natural environment describes a molecular complex comprising at least one small and individual non-coding RNA in combination with a Cas nuclease or another CRISPR nuclease like a Cpf1 nuclease (Zetsche et al., "Cpf1 Is a Single RNA-Guides Endonuclease of a Class 2 CRISPR-Cas System", Cell, 163, pp. 1-13, October 2015) which can produce a specific DNA double-stranded break. Presently, CRISPR systems are categorized into 2 classes comprising five types of CRISPR systems, the type II system, for instance, using Cas9 as effector and the type V system using Cpf1 as effector molecule (Makarova et al., Nature Rev. Microbiol., 2015). In artificial CRISPR systems, a synthetic non-coding RNA and a CRISPR nuclease and/or optionally a modified CRISPR nuclease, modified to act as nickase or lacking any nuclease function, can be used in combination with at least one synthetic or artificial guide RNA or gRNA combining the function of a crRNA and/or a tracrRNA (Makarova *et al*., 2015, supra). The immune response mediated by CRISPR/Cas in natural systems requires CRISPR-RNA (crRNA), wherein the maturation of this guiding RNA, which controls the specific activation of the CRISPR nuclease, varies significantly between the various CRISPR systems, which have been characterized so far. Firstly, the invading DNA, also known as a spacer, is integrated between two adjacent repeat regions at the proximal end of the CRISPR locus. Type II CRISPR systems code for a Cas9 nuclease as key enzyme for the interference step, which system contains both a crRNA, and also a trans-activating RNA (tracrRNA) as the guide motif. These hybridize and form double-stranded (ds) RNA regions, which are recognized by RNAseIII and can be cleaved in order to form mature crRNAs. These then in turn associate with the Cas molecule in order to direct the nuclease specifically to the target nucleic acid region. Recombinant gRNA molecules can comprise both the variable DNA recognition region and also the Cas interaction region and thus can be specifically designed, independently of the specific target nucleic acid and the desired Cas nuclease. As a further safety mechanism, PAMs (protospacer adjacent motifs) must be present in the target nucleic acid region; these are DNA sequences, which follow on directly from the Cas9/RNA complex-recognized DNA. The PAM sequence for the Cas9 from *Streptococcus pyogenes* has been described to be "NGG" or "NAG" (Standard IUPAC nucleotide code) (Jinek et al, "A programmable dual-RNA-guided DNA endonuclease in adaptive bacterial immunity", Science 2012, 337: 816-821). The PAM sequence for Cas9 from *Staphylococcus aureus* is "NNGRRT" or "NNGRR(N)". Further variant CRISPR/Cas9 systems are known. Thus, a *Neisseria meningitidis* Cas9 cleaves at the PAM sequence NNNNGATT. A *Streptococcus thermophilus* Cas9 cleaves at the PAM sequence NNAGAAW. Recently, a further PAM motif NNNNRYAC has been described for a CRISPR system of *Campylobacter* (WO 2016/021973 A1). For Cpf1 nucleases it has been described that the Cpf1 -crRNA complex, without a tracrRNA, efficiently recognize and cleave target DNA proceeded by a short T-rich PAM in contrast to the commonly G-rich PAMs recognized by Cas9 systems (Zetsche et al., supra). Furthermore, by using modified CRISPR polypeptides, specific single-stranded breaks can be obtained. The combined use of Cas nickases with various recombinant gRNAs can also induce highly specific DNA double-stranded breaks by means of double DNA nicking. By using two gRNAs, moreover, the specificity of the DNA binding and thus the DNA cleavage can be optimized. Further CRISPR effectors like CasX and CasY effectors originally described for bacteria, are meanwhile available and represent further effectors, which can be used for genome engineering purposes (Burstein et al., "New CRISPR-Cas systems from uncultivated microbes", Nature, 2017, 542, 237-241).

The cleavage site of a DSBI/SSBI enzyme relates to the exact location on the DNA or RNA where the double-stranded break is induced. The cleavage site may or may not be comprised in (overlap with) the recognition site of the DSBI/SSBI enzyme and hence it is said that the cleavage site of a DSBI/SSBI enzyme is located at or near its recognition site. The recognition site of a DSBI/SSBI enzyme, also sometimes referred to as binding site, is the nucleotide sequence that is (specifically) recognized by the DSBI/SSBI enzyme and determines its binding specificity. For example, a TALEN or ZNF monomer has a recognition site that is determined by their RVD repeats or ZF repeats respectively, whereas its cleavage site is determined by its nuclease domain (e.g. Fokl) and is usually located outside the recognition site. In case of dimeric TALENs or ZFNs, the cleavage site is located between the two recognition/binding sites of the respective monomers, this intervening DNA or RNA region where cleavage occurs being referred to as the spacer region.

A person skilled in the art would be able to either choose a DSBI/SSBI enzyme recognizing a certain recognition site and inducing a DSB or SSB at a cleavage site at or in the vicinity of the preselected/predetermined site or engineer such a DSBI/SSBI enzyme. Alternatively, a DSBI/SSBI enzyme recognition site may be introduced into the target genome using any conventional transformation method or by crossing with an organism having a DSBI/SSBI enzyme recognition site in its genome, and any desired nucleic acid may afterwards be introduced at or in the vicinity of the cleavage site of that DSBI/SSBI enzyme.

A *"base editor enzyme"* or *"base editor"* as used herein refers to a protein or a fragment thereof having the same catalytical activity as the protein it is derived from, which protein or fragment thereof, alone or when provided as molecular complex, referred to as base editing complex herein, has the capacity to mediate a targeted base modification, i.e., the conversion of a base of interest resulting in a point mutation of interest which in turn can result in a targeted mutation, if the base conversion does not cause a silent mutation, but rather a conversion of an amino acid encoded by the codon comprising the position to be converted with the base editor. Preferably, the at least one base editor according to the present invention is temporarily or permanently linked to at least one site-specific effector, or optionally to a component of at least one site-specific effector complex. The linkage can be covalent and/or non-covalent.

Any base editor or site-specific effector, or a catalytically active fragment thereof, or any component of a base editor complex or of a site-specific effector complex as disclosed herein can be introduced into a cell as a nucleic acid fragment, the nucleic acid fragment representing or encoding a DNA, RNA or protein effector, or it can be introduced as DNA, RNA and/or protein, or any combination thereof.

There are two major and distinct pathways to repair breaks - homologous recombination and non-homologous end-joining (NHEJ). Homologous recombination requires the presence of a homologous sequence as a template (e.g., *"donor*") to guide the cellular repair process and the results of the repair are error-free and predictable. In the absence of a template (or *"donor*") sequence for homologous recombination, the cell typically attempts to repair the break via the process of non-homologous end-joining (NHEJ).

In a particularly preferred aspect of this embodiment, a repair nucleic acid molecule is additionally introduced into the plant cell. As used herein, a *"repair nucleic acid molecule"* is a single-stranded or double-stranded DNA molecule or RNA molecule that is used as a template for modification of the genomic DNA at the preselected site in the vicinity of or at the cleavage site. As used herein, *"use* as a *template for modification of the genomic DNA*", means that the repair nucleic acid molecule is copied or integrated at the preselected site by homologous recombination between the flanking region(s) and the corresponding homology region(s) in the target genome flanking the preselected site, optionally in combination with non-homologous end-joining (NHEJ) at one of the two end of the repair nucleic acid molecule (e.g. in case there is only one flanking region). Integration by homologous recombination will allow precise joining of the repair nucleic acid molecule to the target genome up to the nucleotide level, while NHEJ may result in small insertions/deletions at the junction between the repair nucleic acid molecule and genomic DNA.

As used herein, "a *modification of the genome*", means that the genome has changed by at least one nucleotide. This can occur by replacement of at least one nucleotide and/or a deletion of at least one nucleotide and/or an insertion of at least one nucleotide, as long as it results in a total change of at least one nucleotide compared to the nucleotide sequence of the preselected genomic target site before modification, thereby allowing the identification of the modification, e.g. by techniques such as sequencing or PCR analysis and the like, of which the skilled person will be well aware.

As used herein "a *preselected site", "a predetermined site"* or *"predefined site"* indicates a particular nucleotide sequence in the genome (e.g. the nuclear genome or the chloroplast genome) at which location it is desired to insert, replace and/or delete one or more nucleotides. This can e.g. be an endogenous locus or a particular nucleotide sequence in or linked to a previously introduced foreign DNA or transgene. The preselected site can be a particular nucleotide position at (after) which it is intended to make an insertion of one or more nucleotides. The preselected site can also comprise a sequence of one or more nucleotides, which are to be exchanged (replaced) or deleted.

As used in the context of the present application, the term *"about*" means +/- 10% of the recited value, preferably +/- 5% of the recited value. For example, about 100 nucleotides (nt) shall be understood as a value between 90 and 110 nt, preferably between 95 and 105.

As used herein, a *"flanking region*", is a region of the repair nucleic acid molecule having a nucleotide sequence which is homologous to the nucleotide sequence of the DNA region flanking (i.e. upstream or downstream) of the preselected site. It will be clear that the length and percentage sequence identity of the flanking regions should be chosen such as to enable homologous recombination between said flanking regions and their corresponding DNA region upstream or downstream of the preselected site. The DNA region or regions flanking the preselected site having homology to the flanking DNA region or regions of the repair nucleic acid molecule are also referred to as the homology region or regions in the genomic DNA.

To have sufficient homology for recombination, the flanking DNA regions of the repair nucleic acid molecule may vary in length, and should be at least about 10 nt, about 15 nt, about 20 nt, about 25 nt, about 30 nt, about 40 nt or about 50 nt in length. However, the flanking region may be as long as is practically possible (e.g. up to about 100-150 kb such as complete bacterial artificial chromosomes (BACs). Preferably, the flanking region will be about 50 nt to about 2000 nt, e.g. about 100 nt, 200 nt, 500 nt or 1000 nt. Moreover, the regions flanking the DNA of interest need not be identical to the homology regions (the DNA regions flanking the preselected site) and may have between about 80% to about 100% sequence identity, preferably about 95% to about 100% sequence identity with the DNA regions flanking the preselected site. The longer the flanking region, the less stringent the requirement for homology. Furthermore, to achieve exchange of the target DNA sequence at the preselected site without changing the DNA sequence of the adjacent DNA sequences, the flanking DNA sequences should preferably be identical to the upstream and downstream DNA regions flanking the preselected site.

As used herein, *"upstream"* indicates a location on a nucleic acid molecule, which is nearer to the 5' end of said nucleic acid molecule. Likewise, the term *"downstream"* refers to a location on a nucleic acid molecule which is nearer to the 3' end of said nucleic acid molecule. For avoidance of doubt, nucleic acid molecules and their sequences are typically represented in their 5' to 3' direction (left to right).

In order to target sequence modification at the preselected site, the flanking regions must be chosen so that 3' end of the upstream flanking region and/or the 5' end of the downstream flanking region align(s) with the ends of the predefined site. As such, the 3' end of the upstream flanking region determines the 5' end of the predefined site, while the 5' end of the downstream flanking region determines the 3' end of the predefined site.

As used herein, said preselected site being located outside or away from said cleavage (and/or recognition) site, means that the site at which it is intended to make the genomic modification (the preselected site) does not comprise the cleavage site and/or recognition site of the DSBI/SSBI enzyme or the base editor enzyme, i.e. the preselected site does not overlap with the cleavage (and/or recognition) site. Outside/away from in this respect thus means upstream or downstream of the cleavage (and/or recognition) site.

In the present invention it was found that *BnBBM* enhances somatic embryo formation in sugar beet transformation and leads to plants with high quality, comparable to plants grown from seeds. The high quality of the obtained plants had been verified by the absence of obvious visible phenotypes but also experimentally for root features e.g. weight, sucrose content as well as for leaf parameters like photosynthesis. Such an increased plant quality was observed after Agrobacterium-based transformation as well as biolistic transformation methods in combination with *BnBBM* induced somatic embryogenesis
The enhanced somatic embryo formation by using *BnBBM* in a hormone free transformation - leading to plant development without obvious phenotypes - allows fast validation of trait candidate genes especially for root traits and root tissue specific promoters, furthermore it could also be combined with the application of the genome editing technology.
A remarkable time saving is the second improvement for plant transformation. In the described approach T0 plants can be directly used for different kind of analyses, whereas normally T1 plants are used in conventional transformation methods to do such analyses. This is necessary since the conventional T0 plants produced via organogenesis have an obvious phenotype with not forming proper tap roots, the specific storage organ of sugar beet.

Due to the usage of the T0 plants in the described invention up to 2.5 years can be saved for the above-mentioned analyses processes. Moreover, there is also the possibility of transient genome editing in *Beta vulgaris* when using T0 plants. Accordingly, such a plant analysis at the T0 level represents a preferred embodiment of the present invention.

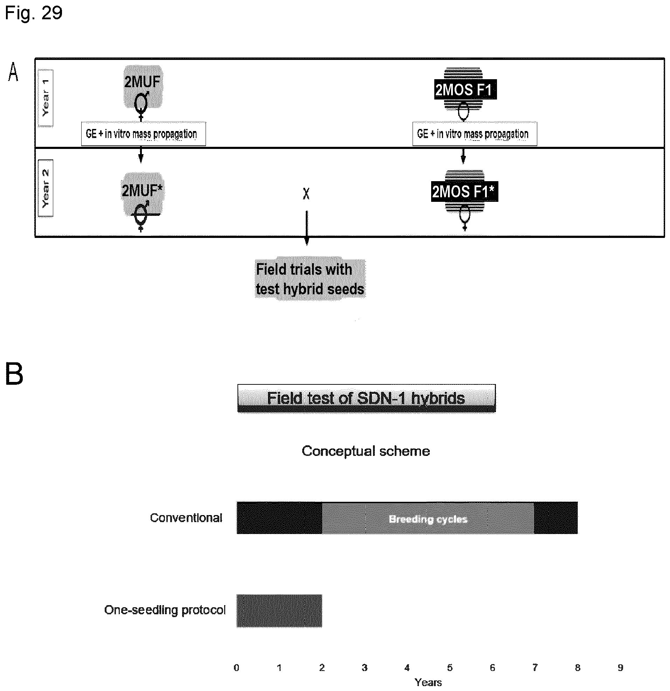

The time savings for creating genetically engineered *Beta vulgaris* hybrids are even higher. Up to 6-7 years might be saved, since instead of generating edits in many parental lines of monogerm, edits could directly be generated in the parents for producing the hybrids (**Fig. 29**). Further improvements of this invention are a less somaclonal variation in plants obtained from this method and a significant increase of seed production of transgenic plants. This is in particular for Beta vulgaris plants often a big problem and leads to strong limitation regarding usability of different lines. A further aspect of the invention is drawn to a method for the production of *Beta vulgaris* plant. It was found, that using the above described methods of somatic embryogenesis and organogenesis, whole plants can be produced even for recalcitrant plant species or plant genotypes. Further, it was found that with the present invention, the resulting *Beta vulgaris* plants show no pleiotropic phenotype.

Accordingly, the invention provides a method for producing *Beta vulgaris* plant comprising somatic embryogenesis or organogenesis as described above with reference to steps (A1)-(A3) and (B1)-(B3), optionally transformation or genomic modification as described above with reference to steps (C1) and (C2), and regenerating a *Beta vulgaris* plant from an embryo and/or a shoot resulting from step (B1), (B2) or (B3) of the above disclosed method, in particular a transgenic plant resulting from step (C1) and/or a modified plant resulting from step (C2).

Preferably, the conditions of somatic embryogenesis are chosen so that embryos are formed directly in step (B1) or (B2). This can be achieved as described above, for example by carrying out the cultivation in a medium free of plant hormones. From the shoots derived from somatic embryogenesis in hormone free medium, seedlings are grown having natural roots instead of the adventitious roots that are artificially induced from *in vitro* shoots which is allowing physiological analysis already at the T0 level. Moreover, the quality of obtained plants is clearly better compared to plants generated with published protocols. This is leading to an enormous time and cost saving due to reduced tissue culture work in comparison with the *state of the art.* Accordingly, such a physiological analysis at the T0 level represents a preferred embodiment of the present invention.

Subject matter of the present invention are also the plants that are obtained or obtainable by the methods described above. Accordingly, one embodiment of the invention is a transgenic *Beta vulgaris* plant obtained or obtainable by the above method of transforming a plant cell and producing a *Beta vulgaris* plant (claim 12), as well as progeny plant or parts thereof, wherein the progeny or the part comprises the at least one nucleotide sequence of interest as transgene.

Another embodiment of the invention is a genetically modified plant obtained or obtainable by the above method of modifying the genome of a plant cell and producing a *Beta vulgaris* plant (claim 12) as well as progeny plants or parts thereof, wherein the progeny or the part comprises the modification in the genome introduced by the above method of modification.

Further subject matter of the present invention is a plant cell or a seed derived from the above transgenic plant or genetically modified plant. Such a plant cell preferably comprises a polynucleotide encoding a BnBBM-derived polypeptide transiently or stably integrated and a single-stranded DNA break (SSB)- or double-stranded DNA break (DSB)-inducing enzyme or base editor enzyme, which preferably recognizes a predetermined site in the genome of said cell and optionally a repair nucleic acid molecule. The polynucleotide encoding the BnBBM-derived polypeptide is preferably operably linked to a suitable regulatory sequence so that the plant cell is capable of expressing the BnBBM-derived polypeptide. A regulatory sequence means, for example, a *"promoter"* which refers to a nucleotide sequence, usually upstream (5') to its coding sequence, which controls the expression of the coding sequence by providing the recognition for RNA polymerase and other factors required for proper transcription. *"Constitutive promoter"* refers to promoters that direct gene expression in nearly all tissues and at all times. Examples of constitutive promoters include CaMV 35S promoter, double CaMV 35S promoter (70S promoter), nopaline synthase (nos) promoter, *Bd*EF1 promoter, or ubiquitin promoters like PcUbi4 or *Zm*Ubi1. *"Regulated promoter"* refers to promoters that direct gene expression not constitutively but in a temporally and/or spatially regulated manner and include both tissue-specific and inducible promoters. It includes natural and synthetic sequences as well as sequences, which may be a combination of synthetic and natural sequences. Different promoters may direct the expression of a gene in different tissues or cell types, or at different stages of development, or in response to different environmental conditions. New promoters of various types useful in plant cells are constantly being discovered and are well-known to a person skilled in the art. *"Tissue-specific promoter"* refers to regulated promoters that are not expressed in all plant cells but only in one or more cell types in specific organs (such as leaves or seeds), specific tissues (such as embryo or cotyledon), or specific cell types (such as leaf parenchyma or seed storage cells). These also include promoters that are temporally regulated (such as in early or late embryogenesis), during fruit ripening in developing seeds or fruit, in fully differentiated leaf, or at the onset of senescence. *"Inducible promoter"* refers to those regulated promoters that can be turned on in one or more cell types by an external stimulus (such as a chemical, light, hormone, stress, or pathogen). Examples for inducible promoter are promoters inducible by ecdysone, dexamethasone, ethanol. Such promoters are well known from the state of the art (e.g., Samalova et al. (2005). pOp6/LhGR: a stringently regulated and highly responsive dexamethasone-inducible gene expression system for tobacco. The Plant Journal, 41(6), 919-935; Gatz & Lenk (1998). Promoters that respond to chemical inducers. Trends in Plant Science, 3(9), 352-358.).

Another subject-matter of the present invention is a plant cell comprising a polynucleotide encoding a BnBBM-derived polypeptide transiently or stable integrated, and a single-stranded DNA break (SSB) or double stranded DNA break (DSB) inducing enzyme or base editor enzyme which preferably recognize a predetermined site in the genome of said cell, and optionally a repair nucleic acid molecule, wherein preferably the polynucleotide encoding the BnBBM-derived polypeptide being operatively linked to a suitable regulatory sequence, so that the plant cell is capable of expressing the polypeptide. Such plant cell can be obtained when conducting the above described method for modifying the genome of a plant cell.

Another aspect of the invention is the use of a nucleic acid encoding a BnBBM-derived polypeptide as described above in a method for somatic embryogenesis or organogenesis and/or plant regeneration of *Beta vulgaris* from callus or a tissue explant. In particular, the invention discloses the use of nucleic acid comprising a coding nucleotide sequence selected from
(i) a nucleotide sequence of SEQ ID NO: 1, or a sequence which is at least 80% identical to the sequence of SEQ ID NO: 1; and
(ii) a nucleotide sequence encoding a polypeptide having the amino acid sequence of SEQ ID NO: 2, or an amino acid sequence which is at least 80% identical to the sequence of SEQ ID NO: 2.
in a method for somatic embryogenesis or organogenesis and/or plant regeneration of *Beta vulgaris* from callus or a tissue explant;
in a method for direct or indirect regeneration of a *Beta vulgaris* plant;
in a method of transformation of a *Beta vulgaris* plant cell; or
in a method of modifying the genome of a *Beta vulgaris* plant cell.

According to a further aspect of the invention, the activity of a BnBBM-derived polypeptide in the above described methods and uses can be combined with a polynucleotide encoding a *WUS2* polypeptide, mRNA encoding a *WUS2* polypeptide, or *WUS2* polypeptide(s).

The activity of BnBBM in terms of the invention can also be used for the fast propagation of specific plant material. This can be helpful to multiply individual plants having a specific phenotype or genetic composition, e.g specific hybrid plants. When using the somatic embryogenesis approach, embyros form this specific plant can be produced without the need of producing seeds via a classical seed production approach. The genetic composition of the multiplied plants remains the same. The produced embryos might be used to be encapsulated in a matric allowing the survival of the embryos and also allowing the outgrowth of the embryo to whole plants → artificial seeds.

According to this embodiment, the invention provides a method of propagating plants, comprising the following steps:
(A1) providing an explant which is from a tissue of a plant and comprises at least one cell comprising an expression cassette comprising a nucleotide sequence
   (i) having a polypeptide coding sequence of SEQ ID NO: 1, or a coding sequence which is at least 80% identical to the sequence of SEQ ID NO: 1; or
   (ii)encoding a polypeptide having the amino acid sequence of SEQ ID NO: 2, or an amino acid sequence which is at least 80% identical to the sequence of SEQ ID NO: 2,
   wherein the nucleotide sequence is operably linked to a heterologous constitutive regulatory element; or
(A2) providing an explant which is from a tissue of a *Beta vulgaris* plant and comprises at least one cell comprising an expression cassette comprising a nucleotide sequence
   (i) having a polypeptide coding sequence of SEQ ID NO: 1, or a coding sequence which is at least 80% identical to the sequence of SEQ ID NO: 1; or
   (ii) encoding a polypeptide having the amino acid sequence of SEQ ID NO: 2, or an amino acid sequence which is at least 80% identical to the sequence of SEQ ID NO: 2,
   wherein the nucleotide sequence is operably linked to a heterologous inducible regulatory element; and
(B1) cultivating the explant of step (A1) under conditions promoting growth of embryos out of the explant, wherein in the explant the polypeptide is expressed from the expression cassette constitutively; or
(B2) cultivating the explant of step (A2) under conditions promoting growth of shoots out of the explant, wherein in the explant the polypeptide is expressed from the expression cassette upon induction of the heterologous inducible expression system.

While the above aspects have primarily been described by reference to *Beta vulgaris,* essential characteristics of the invention apply analogously to *Zea mays.* Accordingly, the terms and procedural steps defined above should be understood in such a way that they also apply to *Zea mays.* In addition, however, it was found that the transformation and regeneration capacity of *Zea mays* plant material can be further improved by combining the activity of the BnBBM-derived polypeptide with WUS2, preferably Zea mays WUS2 (ZmWUS2; cDNA: SEQ ID NO: 12; protein: SEQ ID NO: 13).

Accordingly, in addition to introducing an expression cassette comprising a polynucleotide encoding a BBM-derived polypeptide, the approach of the invention according to this aspect further provides for introducing an expression cassette comprising a polynucleotide encoding a WUS2 polypeptide, mRNA encoding a WUS2 polypeptide, or WUS2 polypeptide(s) into the at least one plant cell concerned. The polynucleotide encoding a WUS2 polypeptide can be integrated into the same expression cassette and operably linked to the same regulatory elements as the BnBBM-derived polynucleotide.

WUS2 can be provided to the at least one plant cell concerned as an expression cassette comprising a second coding nucleotide sequence
(i) having a nucleotide sequence of SEQ ID NO: 12, or a sequence which is at least 80% identical to the sequence of SEQ ID NO: 12; or
(ii) encoding a polypeptide having the amino acid sequence of SEQ ID NO: 13, or a sequence which is at least 80% identical to the sequence of SEQ ID NO: 13,
wherein the second nucleotide sequence is operably linked to a heterologous regulatory element.

Further, WUS2 can be provided to the at least one plant cell as an mRNA encoding a polypeptide having the amino acid sequence of SEQ ID NO: 13, or a sequence, which is at least 80% identical to the sequence of SEQ ID NO: 13.

WUS2 can also be introduced into the at least one plant cell as a polypeptide having the amino acid sequence of SEQ ID NO: 13, or a sequence, which is at least 80% identical to the sequence of SEQ ID NO: 13.

Further, an enhanced expression level of an endogenous gene encoding the polypeptide having the amino acid sequence of SEQ ID NO: 13, or a sequence which is at least 80% identical to the sequence of SEQ ID NO: 13 can be induced in the at least one plant cell. This leads to an enhanced expression level of the endogenous *WUS2* gene, i.e. to the presence or occurrence of WUS2 polypeptide in an enhanced amount in the plant cell.

The activation of the expression of the endogenous gene can be achieved by modifying the activity or structure of the promoter of the endogenous gene encoding the WUS2 polypeptide. For instances, enhancer elements can be introduced into the promoter by means of gene editing; or either an enhancer element regulating the promoter can be further strengthen or a silencer element regulating the promoter can be weakened by e.g. targeted mutagenesis/modification; or modifications can be introduced into the epigenome related to enhancers by means of gene editing tools like CRISPR systems (Hilton et al. (2015). Epigenome editing by a CRISPR-Cas9-based acetyltransferase activates genes from promoters and enhancers. Nature biotechnology, 33(5), 510-517); or synthetic transcription factors based on e.g. TALE activators or dCas9 activators can be introduced into the cell where they are able to bind targeted recognition sites on or near by the promoter und activate transcription of the *WUS2* gene (Cheng et al. (2013). Multiplexed activation of endogenous genes by CRISPR-on, an RNA-guided transcriptional activator system. Cell research, 23(10), 1163.); or the amount of microRNA (miRNA) in the plant cell regulating the expression of the *WUS2* gene by post-transcriptional inhibition can be reduced by e.g. knock out (null mutant) or knock down in order to increase the amount of translated WUS2 polypeptide in the plant cell.

Dependent on the plant species as well as on the cell type different levels of gene or expression activation are needed in order to have adequate amount of WUS2 polypeptide present in the plant cell at the time when regeneration takes place. There are various techniques available to a person skilled in the art in order to measure the actual expression level of an endogenous or an introduced gene, e.g., qPCR, RT-PCR, Northern blot, or microrarrays. These methods allow those skilled in the art by routine work to adjust the level of expression of the *WUS2* gene which effects improved regeneration ability from diverse tissues or somatic and reproductive cells (e.g. microspores). In a preferred embodiment, in the plant cell the expression level of an endogenous gene encoding a WUS2 polypeptide is increased at least by the factor of 2, the factor of 3, or the factor of 5, preferably by the factor of 10, the factor of 25 or factor of 50, more preferred by the factor of 100, the factor of 200, or the factor of 500.

As described further above the induction of an enhanced expression level of an endogenous gene in a plant cell can be carried out by the application of one or more activators or a precursor thereof. These can be applied to the medium in which the plant cells are cultivated and is then actively or passively absorbed by the plant cell. Furthermore, the one or more activator or a precursor thereof can be directly introduced into the plant cell by microinjection, electroporation or biolistic bombardment. Beside the above synthetic transcription activators, a number of further activators are known from the state of the art that can be used for increasing the expression level of an endogenous gene, in particular the expression level of the endogenous *WUS2* gene: In the recent years, the technical fields of chemical plant genetics and chemical plant biology emerged where biological systems are treated with small molecules to specifically perturb cellular functions. Small molecules are used commercially as drugs, herbicides, and fungicides in different systems, but in recent years they are increasingly exploited also as tools for genetic regulation. For instance, chemical genetics involves the discovery of small-molecule effectors of various cellular functions through screens of compound libraries (Dejonghe & Russinova (2017). Plant Chemical Genetics: From Phenotype-Based Screens to Synthetic Biology. Plant Physiology, pp-01805; Kawasumi, M., & Nghiem, P. (2007). Chemical genetics: elucidating biological systems with small-molecule compounds. Journal of Investigative Dermatology, 127(7), 1577-1584.). Such small molecule effectors suitable for the activation of the expression of a target gene like WUS2, can be identified by chemical screens following different strategies (Dejonghe & Russinova, 2017). Comprehensive compound libraries are available which allow the simple screening of countless small molecules and the identification of effectors which can be used for activation of the gene expression of genes like *WUS2.* As mentioned above another approach to enhance the expression level of an endogenous gene like *WUS2* is the application of so-called synthetical transcription activators. They are typically designed by the fusion of a recognition domain and at least one activator domain. The recognition domain can be derived from known systems like Zinc finger, TAL effectors or CRISPR; for activation, fusing for instances the herpes simplex virus derived VP-16 or VP-64 activation domains to a recognition domain can cause an increase in transcription. Weaker activation domains such as the AD of human NF-κB add to the variety of options for gene activation. Furthermore, as shown on endogenous promoters, combinations of activators can be used to introduce synergistic effects (Moore et al. (2014). "Transcription activator-like effectors: a toolkit for synthetic biology." ACS synthetic biology, 3(10), 708-716.; US 2002/0046419 A1; Lowder et al. (2017). "Multiplexed transcriptional activation or repression in plants using CRISPR-dCas9-based systems." Plant Gene Regulatory Networks: Methods and Protocols, 167-184.). The synthetical transcription activator can be delivered to the plant cell or introduced into the plant cell also as precursor, i.e. as DNA or RNA molecule encoding such artificial or synthetical transcription activator or a domain thereof or as inactive form of transcription activator which is activated later in the cell or a in a specific compartment of the cell. Finally, enhancing expression of *WUS2* genes can be also achieved by the inactivation of upstream negative regulators or by the creation of a mutant version of the *BBM* gene that is resistant to such negative regulators.

According to the above described aspects, the invention further provides the following items:
[01] A method of promoting somatic embryogenesis or organogenesis of *Zea mays,* comprising the following steps:
   (a) providing an immature embryo or a mature embryo of a *Zea mays* plant comprising at least one plant cell, and
   (b) introducing into the at least one plant cell
      (I) an expression cassette comprising a first nucleotide sequence
         (i) having a polypeptide coding sequence of SEQ ID NO: 1, or a sequence which is at least 80% identical to the sequence of SEQ ID NO: 1; or
         (ii) encoding a polypeptide having the amino acid sequence of SEQ ID NO: 2, or a sequence which is at least 80% identical to the sequence of SEQ ID NO: 2,
         wherein the first nucleotide sequence is operably linked to a first heterologous regulatory element; and
      (111) an expression cassette comprising a second nucleotide sequence
         (i) having a polypeptide coding sequence of SEQ ID NO: 12, or a sequence which is at least 80% identical to the sequence of SEQ ID NO: 12, preferably at least 85%, at least 90%, more preferably at least 95%, at least 98% or at least 99% identity to SEQ ID NO: 12; or
         (ii) encoding a polypeptide having the amino acid sequence of SEQ ID NO: 13, or a sequence which is at least 80% identical to the sequence of SEQ ID NO: 13, preferably at least 85%, at least 90%, more preferably at least 95%, at least 98% or at least 99% identity to SEQ ID NO: 13,
         wherein the second nucleotide sequence is operably linked to the first heterologous regulatory element or to a second heterologous regulatory element; or
      (112) an mRNA encoding a polypeptide having the amino acid sequence of SEQ ID NO: 13, or a sequence, which is at least 80% identical to the sequence of SEQ ID NO: 13, preferably at least 85%, at least 90%, more preferably at least 95%, at least 98% or at least 99% identity to SEQ ID NO: 13; or
      (113) a polypeptide having the amino acid sequence of SEQ ID NO: 13, or a sequence, which is at least 80% identical to the sequence of SEQ ID NO: 13, preferably at least 85%, at least 90%, more preferably at least 95%, at least 98% or at least 99% identity to SEQ ID NO: 13; or
      (II4) inducing in said at least one plant cell an enhanced expression level of an endogenous gene encoding the polypeptide having the amino acid sequence of SEQ ID NO: 13, or a sequence which is at least 80% identical to the sequence of SEQ ID NO: 13, preferably at least 85%, at least 90%, more preferably at least 95%, at least 98% or at least 99% identity to SEQ ID NO: 13, and
   (c) inducing callus formation from the cell obtained in step (b) or a progeny thereof; and
   (d) cultivating the callus under conditions promoting growth of embryos and/or shoots out of the callus wherein in the callus the first polypeptide is expressed from the expression cassette and the second polypeptide is expressed from the expression cassette, is translated from introduced mRNA, is enhanced expressed from the endogenous gene, or is present.
[02] The method of item [01], wherein the first and the second heterologous regulatory elements are selected from the group consisting of a constitutive promoter, an inducible promoter or an inducible expression system.
[03] The method of item [01] or [02], wherein in step (b) introducing of the expression cassette(s) results in a stable integration thereof into the genome of the at least one plant cell or in a progeny cell thereof.
[04] The method according to any one of items [01]-[03], wherein cultivating in step (d) is carried out in a medium free of plant hormones.
[05] The method according to any one of items [01]-[04], wherein step (b) comprises Agrobacterium-mediated transformation, particle bombardment, electroporation or microinjection.
[06] The method according to any one of items [01]-[05], further comprising a step of
   (i) introducing at least one nucleotide sequence of interest into the at least one plant cell or a predecessor thereof to be used in step (b) or into a cell obtained in step (b), and/or
   (ii) modifying the genome of the at least one plant cell or a predecessor thereof to be used in step (b) or of a cell obtained in step (b) by introducing into said cell a single stranded DNA break (SSB) inducing enzyme or a double stranded DNA break (DSB) inducing enzyme which preferably recognize a predetermined site in the genome of said cell, and optionally a repair nucleic acid molecule, or a single stranded DNA break (SSB) inducing enzyme which preferably recognizes a predetermined site in the genome of said cell and is fused to a base editor enzyme,
      wherein the modification of said genome is selected from
      I. a replacement of at least one nucleotide;
      II. a deletion of at least one nucleotide;
      III. an insertion of at least one nucleotide; or
      IV. any combination of I. - III.
[07] A method of regeneration of *Zea mays* plant from callus comprising somatic embryogenesis or organogenesis according to the method of any one of items [01]-[05], optionally transformation or genomic modification according to item [06] (i) or (ii), and regenerating a plant from an embryo and/or a shoot resulting from step (d) of the method of any one of items [01]-[05], in particular a transgenic plant resulting from step (i) of the method of item [06] and/or a modified plant resulting from step (ii) of the method of item [06].
[08] A transgenic *Zea mays* plant obtained or obtainable by the method of item [07] or a progeny plant thereof.
09] A modified *Zea mays* plant obtained or obtainable by the method of item [07], or a progeny plant thereof.
[10]A plant cell or a seed of the plant of item [08], wherein the plant cell or the seed comprises the at least one nucleotide sequence of interest as transgene.
[11]A plant cell or a seed of the plant of item [09], wherein the plant cell or the seed comprises the modification in the genome.
[12] Use of a first nucleic acid comprising a nucleotide sequence
   (i) having a polypeptide coding sequence of SEQ ID NO: 1, or a sequence which is at least 80% identical to the sequence of SEQ ID NO: 1; or
   (ii) encoding a polypeptide having the amino acid sequence of SEQ ID NO: 2, or a sequence which is at least 80% identical to the sequence of SEQ ID NO: 2,
   and a second nucleic acid comprising a nucleotide sequence
   (i) having a polypeptide coding sequence of SEQ ID NO: 12, or a sequence which is at least 80% identical to the sequence of SEQ ID NO: 12; or
   (ii) encoding a polypeptide having the amino acid sequence of SEQ ID NO: 13, or a sequence which is at least 80% identical to the sequence of SEQ ID NO: 13,
   in a method for somatic embryogenesis or organogenesis and/or plant regeneration of *Zea mays* from callus, in a method for direct or indirect regeneration of a *Zea mays* plant, in a method of transformation of a *Zea mays* plant cell, or in a method of modifying the genome of a *Zea mays* plant cell.

Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Cray, jointly published by BIOS Scientific Publications Ltd (UK) and Blackwell Scientific Publications, UK. Other references for standard molecular biology techniques include Sambrook and Russell (2001) Molecular Cloning: A Laboratory Manual, Third Edition, Cold Spring Harbor Laboratory Press, NY, Volumes I and II of Brown (1998) Molecular Biology LabFax, Second Edition, Academic Press (UK). Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

All patents, patent applications, and publications or public disclosures (including publications on internet) referred to or cited herein are incorporated by reference in their entirety.

The invention will be further described with reference to the following Figures and Examples described herein. However, it is to be understood that the invention is not limited to such Examples.

### Figures:

- **Fig. 1**: Shoot formation in sugar beet with and without DEX inducible BBM-GR expression. A: no shoot formation without application of the DEX inducer; B: Many shoots (arrows) are formed upon treatment with the DEX inducer.
- **Fig. 2**: Dex inducible 35s-BnBBM-GR plants look comparable to control plants regenerated from tissue culture without transgene (control).
- **Fig. 3**: BnBBM increases callus-based transformation efficiency in sugar beet (quantification of transformation rate) in amenable and recalcitrant genotypes. A: inducible system - comparison of percentage regenerated plants between control, BnBBM-GR without DEX Inducer, and BnBBM-GR with DEX Inducer. B: constitutive overexpression - comparison between control and 35s-BnBBM overexpression. Sugar beet plants transformed with Tdt expression construct under a constitutive promoter serves as control in A and B.
- **Fig. 4**: 35s-BnBBM (constitutive expression) give rise to embryo (B, arrow shows embryo (e)) and seedlings (C, arrow shows root (r) and seedling (s)) while standard callus based trafo for sugar beet only generates shoots (A; arrow shows shoot derived from organogenesis but no root (s*)). In total may transgenic events can be produced by means of 35s-BnBBM expression (D).
- **Fig. 5**: 35s-BnBBM plants continuously generate embryo like structures (arrows) from leaves
- **Fig. 6**: 35s-BnBBM produces mainly embryos on hormone free medium (B) while produces shoots and embryos on normal regeneration medium with hormones (A)
- **Fig. 7**: BnBBM-GR triggers shoot regeneration directly from leaf (after 3weeks on DEX medium; (B); arrows indicate shoots). Control (A) is plant material derived from plants transformed with the Dex-inducible 35S-BnBBM-GR system but cultured in media without DEX inducer.
- **Fig. 8**: Regenerated plants directly from leaves as described in Example 6 showing a normal morphology
- **Fig. 9**: BnBBM breaks recalcitrance in sugar beet. 35s:BnBBM overexpressing callus of recalcitrant genotype (B) compared with control callus of the same genotype without overexpression of BnBBM (A). Arrows indicate shoots. C: Regenerated transgenic plants of recalcitrant genotype as shown in B.
- **Fig. 10**: Comparison of control construct with vector 70s-tDT versus co-transformation with vector 35s::BnBBM-GR and vector 70s-tDT with inducible BnBBM in two different genotypes (amenable (left) and recalcitrant (right)).
- **Fig. 11**: Constitutive ZmWUS2 overexpression combined with constitutive BnBBM overexpression promotes maize regeneration after biolistic delivery. Arrows indicate fluorescent embryonic structures.
- **Fig. 12**: Vector map of IPR232-pS-01

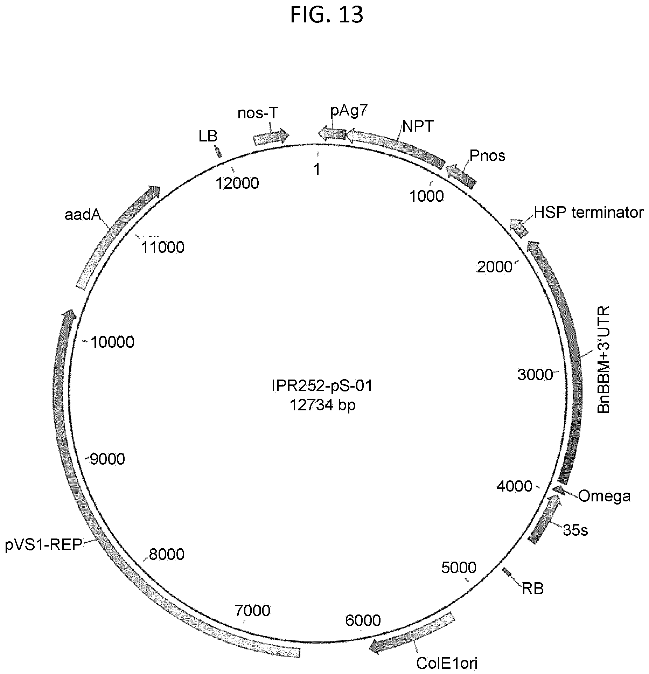

- **Fig. 13**: Vector map of IPR252-pS-01
- **Fig. 14**: Vector map of IPR 252-pK-02
- **Fig. 15**: *In vitro* T0 plants regenerated on hormone-free media and with BnBBM show normal root development.
- **Fig. 16**: Embryogenesis via BnBBM in hormone free media. **A:** Media with hormones; **B:** Media without hormones. By changing media components, i.e. completely removing GA3 and BAP, somatic embryo formation is significantly increased.
- **Fig. 17**: Comparison of seedlings from hormone free media and shoots from media with hormones. **A:** Regenerated shoot on media with the plant hormones GA3 and BAP; **B:** regenerated seedling on hormone free media.
- **Fig. 18**: Seedlings (**A** and **B**) grown on media without hormones, developing a normal root system. left: top view; right: bottom view
- **Fig. 19**: Transgenic 35S-BnBBM-GR plants obtained by particle bombardment. **A:** Seedling cultivated in vitro (left: top view; right: bottom view); **B:** Plantlets after transfer to greenhouse.
- **Fig. 20**: Plant derived from the hormone free protocol develops robust root system.
- **Fig. 21**: Plants derived from the hormone free protocol develops normal upper ground part (one months after transfer to green house)
- **Fig. 22**: Analysis of morphology of tap root (plant 1) after 4 months in the greenhouse
- **Fig. 23**: Analysis of morphology of tap root (plant 2) after 4 months in the greenhouse
- **Fig. 24**: Analysis of transgenic plants from hormone free transformation protocol. **a:** Photosynthesis rate in [µE] **b:** Sucrose concentration in tap root [µmol/g]
- **Fig. 25**: 35s-BnBBM-GR plants are fertile (one-seedling protocol) **a:** Seed production in kg per plant **b:** Viable seed produced per plant
- **Fig. 26**: Comparison of seed production
- **Fig. 27**: Comparison of tap roots after 4 months development in the greenhouse **A:** Tap roots from seed derived plants **B:** Tap roots from plants obtained by the hormone free transformation
- **Fig. 28**: Tap root parameters of WT seed derived plants and transgenic plants obtained from the hormone free transformation protocol **a:** Comparison of tap root weight [g] from seed derived WT and transgenic 35S-BnBBM-GR plants obtained by the hormone free transformation protocol **b:** Comparison of sucrose content [µmol/g FW] from seed derived WT and transgenic 35S-BnBBM-GR plants obtained by the hormone free transformation protocol
- **Fig. 29**: Conceptual time saving for field test of SDN-1 hybrids

### Examples

### 1. Expression of BnBBM

The principle of the inducible expression of BnBBM is based on a fusion of the rat glucocorticoid receptor domain to the transcription factor BnBBM (BnBBM-GR). Upon induction with Dexamethasone, the GR receptor domain will change conformation, so that the BnBBM-GR protein will enter the nucleus and generate the desired response.

The binary plasmids IPR232-pS-01 and IPR252-pS-01 were produced by KWS with standard cloning procedures. The T-DNA also contains the neomycin phosphotransferase II (nptll) gene that confers resistance to a range of aminoglycoside antibiotics such as kanamycin or paromomycin and was used for the selection of transgenic plant cells and tissues. The NOS promoter and the pAG7 terminator flank the nptll gene. The backbone of the binary vector contains the colE1 and the pVS1 origins for plasmid replication in Escherichia coli and Agrobacterium tumefaciens, respectively; and the aadA gene that confers streptomycin / spectinomycin resistance for bacteria selection.

The binary plasmid was transformed into AGL-1 Agrobacterium strain by a standard procedure.

### 2. Dex-induced 35S-BnBBM-GR enhances shoot formation from Beta vulgaris calli in hormone-containing tissue culture media

By applying the following method callus for *Beta vulgaris* (sugar beet) has been induced: Micropropagated shoots were used as starting material. Shoots were multiplied in medium containing MS salts, 30 g/l sucrose, 0.25 mg/l benzyladenine (BAP) and 10 g/l agar (pH 6.0). To induce friable callus, leaf explants were incubated for 7-8 weeks in media having MS salts, 15 g/l sucrose, 2 mg/l BAP, and 8 g/l agar (pH 6.0).

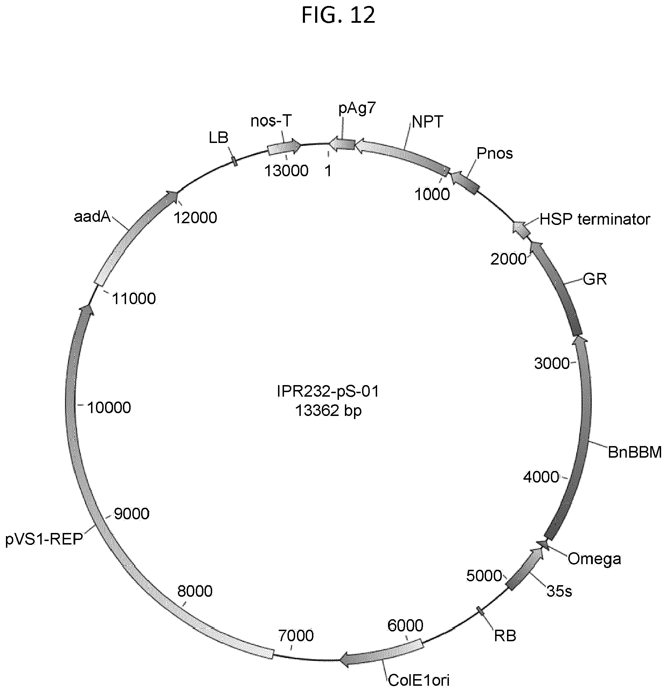

For *Agrobacterium*-mediated transformation friable calli were mounted in media containing MS salts, 30 g/l sucrose, 1 mg/l GA3, 1 mg/l TDZ, and 10 g/l agar (pH 6.0) and kept for 1 week in the dark at 24°C. Agrobacterium harbouring the vector of IPR232-pS-01 (**Fig. 12**) was grown in medium containing 5 g/l tryptone, 2.5 g/l yeast extract, 1 g/l NaCl, 5 g/l mannitol, 0.1 g/l MgSO₄x7H₂O, 0.25 g/l KH₂PO₄, 1 g/l glutamic acid (pH 7.0) supplemented with the appropriate antibiotics, at 28 °C, for 24 h.

Calli were inoculated with Agrobacterium suspension at an OD600 = 0,3, the callus tissue and the Agrobacterium were incubated on a medium containing 440 mg/l CaCl₂x2H₂O, 170 mg/l KH₂PO₄, 1.9 g/l KNO₃, 180.7 mg/l MgSO₄, 1.65 g/l NH₄NO₃, 2 mg/l BAP, 40 µg/l Acetosyringone, 20 g/l sucrose, 2 g/l glucose, and 10 g/l agar (pH 6.0) at 21°C for 3 days in the dark.

Calli were subcultured to a medium containing MS salts (Duchefa #0222), 30 g/l sucrose, 1 mg/l GA3, 1 mg/l TDZ, 500 mg/l Timentin, and 10 g/l agar (pH 6.0) and incubated in the dark, at 24°C for 1 week.

To select the transgenic calli, samples were transferred to a medium containing MS salts (Duchefa #0222),, 30 g/l sucrose, 1 mg/l GA3, 1 mg/l TDZ, 500 mg/l Timentin, 10 g/l agar (pH 6.0), and 100 mg/l paromomycin. 1 µM dexamethasone (DEX) was included into the selection media for induction of BnBBM-GR protein.

The principle of the inducible expression of BnBBM is based on a fusion of the rat glucocorticoid receptor domain (GR) to the transcription factor BnBBM (BnBBM-GR). Upon induction with dexamethasone (DEX), the GR receptor domain will change conformation, so that the BnBBM-GR protein will enter the nucleus and generate the desired response.

Regenerating shoots were isolated and propagated in medium containing MS salts, 30 g/l sucrose, 0.25 mg/l benzyladenine (BAP), 100 mg/l kanamycin and 10 g/l agar (pH 6.0). Leaf explants were isolated from the green growing shoots for DNA extraction and PCR analysis, in order to confirm the presence of the transgene. Selected shoots were rooted in medium containing MS salts, 30 g/l sucrose, 6.25 mg/l NAA, and 10 g/l agar (pH 6.0) and transferred to the green house for seed production.

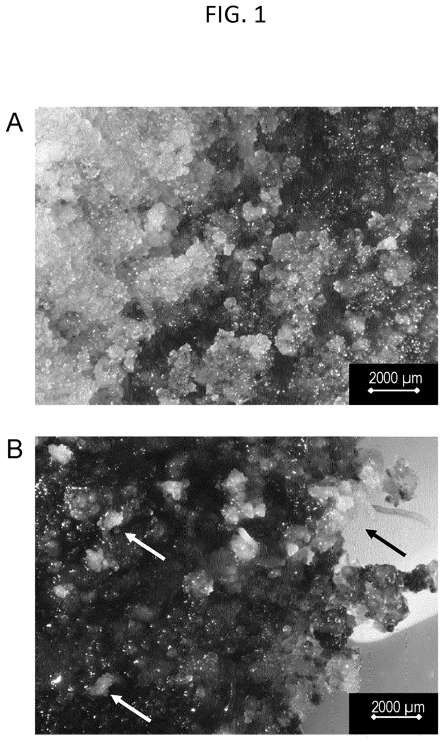

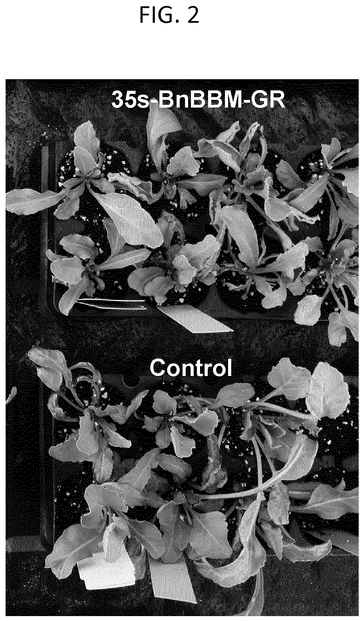

**Figure 1** shows that with DEX many shoots were formed from callus (B), while without DEX only less or no shoot formation took place (A). Transgenic plants obtained from DEX-induced shoot formation show on soil similar morphology compared to non-transgenic wildtype plants (control) (see **Fig. 2**).

Thus, shoot formation from sugar beet callus is increased on DEX media in comparison to DEX free media. The transgenic 35s-BnBBM-GR plants without inducer on soil do not show obvious phenotype.

### 3. BnBBM dramatically increases callus-based transformation efficiency in Beta vulgaris

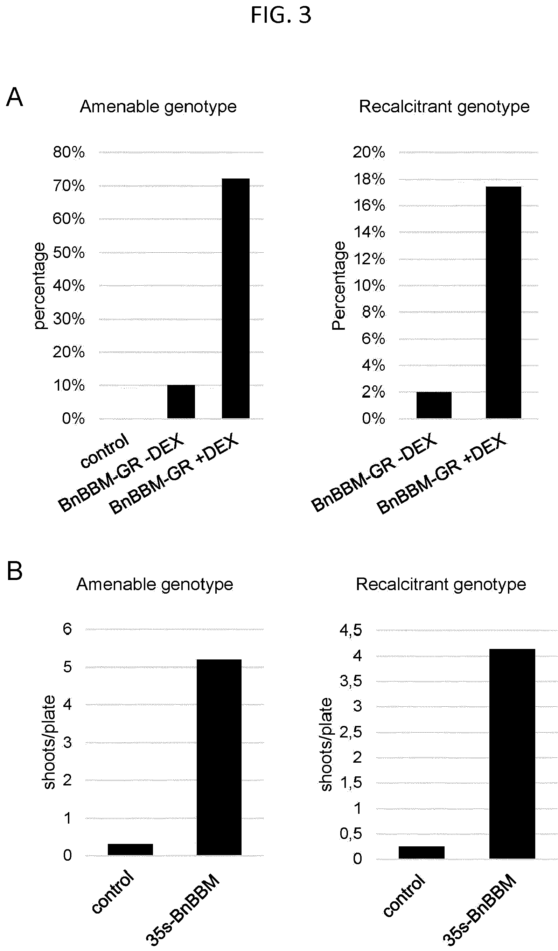

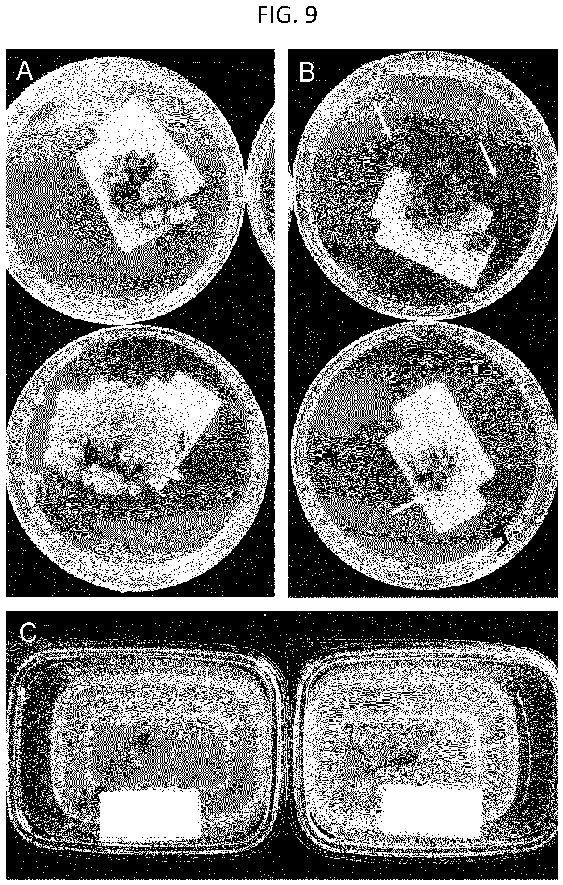

For quantification of transformation rate a) the inducible BnBBM system based on DEX/GR as described above as well as a constitutive overexpression of BnBBM have been tested on an amenable genotype and a recalcitrant genotype. As control served the respective genotype transformed with a vector carrying the fluorescent marker gene tdTomato.
a) The inducible BnBBM system was tested with and without addition of DEX to the media as described above. **Fig. 3A** shows the quantification of regenerated transgenic plants grown from induced shoots. It is apparent that by DEX-induction the frequency of transgenic plant formation is dramatically increased. In both genotypes a seven to eight fold increase in the transformation efficiency is observed, reaching of around 18% in the recalcitrant genotype and up to around 70 % in the amenable genotype.
b) For the constitutive overexpression of BnBBM under the control of the CaMV 35S promoter the transformation efficiency is determined by counting transgenic plants obtained per plate (for detailed transformation protocol see Example 4). Generated transgenic plants are grown from shoots (derived from organogenesis) or embryos produced from callus (derived from embryogenesis) (**Fig. 9**). **Fig. 3B** shows a very similar tendency. The transformation efficiency of the constitutive expression BnBBM has been increased at least ten-fold for the amenable as well as for the recalcitrant genotype.

Thus, due to the combination of BnBBM-GR with DEX callus-based transformation efficiency is dramatically increased in amenable as well as recalcitrant genotypes. Similar results of breaking recalcitrance in sugar beet genotypes where also obtained with constitutive expression.

### 4. Constitutive (over) expression give rise to embryo and seedlings

The same protocol for transformation as described in Example 2 has been used, except that the vector IPR252-pS-01 instead of the vector of IPR232-pS-01 has been used. As negative control, callus as described in Example 2 has been produced, but no agrobacterium infection took place. For shoot induction and propagation, the calli were harvested and transferred to the shoot induction media containing MS salts, 30 g/l sucrose, 1 mg/l GA3, 1 mg/l TDZ and 10 g/l agar (pH 6.0). The calli were incubated at 24°C in the light/dark cycle (16 h/8h) for 1-2 weeks. Regenerated shoots were mounted and cultured in media containing MS salts, 30 g/l sucrose, 0.25 mg/l BAP, and 10g/l agar (pH 6.0) and plants were grown at 24°C in the light/dark cycle (16 h/8h).

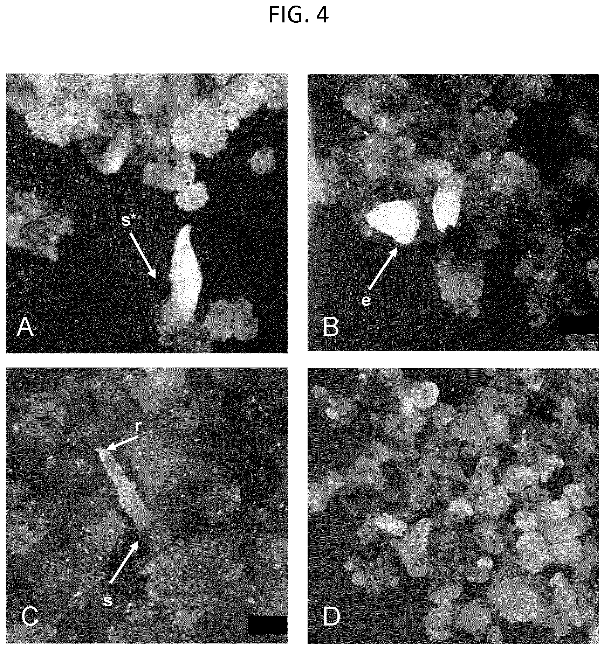

As for the inducible expression, even constitutive expression of BnBBM gives rise to embryo and seedling development (see **Fig. 4B and C**) while standard callus-based transformation for sugar beet only generates shoots (see **Fig. 4A**). This shows that constitutive expression of BnBBM triggers embryogenesis and organogenesis. Many transgenic events could be produced compared to the control (**Fig. 4** **D**).

Thus, constitutive and inducible expression of BnBBM in *Beta vulgaris* develop somatic embryos and shoots while standard callus-based transformation only generates shoots.

### 5. 35s BnBBM plants continuously generate embryos from leaves

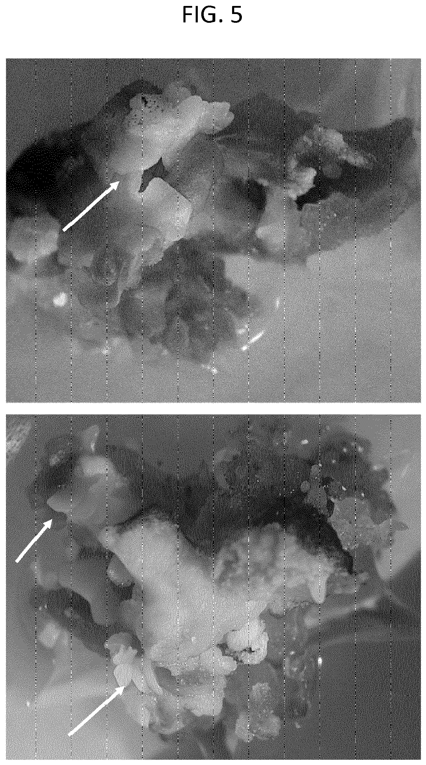

Leaf material derived from different BnBBM overexpressor-lines showed the phenomenon of continuous generation of embryos on the leaves (**Fig. 5**). These embryos can be isolated and grown to whole plants. Thus, this approach might be used for rapid propagation of individual, high value plants.

Thus, plants with a constitutive expression of BnBBM continuously generate embryos from leaves.

### 6. Constitutive (over) expression on hormone-free medium

The same protocol as described in Example 4 has been used, except that the plant hormones TDZ and GA3 have not been supplied to the media.

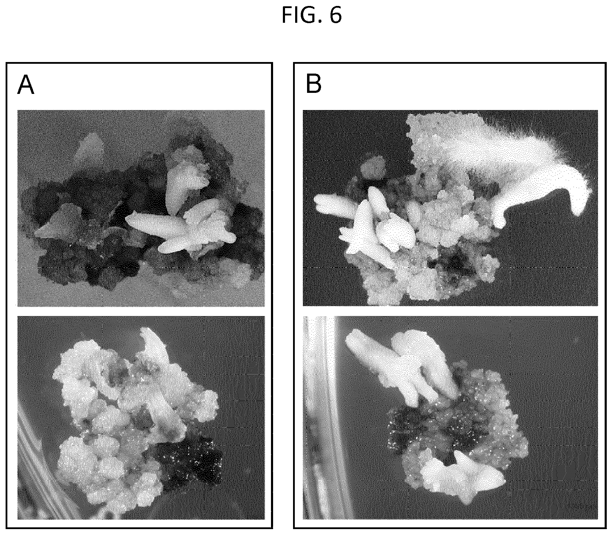

From callus tissue with a constitutive expression of BnBBM mainly embryos can be obtained on hormone free medium (**Fig. 6B**). When such tissue is placed on normal regeneration medium with hormones shoots and embryos are produced (**Fig. 6A**).

### 7. Direct regeneration of shoots from leaf

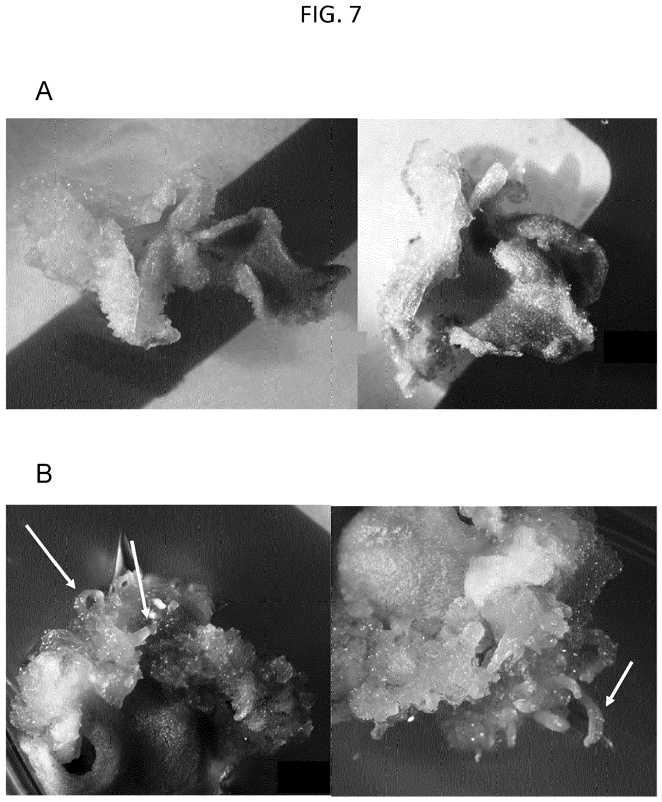

Leaf material was cut from *Beta vulgaris* Dex-inducable 35S-BnBBM-GR transgenic plants as described in Example 2. Leaf material is cultivated on DEX and hormone containing media. BnBBM triggers shoot regeneration from the leaf material after 3 weeks on the media (**Fig. 7**). The generation of embryos has not been observed.

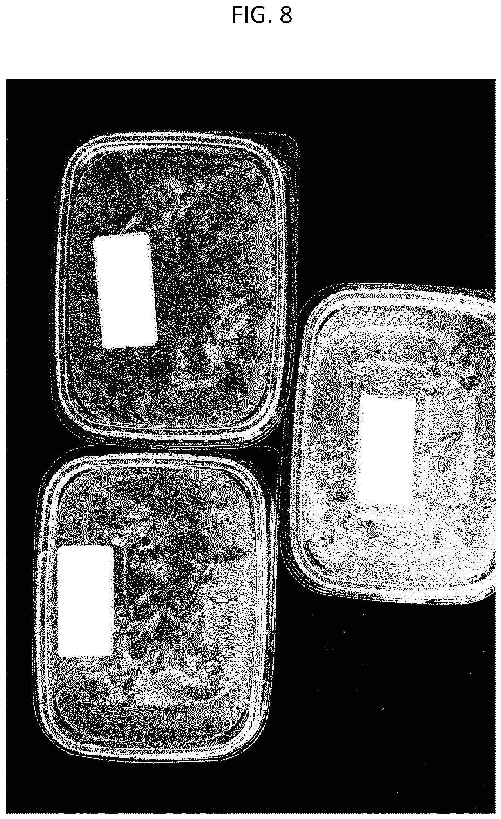

Plants regenerated from the cultivated leaf segments do not show obvious phenotype (**Fig. 8**).

### 8. Co-transformation with inducible BnBBM

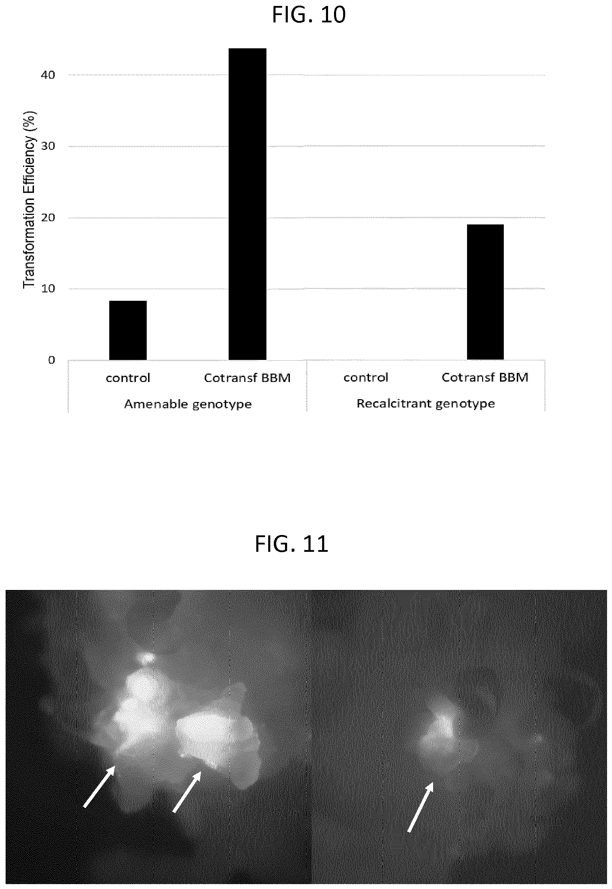

For co-transformation, agrobacteria harbouring each one of the vectors of interest were mixed 1:1 ratio (the vector 35s::BnBBM-GR and the vector 70s-tDT). Calli were inoculated with the obtained Agrobacterium suspension. The callus tissue and the Agrobacterium were incubated at 21 °C for 3 days in the dark and subsequently subcultured as described in Example 2. Co-transformation shows significantly increased regenerative capacities of plant tissue leading to a more efficient co-transformation rate in amenable and recalcitrant genotypes (**Fig. 10**). Further, co-transformants were obtained three weeks earlier if BnBBM has been expressed.

Same result has been obtained for co-transformation with the **35s BnBBM** and the vector 70s-tDT.

The increased efficiency of co-transformation is of particular importance for the co-delivery of genome editing components for the targeted modification of the plant genome of co-transformed plant cells. By co-delivery of a CRISPR/Cpf1 system, SDN-1 events were successfully obtained after co-transformation with BnBBM and said genome editing components.

### 9. The use of BnBBM and the avoidance of hormones effects positively the development of the tap root (storage organ) at T0 level

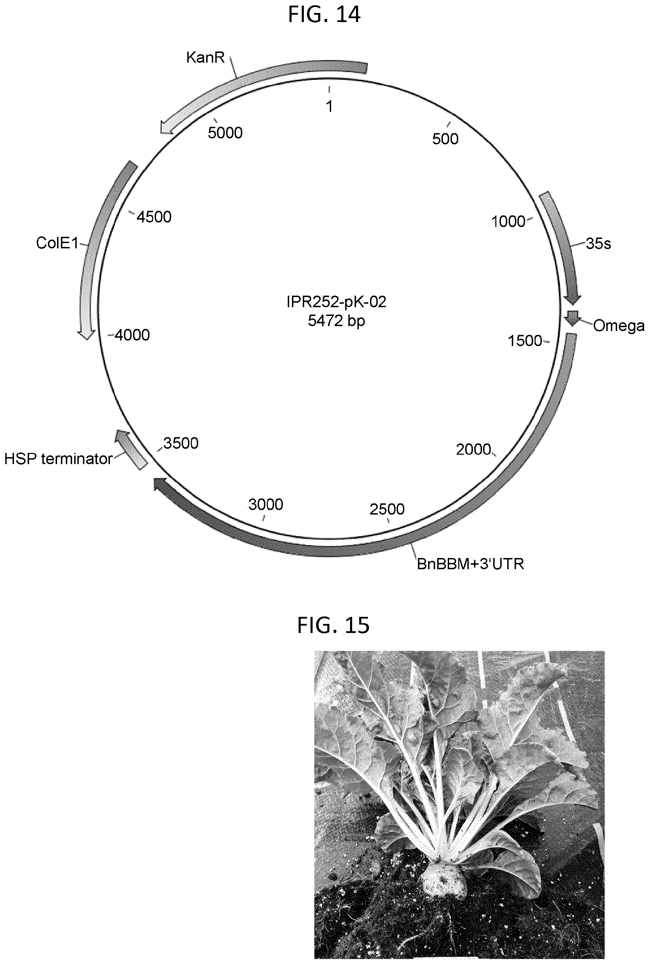

For Beta vulgaris and in particular for sugar beets or red beets, plants regenerated in *vitro* from callus show always an atypical phenotype particularly with respect to the hypocotyl and the roots. Typically, there is not a normal root body developed, so that the T0 generation of e.g. sugar beets or red beets are not usable for testing root-related traits, e.g. sugar accumulation, nematode resistance, rhizomania resistance, etc or promoter expression analysis. Surprisingly, the inventors found that plant regenerated by means of BnBBM, inducible, but without hormones developed a normal tap root. Thereby, phenotyping of root-related traits and analysis of the storage-root ingredients are possible at T0 level. **Fig. 15** shows T0 plants regenerated with inducible BnBBM and without hormone treatment.

### 10. Biolistic delivery method for corn

With the microparticle bombardment, one or more boost genes, booster polypeptides, genome engineering components, and/or transgenes are co-delivered into immature embryos of corn via gold particles. Biolistic delivery has been conducted according the protocol as described in WO 2019/238911 A1.

The boost genes, booster polypeptides, genome engineering components, and/or transgenes can be delivered into target cells using for instances a Bio-Rad PDS-1000/He particle gun or handheld Helios gene gun system. More than one construct can be co-delivered with genome engineering components into target cells simultaneously. For biolistic transformation of corn plants, the plasmid IPR252-pK-02 (**Fig. 14**) was used.

**Fig. 11** shows the biolistic co-delivery of ZmWUS2, BnBBM and fluorescent marker Tdt. One month after bombardment there are fluorescent embryonic structures forming, which indicates that BnBBM combined with ZmWUS2 promotes maize regeneration from immature embryos.

### 11. Plant development on media with and without hormones

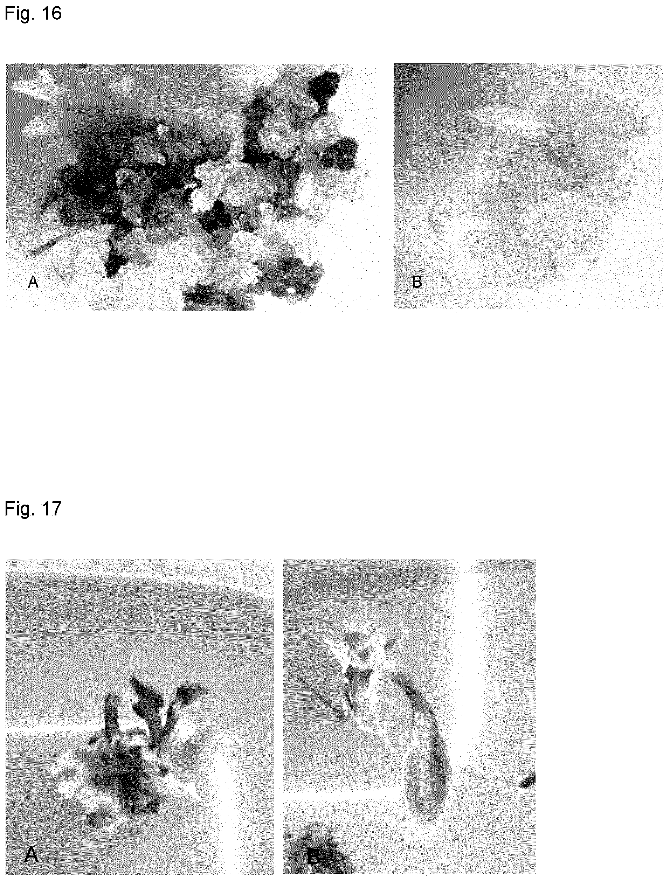

Sugar beet calli were transformed with 35S-BnBBM-GR and placed either on standard medium with hormones (GA3 and BAP) or without hormones. By omitting the hormones GA3 and BAP as medium components somatic embryo formation was clearly more pronounced in comparison to calli cultivated on media with hormones **(****Fig. 16A** and **B****).** In the standard protocol the development of shoots is clearly visible. In subsequent development, seedlings derived from embryos cultivated on hormone free media developed a normal root system **(****Fig. 17B****),** whereas shoots, which were cultivated on media containing hormones, showed no roots **(****Fig. 17A****).** This protocol for plant development is also called one-seedling protocol.

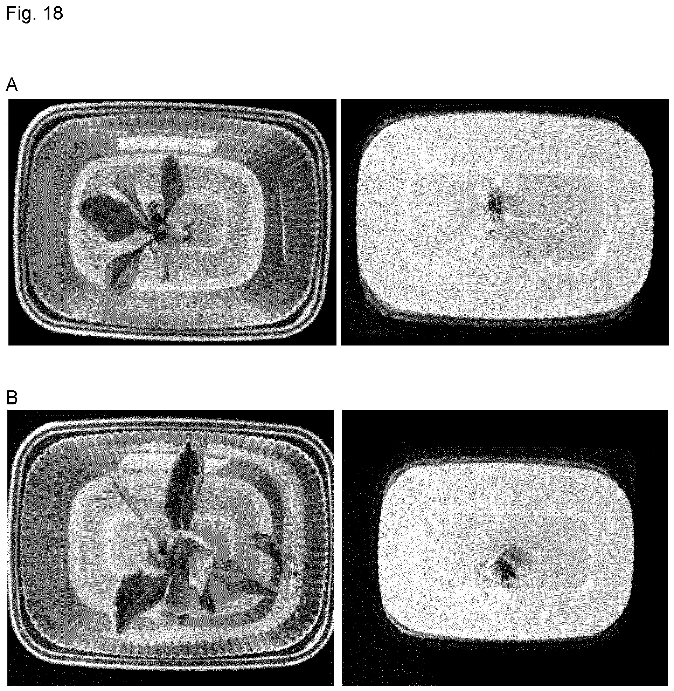

In **Figs. 18A** and **B** plants are shown which have been developed from seedlings grown on hormone free media. These plants have no obvious phenotype and developed a normal root system without artificial induction of root growth.

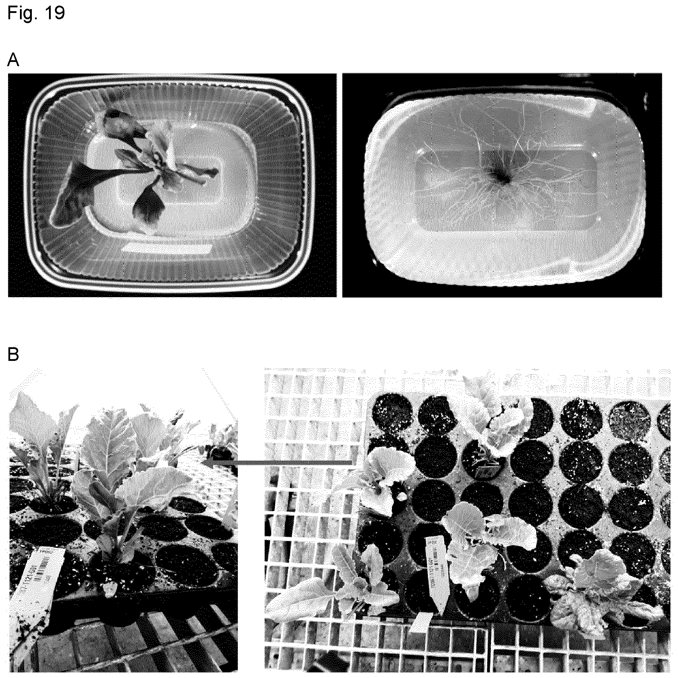

It could be demonstrated that this beneficial effect on BnBBM is achievable by Agrobacterium transformation (see Figs 16-18) as well as by particle bombardment (**Fig. 19**). Transgenic 35S-BnBBM-GR plants obtained by transformation via particle bombardment developed in vitro also a proper root system **(****Fig. 19A****).** After transfer to the greenhouse these plants do not develop an obvious visible phenotype **(****Fig. 19B****)** and have a similar appearance like plants obtained by Agrobacterium mediated transformation.

### 12. Development of plants after transfer to soil and cultivation in the greenhouse

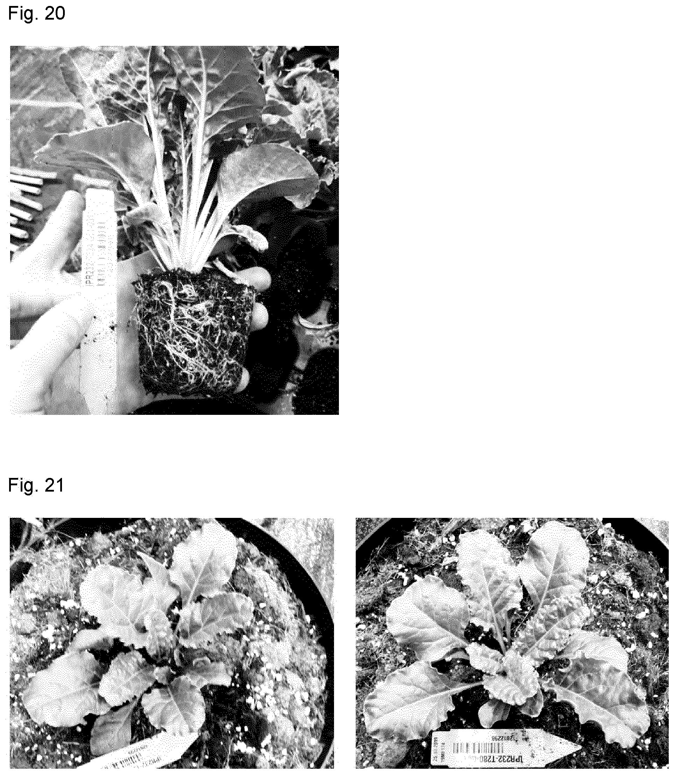

Plants were transferred from hormone free media to soil after one to three months depending on the developmental status and were cultivated for one month in the greenhouse. In **Fig. 20** the root system of such a Beta vulgaris plant is shown which has been developed from a seedling grown on hormone free media. The plant shows a robust root system and also the upper ground part of these plants have developed no obvious phenotype (**Fig. 21****).**

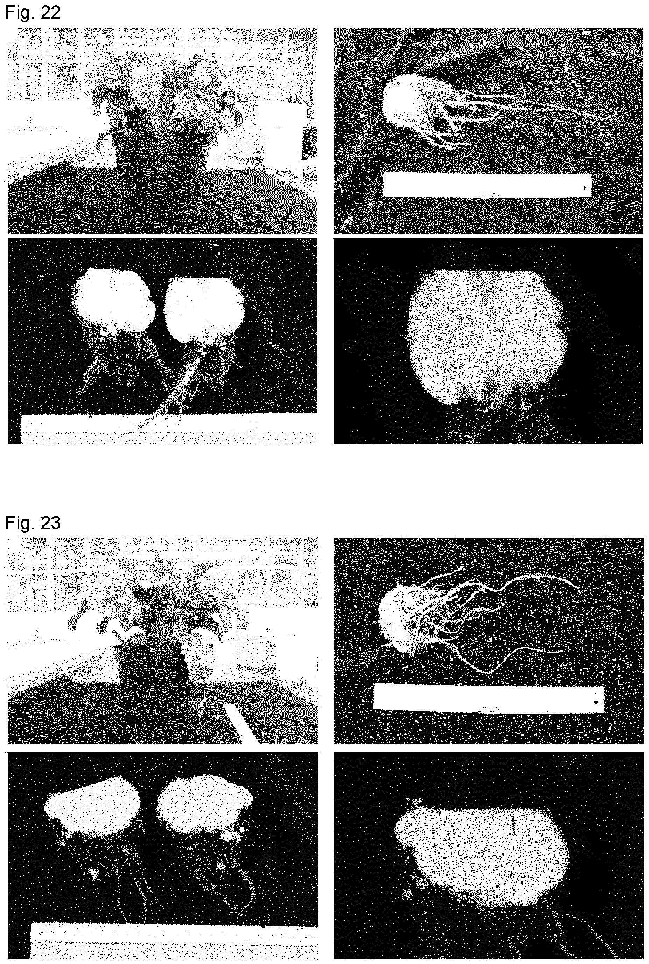

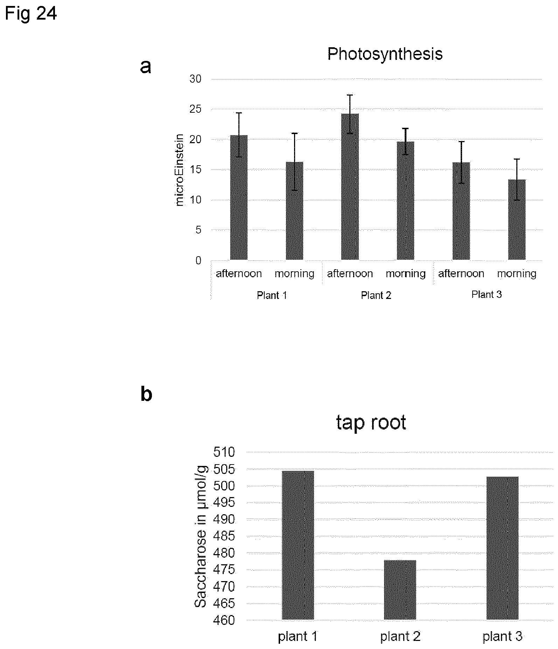

After 4 months in the greenhouse the morphology of the tap root was evaluated. In the cross section, the overall size as well as the overall appearance no obvious abnormalities of the tap root morphology had been observed **(****Figs. 22** and **23****)** Beside the tap root morphology also photosynthesis rate **(****Fig. 24a****),** and sucrose concentration in tap roots have been analysed **(****Fig. 24b****).**

As shown in **Fig. 24a** for three plants the photosynthesis rate has been determined two times a day. All results were approximately in the range of 15 microEinstein to 25 microEinstein.

In **Fig. 24b** the results of the sucrose content in the tap roots are shown in µmol/g for three different plants, the results are between 475 and 505 µmol/g.

### Starch and soluble sugar contents

Extraction: Plant material is extracted with 80% ethanol at 80°C for 1h. Soluble sugars dissolve in ethanol. The extract is dried with help of a vacuum centrifuge and material resolved in water. The remaining extracted material will be ground if it had not already been so for ethanol extraction and again extracted with 0.2N KOH at 95°C for 1h. Starch is partly hydrolyzed and dissolved at this step. After cooling down the samples to room temperature, 1M acetic acid is added to set the pH to 5-6. By addition of appropriate amounts of amyloglucosidase and alpha-amylase and incubation at 37°C for at least 4h and additionally at room temperature overnight starch is degraded to glucose.

Determination of concentrations: Aqueous solutions are used to measure the concentrations of glucose, fructose, sucrose and starch (via glucose) by a coupled optical-enzymatic test using any type of photometer. The measuring buffer contains of 100 mM HEPES pH7,4, 1 mM NAD, 2 mM ATP, 10 mM MgCl₂ and an appropriate amount of glucose-6-phosphate dehydrogenase. After addition of aqueous extract extinction/absorption is measured at a wavelength of l=340nm (0 value). To measure glucose, an appropriate amount of hexokinase is added and measured long enough to convert all glucose to glucose-6-phosphate and fructose to fructose-6-phosphate. To measure fructose, an appropriate amount of phosphoglucoisomerase is added to convert all fructose-6-phosphate to glucose-6-phosphate. To measure sucrose, an appropriate amount of invertase (beta-fructosidase) is added to fully split sucrose into glucose and fructose which, in turn, are further converted to glucose-6-phosphate intermediary. Whenever in this series of enzyme reactions glucose-6-phosphate is produced, it is further oxidized to gluconate-6-phosphate with a simultaneous reduction of NAD to NADH. The latter reduction gives the read-out for the respective concentrations as NAD does not absorb light of 340 nm wavelength whereas NADH does. With help of the extinction coefficient of NADH at 340 nm and the Lambert-Beer quotation, the concentration of NADH in solution can be calculated. Knowing that glucose and fructose give rise to an equimolar concentration of NADH, both sugar concentrations can be calculated. For 1 mole of sucrose, 2 moles of NADH are formed. By also knowing the sample weight and volumes of liquids used in the process, the amounts of glucose, fructose, sucrose and starch (as mol hexose unit per mass) can be calculated form the concentrations measured.

The described data for plants derived from a hormone free transformation are in the normal range of seed derived plants.

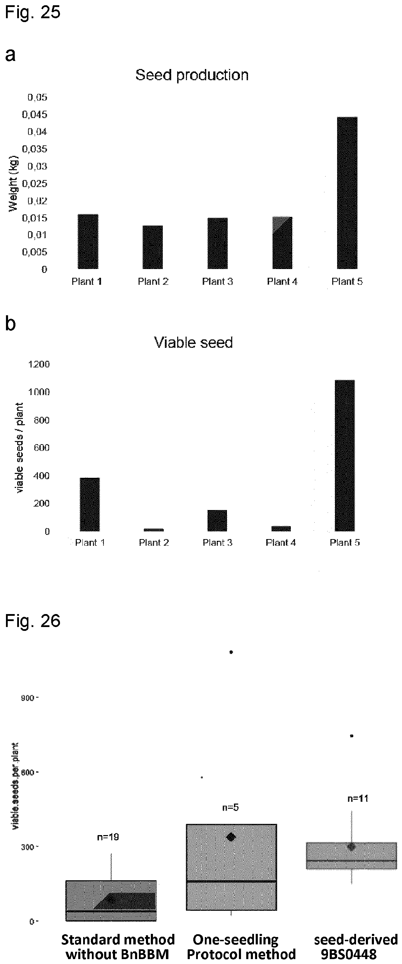

The transgenic 35S-BnBBM-GR plants obtained by the hormone free transformation protocol show a normal development and a properly developed root system, furthermore these plants are also fertile as shown in **Fig. 25****.** The seed weight in kg per plants has been determined from 5 different transgenic plants (**Fig. 25**), additionally the viable seeds per plants have been counted (**Fig. 25**).

The results for seed weight are around 0,015 kg/plant for 4 plants, one plant had almost 0,045 kg seeds. Viable seeds have been obtained from all 5 tested plants; however, the results show a certain range with 30 viable seeds per plant to 1100 viable seeds per plant.

In **Fig. 26** the number of viable seeds per plants is compared between plants obtained by a standard Agrobacterium-based transformation without BnBBM, the described hormone free transformation method with BnBBM and plants derived from seeds of SB line 9BS0448 as reference. The presented results show that seed production from transgenic 35S-BnBBM-GR plants is better than from plants obtained by a standard Agrobacterium transformation method.

### 13. Analysis of tap roots from a second batch of plants derived in a hormone free environment.

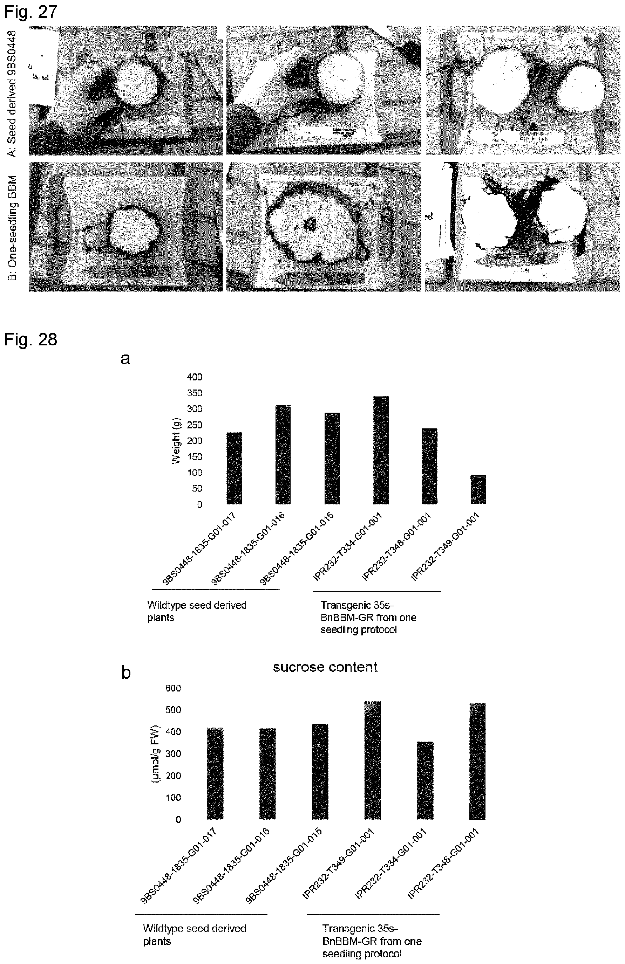

After 4 months of growth in the greenhouse tap roots were harvested from seed derived Beta vulgaris plants or from transgenic 35s-BnBBM-GR plants derived from Example 9. No obvious differences were identified as shown in **Figs. 27** and **28****.**

Furthermore, the tap root weight (**Fig. 28**) as well as the sucrose content (µmol/g FW (fresh weight)) (**Fig. 28**) were analysed. In **Fig. 28a** the root weight of WT (wild type) seed derived plants and transgenic 35s-BnBBM-GR from the hormone free approach are shown. Results vary between approx. 100-350 g. For the sucrose content values between 350 - 520 µmol/g FW have been determined.

Overall, these results show that the tap root weight as well as the sucrose content of the transgenic plants from the described hormone free protocol are comparable with seed derived wild type controls.

### Sequences:

| **SEQ ID NO:** | **Name** | **Description** |
|---|---|---|
| 1 | BnBBM | cDNA of *Brassica napus* Babyboom |
| 2 | BnBBM | protein of *Brassica napus* Babyboom |
| 3 | BnBBM+3'UTR | DNA sequence of *Brassica napus* Babyboom with 3'UTR |
| 4 | BnBBM+GR | DNA sequence of *Brassica napus* Babyboom with rat glucocorticoid receptor domain |
| 5 | pZFNnptll | Vector sequence |
| 6 | NPT | Neomycin phosphotransferase |
| 7 | tDt | tdTomato, orange fluorescent protein |
| 8 | d35S | double 35s promoter |
| 9 | ColE1 ori | Col E1 origin of replication |
| 10 | pVS1 REP | pVS1 Replicon |
| 11 | aadA | Aminoglycoside 3'-adenyl transferase from *Escherichia coli* |
| 12 | ZmWUS2 | cDNA sequence of ZmWUS2 |
| 13 | ZmWUS2 | protein sequence of ZmWUS2 |
| 14 | IPR232-pS-01 | Full length vector |
| 15 | IPR252-pS-01 | Full length vector |
| 16 | IPR 252-pK-02 | Full length vector |

## Claims

1. A method of promoting somatic embryogenesis or organogenesis of *Beta vulgaris*, comprising the following steps:
(A1)
(a) inducing callus formation from at least one *Beta vulgaris* plant cell, and
(b) introducing into the at least one plant cell to be used in step (a) or into the at least one cell of the callus obtained in step (a) an expression cassette comprising a coding nucleotide sequence selected from
(i) a nucleotide sequence of SEQ ID NO: 1, or a sequence which is at least 80% identical to the sequence of SEQ ID NO: 1; and
(ii) a nucleotide sequence encoding a polypeptide having the amino acid sequence of SEQ ID NO: 2, or a sequence which is at least 80% identical to the sequence of SEQ ID NO: 2,
wherein the nucleotide sequence is operably linked to a heterologous constitutive regulatory element or a heterologous inducible regulatory element; or
(A2) providing an explant which is from a tissue of a *Beta vulgaris* plant and comprises at least one cell comprising an expression cassette comprising a coding nucleotide sequence selected from
(i) a nucleotide sequence of SEQ ID NO: 1, or a sequence which is at least 80% identical to the sequence of SEQ ID NO: 1; and
(ii) a nucleotide sequence encoding a polypeptide having the amino acid sequence of SEQ ID NO: 2, or an amino acid sequence which is at least 80% identical to the sequence of SEQ ID NO: 2,
wherein the nucleotide sequence is operably linked to a heterologous constitutive regulatory element; or
(A3) providing an explant which is from a tissue of a *Beta vulgaris* plant and comprises at least one cell comprising an expression cassette comprising a coding nucleotide sequence selected from
(i) a nucleotide sequence of SEQ ID NO: 1, or a sequence which is at least 80% identical to the sequence of SEQ ID NO: 1; and
(ii) a nucleotide sequence encoding a polypeptide having the amino acid sequence of SEQ ID NO: 2, or an amino acid sequence which is at least 80% identical to the sequence of SEQ ID NO: 2,
wherein the nucleotide sequence is operably linked to a heterologous inducible regulatory element; and
(B1) cultivating the callus obtained in step (A1) under conditions promoting growth of embryos and/or shoots out of the callus, wherein in the callus the polypeptide is expressed from the expression cassette constitutively or upon induction of the heterologous inducible expression system; or
(B2) cultivating the explant of step (A2) under conditions promoting growth of embryos out of the explant, wherein in the explant the polypeptide is expressed from the expression cassette constitutively; or
(B3) cultivating the explant of step (A3) under conditions promoting growth of shoots out of the explant, wherein in the explant the polypeptide is expressed from the expression cassette upon induction of the heterologous inducible expression system.

2. The method of claim 1, wherein the heterologous constitutive regulatory element is a constitutive promoter.

3. The method of claim 1 or 2, wherein the heterologous inducible regulatory element is an inducible promoter or an inducible expression system.

4. The method of any one of claim 1 to 3, wherein in step (B1) and (B2), embryos are directly grown from the callus or the explant.

5. The method of any one of claim 1 to 4, wherein in step (B1) and (B3), shoots are directly grown from the callus or the explant.

6. The method of any one of claims 1 to 5, wherein the at least one *Beta vulgaris* plant cell of step (A1) is a somatic or embryonic cell and preferably an explant or a part thereof isolated from a plant.

7. The method of any one of claims 1 to 6, wherein the explant of step (A2) or (A3) is obtained from leaf tissue, hypocotyl tissue, floral tissue, shoot tissue, shoot apical meristem or root tip tissue.

8. The method of any one of claims 1 to 7, wherein in step (A1)(b) introducing the expression cassette results in a stable integration thereof into the genome of the at least one plant cell or in a progeny cell thereof; and/or wherein the expression cassette in the at least one plant cell of step (A2) and/or (A3) is stably integrated into the genome of the at least one plant cell or in a progeny cell thereof.

9. The method according to any one of the preceding claims, wherein cultivating in step (B1), (B2) and/or (B3) is carried out in a medium free of plant hormones.

10. The method according to any one of claims 1-9, further comprising a step of
(C1) introducing at least one nucleotide sequence of interest into the at least one plant cell or a predecessor thereof to be used in step (A1)(a), into at least one cell of the callus obtained in step (A1)(a) which itself or a progeny thereof is then to be used in step (A1)(b) or has been used in step (A1)(b), into the at least one plant cell of step (A2) or (A3), and/or
(C2) modifying the genome of the at least one plant cell or a predecessor thereof to be used in step (A1)(a), of the at least one cell of the callus obtained in step (A1)(a) which itself or a progeny thereof is then to be used in step (A1)(b) or has been used in step (A1)(b), into the at least one plant cell of step (A2) or (A3) by introducing into said cell a single stranded DNA break (SSB) inducing enzyme or a double stranded DNA break (DSB) inducing enzyme which preferably recognize a predetermined site in the genome of said cell, and optionally a repair nucleic acid molecule, or a single stranded DNA break (SSB) inducing enzyme which preferably recognizes a predetermined site in the genome of said cell and is fused to a base editor enzyme,
wherein the modification of said genome is selected from
I. a replacement of at least one nucleotide;
II. a deletion of at least one nucleotide;
III. an insertion of at least one nucleotide; or
IV. any combination of I. - III.

11. A method of production of a *Beta vulgaris* plant comprising
somatic embryogenesis or organogenesis according to the method of any one of claims 1-9, optionally transformation or genomic modification according to claim 10 (C1) or (C2), and regenerating a *Beta vulgaris* plant from an embryo and/or a shoot resulting from step (B1), (B2) or (B3) of the method of any one of claims 1-9, in particular a transgenic plant resulting from step (C1) of the method of claim 10 and/or a modified plant resulting from step (C2) of the method of claim 10.

12. A transgenic *Beta vulgaris* plant obtained or obtainable by the method of claim 11 or a progeny plant thereof.

13. A modified *Beta vulgaris* plant obtained or obtainable by the method of claim 11, or a progeny plant thereof.

14. A plant cell or a seed of the plant of claim 12, wherein the plant cell or the seed comprises the at least one nucleotide sequence of interest as transgene.

15. A plant cell or a seed of the plant of claim 13, wherein the plant cell or the seed comprises the modification in the genome.


SEQUENCE LISTING

<110>  KWS SAAT SE & Co. KGaA

<120>  PROMOTING REGENERATION AND TRANSFORMATION IN PLANTS

<130>  KWS0348EP

<160>  16

<170>  PatentIn version 3.5

<210>  1
<211>  1737
<212>  DNA
<213>  Artificial Sequence

<220>
<223>  cDNA of Brassica napus Babyboom

<400>  1
atggataata actggttagg cttttctctc tctccttatg aacaaaatca ccatcgtaag      60

gacgtctact cttccaccac cacaaccgtc gtagatgtcg ccggagagta ctgttacgat     120

ccgaccgctg cctccgatga gtcttcagcc atccaaacat cgtttccttc tccctttggt     180

gtcgtcgtcg atgctttcac cagagacaac aatagtcact cccgagattg ggacatcaat     240

ggttgtgcat gcaataacat ccacaacgat gagcaagatg gaccaaagct tgagaatttc     300

cttggccgca ccaccacgat ttacaacacc aacgaaaacg ttggagatgg aagtggaagt     360

ggctgttatg gaggaggaga cggtggtggt ggctcactag gactttcgat gataaagaca     420

tggctgagaa atcaacccgt ggataatgtt gataatcaag aaaatggcaa tgctgcaaaa     480

ggcctgtccc tctcaatgaa ctcatctact tcttgtgata acaacaacga cagcaataac     540

aacgttgttg cccaagggaa gactattgat gatagcgttg aagctacacc gaagaaaact     600

attgagagtt ttggacagag gacgtctata taccgcggtg ttacaaggca tcggtggaca     660

ggaagatatg aggcacattt atgggataat agttgtaaaa gagaaggcca aacgcgcaaa     720

ggaagacaag tttatttggg aggttatgac aaagaagaaa aagcagctag ggcttatgat     780

ttagccgcac tcaagtattg gggaaccacc actactacta acttccccat gagcgaatat     840

gaaaaagagg tagaagagat gaagcacatg acaaggcaag agtatgttgc ctcactgcgc     900

aggaaaagta gtggtttctc tcgtggtgca tcgatttatc gtggagtaac aagacatcac     960

caacatggaa gatggcaagc taggatagga agagtcgccg gtaacaaaga cctctacttg    1020

ggaacttttg gcacacaaga agaagctgca gaggcatacg acattgcggc catcaaattc    1080

agaggattaa ccgcagtgac taacttcgac atgaacagat acaacgttaa agcaatcctc    1140

gaaagcccta gtcttcctat tggtagcgcc gcaaaacgtc tcaaggaggc taaccgtccg    1200

gttccaagta tgatgatgat cagtaataac gtttcagaga gtgagaatag tgctagcggt    1260

tggcaaaacg ctgcggttca gcatcatcag ggagtagatt tgagcttatt gcaccaacat 1320

caagagaggt acaatggtta ttattacaat ggaggaaact tgtcttcgga gagtgctagg 1380

gcttgtttca aacaagagga tgatcaacac catttcttga gcaacacgca gagcctcatg 1440

actaatatcg atcatcaaag ttctgtttcg gatgattcgg ttactgtttg tggaaatgtt 1500

gttggttatg gtggttatca aggatttgca gccccggtta actgcgatgc ctacgctgct 1560

agtgagtttg attataacgc aagaaaccat tattactttg ctcagcagca gcagacccag 1620

cagtcgccag gtggagattt tcccgcggca atgacgaata atgttggctc taatatgtat 1680

taccatgggg aaggtggtgg agaagttgct ccaacattta cagtttggaa cgacaat 1737

<210> 2
<211> 579
<212> PRT
<213> Brassica napus

<400> 2

Met Asp Asn Asn Trp Leu Gly Phe Ser Leu Ser Pro Tyr Glu Gln Asn
1               5                   10                  15

His His Arg Lys Asp Val Tyr Ser Ser Thr Thr Thr Thr Val Val Asp
            20                  25                  30

Val Ala Gly Glu Tyr Cys Tyr Asp Pro Thr Ala Ala Ser Asp Glu Ser
        35                  40                  45

Ser Ala Ile Gln Thr Ser Phe Pro Ser Pro Phe Gly Val Val Val Asp
    50                  55                  60

Ala Phe Thr Arg Asp Asn Asn Ser His Ser Arg Asp Trp Asp Ile Asn
65                  70                  75                  80

Gly Cys Ala Cys Asn Asn Ile His Asn Asp Glu Gln Asp Gly Pro Lys
                85                  90                  95

Leu Glu Asn Phe Leu Gly Arg Thr Thr Thr Ile Tyr Asn Thr Asn Glu
            100                 105                 110

Asn Val Gly Asp Gly Ser Gly Ser Gly Cys Tyr Gly Gly Gly Asp Gly
        115                 120                 125

Gly Gly Gly Ser Leu Gly Leu Ser Met Ile Lys Thr Trp Leu Arg Asn
    130                 135                 140

Gln Pro Val Asp Asn Val Asp Asn Gln Glu Asn Gly Asn Ala Ala Lys
145                 150                 155                 160

Gly Leu Ser Leu Ser Met Asn Ser Ser Thr Ser Cys Asp Asn Asn Asn
                165                 170                 175

Asp Ser Asn Asn Asn Val Val Ala Gln Gly Lys Thr Ile Asp Asp Ser
            180                 185                 190

Val Glu Ala Thr Pro Lys Lys Thr Ile Glu Ser Phe Gly Gln Arg Thr
        195                 200                 205

Ser Ile Tyr Arg Gly Val Thr Arg His Arg Trp Thr Gly Arg Tyr Glu
    210                 215                 220

Ala His Leu Trp Asp Asn Ser Cys Lys Arg Glu Gly Gln Thr Arg Lys
225                 230                 235                 240

Gly Arg Gln Val Tyr Leu Gly Gly Tyr Asp Lys Glu Glu Lys Ala Ala
                245                 250                 255

Arg Ala Tyr Asp Leu Ala Ala Leu Lys Tyr Trp Gly Thr Thr Thr Thr
            260                 265                 270

Thr Asn Phe Pro Met Ser Glu Tyr Glu Lys Glu Val Glu Glu Met Lys
        275                 280                 285

His Met Thr Arg Gln Glu Tyr Val Ala Ser Leu Arg Arg Lys Ser Ser
    290                 295                 300

Gly Phe Ser Arg Gly Ala Ser Ile Tyr Arg Gly Val Thr Arg His His
305                 310                 315                 320

Gln His Gly Arg Trp Gln Ala Arg Ile Gly Arg Val Ala Gly Asn Lys
                325                 330                 335

Asp Leu Tyr Leu Gly Thr Phe Gly Thr Gln Glu Glu Ala Ala Glu Ala
            340                 345                 350

Tyr Asp Ile Ala Ala Ile Lys Phe Arg Gly Leu Thr Ala Val Thr Asn
        355                 360                 365

Phe Asp Met Asn Arg Tyr Asn Val Lys Ala Ile Leu Glu Ser Pro Ser
    370                 375                 380

Leu Pro Ile Gly Ser Ala Ala Lys Arg Leu Lys Glu Ala Asn Arg Pro
385                 390                 395                 400

Val Pro Ser Met Met Met Ile Ser Asn Asn Val Ser Glu Ser Glu Asn
                405                 410                 415

Ser Ala Ser Gly Trp Gln Asn Ala Ala Val Gln His His Gln Gly Val
            420                 425                 430

Asp Leu Ser Leu Leu His Gln His Gln Glu Arg Tyr Asn Gly Tyr Tyr
        435                 440                 445

Tyr Asn Gly Gly Asn Leu Ser Ser Glu Ser Ala Arg Ala Cys Phe Lys
    450                 455                 460

Gln Glu Asp Asp Gln His His Phe Leu Ser Asn Thr Gln Ser Leu Met
465                 470                 475                 480

Thr Asn Ile Asp His Gln Ser Ser Val Ser Asp Asp Ser Val Thr Val
                485                 490                 495

Cys Gly Asn Val Val Gly Tyr Gly Gly Tyr Gln Gly Phe Ala Ala Pro
            500                 505                 510

Val Asn Cys Asp Ala Tyr Ala Ala Ser Glu Phe Asp Tyr Asn Ala Arg
        515                 520                 525

Asn His Tyr Tyr Phe Ala Gln Gln Gln Gln Thr Gln Gln Ser Pro Gly
    530                 535                 540

Gly Asp Phe Pro Ala Ala Met Thr Asn Asn Val Gly Ser Asn Met Tyr
545                 550                 555                 560

Tyr His Gly Glu Gly Gly Gly Glu Val Ala Pro Thr Phe Thr Val Trp
                565                 570                 575

Asn Asp Asn

<210>  3
<211>  1974
<212>  DNA
<213>  Artificial Sequence

<220>
<223>  cDNA sequence of Brassica napus Babyboom with 3\222UTR

<400>  3
atggataata actggttagg cttttctctc tctccttatg aacaaaatca ccatcgtaag      60

gacgtctact cttccaccac cacaaccgtc gtagatgtcg ccggagagta ctgttacgat     120

ccgaccgctg cctccgatga gtcttcagcc atccaaacat cgtttccttc tccctttggt     180

gtcgtcgtcg atgctttcac cagagacaac aatagtcact cccgagattg ggacatcaat     240

ggttgtgcat gcaataacat ccacaacgat gagcaagatg gaccaaagct tgagaatttc 300

cttggccgca ccaccacgat ttacaacacc aacgaaaacg ttggagatgg aagtggaagt 360

ggctgttatg gaggaggaga cggtggtggt ggctcactag gactttcgat gataaagaca 420

tggctgagaa atcaacccgt ggataatgtt gataatcaag aaaatggcaa tgctgcaaaa 480

ggcctgtccc tctcaatgaa ctcatctact tcttgtgata acaacaacga cagcaataac 540

aacgttgttg cccaagggaa gactattgat gatagcgttg aagctacacc gaagaaaact 600

attgagagtt ttggacagag gacgtctata taccgcggtg ttacaaggca tcggtggaca 660

ggaagatatg aggcacattt atgggataat agttgtaaaa gagaaggcca aacgcgcaaa 720

ggaagacaag tttatttggg aggttatgac aaagaagaaa aagcagctag ggcttatgat 780

ttagccgcac tcaagtattg gggaaccacc actactacta acttccccat gagcgaatat 840

gaaaaagagg tagaagagat gaagcacatg acaaggcaag agtatgttgc ctcactgcgc 900

aggaaaagta gtggtttctc tcgtggtgca tcgatttatc gtggagtaac aagacatcac 960

caacatggaa gatggcaagc taggatagga agagtcgccg gtaacaaaga cctctacttg 1020

ggaacttttg gcacacaaga agaagctgca gaggcatacg acattgcggc catcaaattc 1080

agaggattaa ccgcagtgac taacttcgac atgaacagat acaacgttaa agcaatcctc 1140

gaaagcccta gtcttcctat tggtagcgcc gcaaaacgtc tcaaggaggc taaccgtccg 1200

gttccaagta tgatgatgat cagtaataac gtttcagaga gtgagaatag tgctagcggt 1260

tggcaaaacg ctgcggttca gcatcatcag ggagtagatt tgagcttatt gcaccaacat 1320

caagagaggt acaatggtta ttattacaat ggaggaaact tgtcttcgga gagtgctagg 1380

gcttgtttca aacaagagga tgatcaacac catttcttga gcaacacgca gagcctcatg 1440

actaatatcg atcatcaaag ttctgtttcg gatgattcgg ttactgtttg tggaaatgtt 1500

gttggttatg gtggttatca aggatttgca gccccggtta actgcgatgc ctacgctgct 1560

agtgagtttg attataacgc aagaaaccat tattactttg ctcagcagca gcagacccag 1620

cagtcgccag gtggagattt tcccgcggca atgacgaata atgttggctc taatatgtat 1680

taccatgggg aaggtggtgg agaagttgct ccaacattta cagtttggaa cgacaattag 1740

aaaaaatagt taaagatctt tagttatatg cgttgttgtg tgctggtgaa cagtgtgata 1800

ctttgattat gtttttttct ttctcttttt ctttttcttg gttaatttct taagacttat 1860

ttttagtttc cattagttgg ataaattttc agactaaaaa aaaaaaaaaa aaaaaactcg 1920

aggggggccc cggtaccgag ctcgaatttc cccgatcgtt caaacatttg tacc 1974

<210> 4
<211> 2603
<212> DNA
<213> Artificial Sequence

<220>
<223>  DNA sequence of Brassica napus Babyboom with rat glucocorticoid
       receptor domain

<400>  4
atggataata actggttagg cttttctctc tctccttatg aacaaaatca ccatcgtaag      60

gacgtctact cttccaccac cacaaccgtc gtagatgtcg ccggagagta ctgttacgat     120

ccgaccgctg cctccgatga gtcttcagcc atccaaacat cgtttccttc tccctttggt     180

gtcgtcgtcg atgctttcac cagagacaac aatagtcact cccgagattg ggacatcaat     240

ggttgtgcat gcaataacat ccacaacgat gagcaagatg gaccaaagct tgagaatttc     300

cttggccgca ccaccacgat ttacaacacc aacgaaaacg ttggagatgg aagtggaagt     360

ggctgttatg gaggaggaga cggtggtggt ggctcactag gactttcgat gataaagaca     420

tggctgagaa atcaacccgt ggataatgtt gataatcaag aaaatggcaa tgctgcaaaa     480

ggcctgtccc tctcaatgaa ctcatctact tcttgtgata acaacaacga cagcaataac     540

aacgttgttg cccaagggaa gactattgat gatagcgttg aagctacacc gaagaaaact     600

attgagagtt ttggacagag gacgtctata taccgcggtg ttacaaggca tcggtggaca     660

ggaagatatg aggcacattt atgggataat agttgtaaaa gagaaggcca aacgcgcaaa     720

ggaagacaag tttatttggg aggttatgac aaagaagaaa aagcagctag ggcttatgat     780

ttagccgcac tcaagtattg gggaaccacc actactacta acttccccat gagcgaatat     840

gaaaaagagg tagaagagat gaagcacatg acaaggcaag agtatgttgc ctcactgcgc     900

aggaaaagta gtggtttctc tcgtggtgca tcgatttatc gtggagtaac aagacatcac     960

caacatggaa gatggcaagc taggatagga agagtcgccg gtaacaaaga cctctacttg    1020

ggaacttttg gcacacaaga agaagctgca gaggcatacg acattgcggc catcaaattc    1080

agaggattaa ccgcagtgac taacttcgac atgaacagat acaacgttaa agcaatcctc    1140

gaaagcccta gtcttcctat tggtagcgcc gcaaaacgtc tcaaggaggc taaccgtccg    1200

gttccaagta tgatgatgat cagtaataac gtttcagaga gtgagaatag tgctagcggt    1260

tggcaaaacg ctgcggttca gcatcatcag ggagtagatt tgagcttatt gcaccaacat    1320

caagagaggt acaatggtta ttattacaat ggaggaaact tgtcttcgga gagtgctagg    1380

gcttgtttca aacaagagga tgatcaacac catttcttga gcaacacgca gagcctcatg    1440

actaatatcg atcatcaaag ttctgtttcg gatgattcgg ttactgtttg tggaaatgtt    1500

gttggttatg gtggttatca aggatttgca gccccggtta actgcgatgc ctacgctgct    1560

agtgagtttg attataacgc aagaaaccat tattactttg ctcagcagca gcagacccag    1620

cagtcgccag gtggagattt tcccgcggca atgacgaata atgttggctc taatatgtat    1680

taccatgggg aaggtggtgg agaagttgct ccaacattta cagtttggaa cgacaatgga    1740

tctacaaaga aaaaaatcaa agggattcag caagccactg caggagtctc acaagacact 1800

tcggaaaatc ctaacaaaac aatagttcct gctgcattac cacagctcac ccctaccttg 1860

gtgtcactgc tggaggtgat tgaacccgag gtgttgtatg caggatatga tagctctgtt 1920

ccagattcag cgtggagaat tatgaccaca ctcaacatgt taggtgggcg tcaagtgatt 1980

gcagcagtga aatgggcaaa ggcgatacca ggcttcagaa acttacacct ggatgaccaa 2040

atgaccctgc tacagtactc atggatgttt ctcatggcat ttgccctggg ttggagatca 2100

tacagacaat caagtggaaa cctgctctgc tttgctcctg atctgattat taatgagcag 2160

agaatgtctc taccctgcat gtatgaccaa tgtaaacaca tgctgtttgt ctcctctgaa 2220

ttacaaagat tgcaggtatc ctatgaagag tatctctgta tgaaaacctt actgcttctc 2280

tcctcagttc ctaaggaagg tctgaagagc caagagttat ttgatgagat tcgaatgact 2340

tatatcaaag agctaggaaa agccatcgtc aaaagggaag ggaactccag tcagaactgg 2400

caacggtttt accaactgac aaagcttctg gactccatgc atgaggtggt tgagaatctc 2460

cttacctact gcttccagac atttttggat aagaccatga gtattgagtt cccagagatg 2520

ttagctgaaa tcatcactaa tcagatacca aaatattcaa atggaaatat caaaaagctc 2580

ctgtttcatc aaaaatgaca ccc 2603

<210> 5
<211> 11632
<212> DNA
<213> Artificial Sequence

<220>
<223> vector pZFNnptII

<400> 5
cggccgcgtc gagcgatcta gtaacataga tgacaccgcg cgcgataatt tatcctagtt 60

tgcgcgctat attttgtttt ctatcgcgta ttaaatgtat aattgcggga ctctaatcat 120

aaaaacccat ctcataaata acgtcatgca ttacatgtta attattacat gcttaacgta 180

attcaacaga aattatatga taatcatcgc aagaccggca acaggattca atcttaagaa 240

actttattgc caaatgtttg aacgatcggg gaaattcgag tcgagttttt cagcaagatt 300

ctagacttgt acagctcgtc catgccgtac aggaacaggt ggtggcggcc ctcggagcgc 360

tcgtactgtt ccacgatggt gtagtcctcg ttgtgggagg tgatgtccag cttggtgtcc 420

acgtagtagt agccgggcag ttgcacgggc ttcttggcca tgtagatggt cttgaactcc 480

accaggtagt ggccgccgtc cttcagcttc agggcctggt ggatctcgcc cttcagcacg 540

ccgtcgcggg ggtacaggcg ctcggtggag gcctcccagc ccatggtctt cttctgcatt 600

acggggccgt cgggggggaa gttggtgccg cgcatcttca ccttgtagat cagcgtgccg 660

tcctgcaggg aggagtcctg ggtcacggtc accagaccgc cgtcctcgaa gttcatcacg 720

cgctcccact tgaagccctc ggggaaggac agcttcttgt aatcggggat gtcggcgggg 780

tgcttcacgt acgccttgga gccgtacatg aactgggggg acaggatgtc ccaggcgaag 840

ggcagggggc cgcccttggt caccttcagc ttggcggtct gggtgccctc gtaggggcgg 900

ccctcgccct cgccctcgat ctcgaactcg tggccgttca tggagccctc catgcgcacc 960

ttgaagcgca tgaactcttt gatgacctcc tcgcccttgc tcaccatggt ggcgggatcg 1020

cgccctatcg ttcgtaaatg gtgaaaattt tcagaaaatt gcttttgctt taaaagaaat 1080

gatttaaatt gctgcaatag aagtagaatg cttgattgct tgagattcgt ttgttttgta 1140

tatgttgtgt tgagaggatc cactagttct agagcggccg ccaccgcggt ggagctcgaa 1200

ttctcgaggt cctctccaaa tgaaatgaac ttccttatat agaggaaggg tcttgcgaag 1260

gatagtggga ttgtgcgtca tcccttacgt cagtggagat atcacatcaa tccacttgct 1320

ttgaagacgt ggttggaacg tcttcttttt ccacgatgtt cctcgtgggt gggggtccat 1380

ctttgggacc actgtcggta gaggcatctt gaacgatagc ctttccttta tcgcaatgat 1440

ggcatttgta gaagccatct tccttttcta ctgtcctttc gatgaagtga cagatagctg 1500

ggcaatggaa tccgaggagg tttcccgata ttaccctttg ttgaaaagtc tcaatagccc 1560

tctggtcttc tgagactgta tctttgatat tcttggagta gacgagagtg tcgtgctcca 1620

ccatgtatca catcaatcca cttgctttga agacgtggtt ggaacgtctt ctttttccac 1680

gatgttcctc gtgggtgggg gtccatcttt gggaccactg tcggtagagg catcttgaac 1740

gatagccttt cctttatcgc aatgatggca tttgtagaag ccatcttcct tttctactgt 1800

cctttcgatg aagtgacaga tagctgggca atggaatccg aggaggtttc ccgatattac 1860

cctttgttga aaagtctcaa tagccctctg gtcttctgaa cctgcaggca tgcaagctct 1920

agtaaggcct taagggccag atcttggccc cggtacccga tcagattgtc gtttcccgcc 1980

ttcggtttaa actatcagtg tttgacagga tatattggcg ggtaaaccta agagaaaaga 2040

gcgtttatta gaataatcgg atatttaaaa gggcgtgaaa aggtttatcc gttcgtccat 2100

ttgtatgtgc atgccaacca cagggttccc ctcgggagtg cttggcattc cgtgcgataa 2160

tgacttctgt tcaaccaccc aaacgtcgga aagcctgacg acggagcagc attccaaaaa 2220

gatcccttgg ctcgtctggg tcggctagaa ggtcgagtgg gctgctgtgg cttgatccct 2280

caacgcggtc gcggacgtag cgcagcgccg aaaaatcctc gatcgcaaat ccgacgctgt 2340

cgaaaagcgt gatctgcttg tcgctctttc ggccgacgtc ctggccagtc atcacgcgcc 2400

aaagttccgt cacaggatga tctggcgcga gttgctggat ctcgccttca atccgggtct 2460

gtggcgggaa ctccacgaaa atatccgaac gcagcaagat atcgcggtgc atctcggtct 2520

tgcctgggca gtcgccgccg acgccgttga tgtggacgcc gaaaaggatc taggtgaaga 2580

tcctttttga taatctcatg accaaaatcc cttaacgtga gttttcgttc cactgagcgt 2640

cagaccccgt agaaaagatc aaaggatctt cttgagatcc tttttttctg cgcgtaatct 2700

gctgcttgca aacaaaaaaa ccaccgctac cagcggtggt ttgtttgccg gatcaagagc 2760

taccaactct ttttccgaag gtaactggct tcagcagagc gcagatacca aatactgttc 2820

ttctagtgta gccgtagtta ggccaccact tcaagaactc tgtagcaccg cctacatacc 2880

tcgctctgct aatcctgtta ccagtggctg ctgccagtgg cgataagtcg tgtcttaccg 2940

ggttggactc aagacgatag ttaccggata aggcgcagcg gtcgggctga acggggggtt 3000

cgtgcacaca gcccagcttg gagcgaacga cctacaccga actgagatac ctacagcgtg 3060

agctatgaga aagcgccacg cttcccgaag ggagaaaggc ggacaggtat ccggtaagcg 3120

gcagggtcgg aacaggagag cgcacgaggg agcttccagg gggaaacgcc tggtatcttt 3180

atagtcctgt cgggtttcgc cacctctgac ttgagcgtcg atttttgtga tgctcgtcag 3240

gggggcggag cctatggaaa aacgccagca acgcggcctt tttacggttc ctggcctttt 3300

gctggccttt tgctcacatg ttctttcctg cgttatcccc tgattctgtg gataaccgat 3360

taccgccttt gagtgagctg ataccgctcg ccgcagccga acgaccgagc gcagcgagtc 3420

agtgagcgag gaagcggaag agcgcctgat gcggtatttt ctccttacgc atctgtgcgg 3480

tatttcacac cgcatatggt gcactctcag tacaatctgc tctgatgccg catagttaag 3540

ccagtataca ctccgctatc gctacgtgac tgggtcatgg ctgcgccccg acacccgcca 3600

acacccgctg acgcgccctg acgggcttgt ctgctcccgg catccgctta cagacaagct 3660

gtgaccgtct ccgggagctg catgtgtcag aggttttcac cgtcatcacc gaaacgcgcg 3720

aggcaggggt acgtcgaggt cgatccaacc cctccgctgc tatagtgcag tcggcttctg 3780

acgttcagtg cagccgtctt ctgaaaacga catgtcgcac aagtcctaag ttacgcgaca 3840

ggctgccgcc ctgccctttt cctggcgttt tcttgtcgcg tgttttagtc gcataaagta 3900

gaatacttgc gactagaacc ggagacatta cgccatgaac aagagcgccg ccgctggcct 3960

gctgggctat gcccgcgtca gcaccgacga ccaggacttg accaaccaac gggccgaact 4020

gcacgcggcc ggctgcacca agctgttttc cgagaagatc accggcacca ggcgcgaccg 4080

cccggagctg gccaggatgc ttgaccacct acgccctggc gacgttgtga cagtgaccag 4140

gctagaccgc ctggcccgca gcacccgcga cctactggac attgccgagc gcatccagga 4200

ggccggcgcg ggcctgcgta gcctggcaga gccgtgggcc gacaccacca cgccggccgg 4260

ccgcatggtg ttgaccgtgt tcgccggcat tgccgagttc gagcgttccc taatcatcga 4320

ccgcacccgg agcgggcgcg aggccgccaa ggcgcgaggc gtgaagtttg gcccccgccc 4380

taccctcacc ccggcacaga tcgcgcacgc ccgcgagctg atcgaccagg aaggccgcac 4440

cgtgaaagag gcggctgcac tgcttggcgt gcatcgctcg accctgtacc gcgcacttga 4500

gcgcagcgag gaagtgacgc ccaccgaggc caggcggcgc ggtgccttcc gtgaggacgc 4560

attgaccgag gccgacgccc tggcggccgc cgagaatgaa cgccaagagg aacaagcatg 4620

aaaccgcacc aggacggcca ggacgaaccg tttttcatta ccgaagagat cgaggcggag 4680

atgatcgcgg ccgggtacgt gttcgagccg cccgcgcacg tctcaaccgt gcggctgcat 4740

gaaatcctgg ccggtttgtc tgatgccaag ctcgcggcct ggccggcgag cttggccgct 4800

gaagaaaccg agcgccgccg tctaaaaagg tgatgtgtat ttgagtaaaa cagcttgcgt 4860

catgcggtcg ctgcgtatat gatgcgatga gtaaataaac aaatacgcaa ggggaacgca 4920

tgaaggttat cgctgtactt aaccagaaag gcgggtcagg caagacgacc atcgcaaccc 4980

atctagcccg cgccctgcaa ctcgccgggg ccgatgttct gttagtcgat tccgatcccc 5040

agggcagtgc ccgcgattgg gcggccgtgc gggaagatca accgctaacc gttgtcggca 5100

tcgaccgccc gacgattgac cgcgacgtga aggccatcgg ccggcgcgac ttcgtagtga 5160

tcgacggagc gccccaggcg gcggacttgg ctgtgtccgc gatcaaggca gccgacttcg 5220

tgctgattcc ggtgcagcca agcccttacg acatatgggc caccgccgac ctggtggagc 5280

tggttaagca gcgcattgag gtcacggatg gaaggctaca agcggccttt gtcgtgtcgc 5340

gggcgatcaa aggcacgcgc atcggcggtg aggttgccga ggcgctggcc gggtacgagc 5400

tgcccattct tgagtcccgt atcacgcagc gcgtgagcta cccaggcact gccgccgccg 5460

gcacaaccgt tcttgaatca gaacccgagg gcgacgctgc ccgcgaggtc caggcgctgg 5520

ccgctgaaat taaatcaaaa ctcatttgag ttaatgaggt aaagagaaaa tgagcaaaag 5580

cacaaacacg ctaagtgccg gccgtccgag cgcacgcagc agcaaggctg caacgttggc 5640

cagcctggca gacacgccag ccatgaagcg ggtcaacttt cagttgccgg cggaggatca 5700

caccaagctg aagatgtacg cggtacgcca aggcaagacc attaccgagc tgctatctga 5760

atacatcgcg cagctaccag agtaaatgag caaatgaata aatgagtaga tgaattttag 5820

cggctaaagg aggcggcatg gaaaatcaag aacaaccagg caccgacgcc gtggaatgcc 5880

ccatgtgtgg aggaacgggc ggttggccag gcgtaagcgg ctgggttgtc tgccggccct 5940

gcaatggcac tggaaccccc aagcccgagg aatcggcgtg agcggtcgca aaccatccgg 6000

cccggtacaa atcggcgcgg cgctgggtga tgacctggtg gagaagttga aggcggcgca 6060

ggccgcccag cggcaacgca tcgaggcaga agcacgcccc ggtgaatcgt ggcaagcggc 6120

cgctgatcga atccgcaaag aatcccggca accgccggca gccggtgcgc cgtcgattag 6180

gaagccgccc aagggcgacg agcaaccaga ttttttcgtt ccgatgctct atgacgtggg 6240

cacccgcgat agtcgcagca tcatggacgt ggccgttttc cgtctgtcga agcgtgaccg 6300

acgagctggc gaggtgatcc gctacgagct tccagacggg cacgtagagg tttccgcagg 6360

gccggccggc atggcgagtg tgtgggatta cgacctggta ctgatggcgg tttcccatct 6420

aaccgaatcc atgaaccgat accgggaagg gaagggagac aagcccggcc gcgtgttccg 6480

tccacacgtt gcggacgtac tcaagttctg ccggcgagcc gatggcggaa agcagaaaga 6540

cgacctggta gaaacctgca ttcggttaaa caccacgcac gttgccatgc agcgtacgaa 6600

gaaggccaag aacggccgcc tggtgacggt atccgagggt gaagccttga ttagccgcta 6660

caagatcgta aagagcgaaa ccgggcggcc ggagtacatc gagatcgagc tagctgattg 6720

gatgtaccgc gagatcacag aaggcaagaa cccggacgtg ctgacggttc accccgatta 6780

ctttttgatc gatcccggca tcggccgttt tctctaccgc ctggcacgcc gcgccgcagg 6840

caaggcagaa gccagatggt tgttcaagac gatctacgaa cgcagtggca gcgccggaga 6900

gttcaagaag ttctgtttca ccgtgcgcaa gctgatcggg tcaaatgacc tgccggagta 6960

cgatttgaag gaggaggcgg ggcaggctgg cccgatccta gtcatgcgct accgcaacct 7020

gatcgagggc gaagcatccg ccggttccta atgtacggag cagatgctag ggcaaattgc 7080

cctagcaggg gaaaaaggtc gaaaaggtct ctttcctgtg gatagcacgt acattgggaa 7140

cccaaagccg tacattggga accggaaccc gtacattggg aacccaaagc cgtacattgg 7200

gaaccggtca cacatgtaag tgactgatat aaaagagaaa aaaggcgatt tttccgccta 7260

aaactcttta aaacttatta aaactcttaa aacccgcctg gcctgtgcat aactgtctgg 7320

ccagcgcaca gccgaagagc tgcaaaaagc gcctaccctt cggtcgctgc gctccctacg 7380

ccccgccgct tcgcgtcggc ctatcgcggc cgctggccgc tcaaaaatgg ctggcctacg 7440

gccaggcaat ctaccagggc gcggacaagc cgcgccgtcg ccactcgacc gccggcgccc 7500

acatcaaggc accggtgggt atgcctgacg atgcgtggag accgaaacct tgcgctcgtt 7560

cgccagccag gacagaaatg cctcgacttc gctgctgccc aaggttgccg ggtgacgcac 7620

accgtggaaa cggatgaagg cacgaaccca gtggacataa gcctgttcgg ttcgtaagct 7680

gtaatgcaag tagcgtatgc gctcacgcaa ctggtccaga accttgaccg aacgcagcgg 7740

tggtaacggc gcagtggcgg ttttcatggc ttgttatgac tgtttttttg gggtacagtc 7800

tatgcctcgg gcatccaagc agcaagcgcg ttacgccgtg ggtcgatgtt tgatgttatg 7860

gagcagcaac gatgttacgc agcagggcag tcgccctaaa acaaagttaa acatcatgag 7920

ggaagcggtg atcgccgaag tatcgactca actatcagag gtagttggcg tcatcgagcg 7980

ccatctcgaa ccgacgttgc tggccgtaca tttgtacggc tccgcagtgg atggcggcct 8040

gaagccacac agtgatattg atttgctggt tacggtgacc gtaaggcttg atgaaacaac 8100

gcggcgagct ttgatcaacg accttttgga aacttcggct tcccctggag agagcgagat 8160

tctccgcgct gtagaagtca ccattgttgt gcacgacgac atcattccgt ggcgttatcc 8220

agctaagcgc gaactgcaat ttggagaatg gcagcgcaat gacattcttg caggtatctt 8280

cgagccagcc acgatcgaca ttgatctggc tatcttgctg acaaaagcaa gagaacatag 8340

cgttgccttg gtaggtccag cggcggagga actctttgat ccggttcctg aacaggatct 8400

atttgaggcg ctaaatgaaa ccttaacgct atggaactcg ccgcccgact gggctggcga 8460

tgagcgaaat gtagtgctta cgttgtcccg catttggtac agcgcagtaa ccggcaaaat 8520

cgcgccgaag gatgtcgctg ccgactgggc aatggagcgc ctgccggccc agtatcagcc 8580

cgtcatactt gaagctagac aggcttatct tggacaagaa gaagatcgct tggcctcgcg 8640

cgcagatcag ttggaagaat ttgtccacta cgtgaaaggc gagatcacca aggtagtcgg 8700

caaataatgt ctaacaattc gttcaagccg acgccgcttc gcggcgcggc ttaactcaag 8760

cgttagatgc actaagcaca taattgctca cagccaaact atcaggtcaa gtctgctttt 8820

attattttta agcgtgcata ataagcccta cacaaattgg gagatatatc atgaaaggct 8880

ggctttttct tgttatcgca atagttggcg aagtaatcgc aacatagctt gcttggtcgt 8940

tccgcgtgaa cgtcggctcg attgtacctg cgttcaaata ctttgcgatc gtgttgcgcg 9000

cctgcccggt gcgtcggctg atctcacgga tcgactgctt ctctcgcaac gccatccgac 9060

ggatgatgtt taaaagtccc atgtggatca ctccgttgcc ccgtcgctca ccgtgttggg 9120

gggaaggtgc acatggctca gttctcaatg gaaattatct gcctaaccgg ctcagttctg 9180

cgtagaaacc aacatgcaag ctccaccggg tgcaaagcgg cagcggcggc aggatatatt 9240

caattgtaaa tggcttcatg tccgggaaat ctacatggat cagcaatgag tatgatggtc 9300

aatatggaga aaaagaaaga gtaattacca attttttttc aattcaaaaa tgtagatgtc 9360

cgcagcgtta ttataaaatg aaagtacatt ttgataaaac gacaaattac gatccgtcgt 9420

atttataggc gaaagcaata aacaaattat tctaattcgg aaatctttat ttcgacgtgt 9480

ctacattcac gtccaaatgg gggcttagat gagaaacttc acgatcggct ctagttcgag 9540

cgatctagta acatagatga caccgcgcgc gataatttat cctagtttgc gcgctatatt 9600

ttgttttcta tcgcgtatta aatgtataat tgcgggactc taatcataaa aacccatctc 9660

ataaataacg tcatgcatta catgttaatt attacatgct taacgtaatt caacagaaat 9720

tatatgataa tcatcgcaag accggcaaca ggattcaatc ttaagaaact ttattgccaa 9780

atgtttgaac gatcggggaa attcgtttct atgcttcaat tctgctctat agatgttctt 9840

ctagtcttaa cttttagaag gcacaacatt tgaaatgttg gtaatttttc tgctgggtag 9900

tgaaccattg taatttgtgt ttgcttatat tatccgttgg gtttctctat gttatctgtg 9960

gggccatatc ccagaactgg ttgagtcggt ccaacacctg ggtgccaatc atgtcgatgg 10020

tggggtatgg tccggattga aagaaatata gtttaaatat ttattgataa aataacaagt 10080

caggtattat agtccaagca aaaacataaa tttattgatg caagtttaaa ttcagaaata 10140

tttcaataac tgattatatc agctggtaca ttgccgtaga tgaaagactg agtgcgatat 10200

tatgtgtaat acataaattg atgatatagc tagcttagct catcgggtca gaagaactcg 10260

tcaagaaggc gatagaaggc gatgcgctgc gaatcgggag cggcgatacc gtaaagcacg 10320

aggaagcggt cagcccattc gccgccaagc tcttcagcaa tatcacgggt agccaacgct 10380

atgtcctgat agcggtccgc cacacccagc cggccacagt cgatgaatcc agaaaagcgg 10440

ccattttcca ccatgatatt cggcaagcag gcatcgccat gagtcacgac gagatcctcg 10500

ccgtcgggca tgcgcgcctt gagcctggcg aacagttcgg ctggcgcgag cccctgatgc 10560

tcttcgtcca gatcatcctg atcgacaaga ccggcttcca tccgagtacg tgctcgctcg 10620

atgcgatgtt tcgcttggtg gtcgaatggg caggtagccg gatcaagcgt atgcagccgc 10680

cgcattgcat cagccatgat ggatactttc tcggcaggag caaggtgaga tgacaggaga 10740

tcctgccccg gcacttcgcc caatagcagc cagtcccttc ccgcttcagt gacaacgtcg 10800

agcacagctg cgcaaggaac gcccgtcgtg gccagccacg atagccgcgc tgcctcgtcc 10860

tgaagttcat tcagggcacc ggacaggtcg gtcttgacaa aaagaaccgg gcgcccctgc 10920

gctgacagcc ggaacacggc ggcatcagag cagccgattg tctgttgtgc ccagtcatag 10980

ccgaatagcc tctccaccca agcggccgga gaacctgcgt gcaatccatc ttgttcaatc 11040

caagctccca ttttctgtga ttttggatgt gtttttgtat ggattgagag tgaatatgag 11100

actctaattg gataccgagg ggaatttatg gaacgtcagt ggagcatttt tgacaagaaa 11160

tatttgctag ctgatagtga ccttaggcga cttttgaacg cgcaataatg gtttctgacg 11220

tatgtgctta gctcattaaa ctccagaaac ccgcggctga gtggctcctt caacgttgcg 11280

gttctgtcag ttccaaacgt aaaacggctt gtcccgcgtc atcggcgggg gtcattccgg 11340

actgtggggc catatcccag aactggttga gtcggtccaa cacctgggtg ccaatcatgt 11400

cgatggtggg gtatggccaa ttttttttca attcaaaaat gtagatgtcc gcagcgttat 11460

tataaaatga aagtacattt tgataaaacg acaaattacg atccgtcgta tttataggcg 11520

aaagcaataa acaaattatt ctaattcgga aatctttatt tcgacgtgtc tacattcacg 11580

tccaaatggg ggcttagatg agaaacttca cgatcggctc tagaggccat gg 11632

<210> 6
<211> 801
<212> DNA
<213> Artificial Sequence

<220>
<223> Neomycin phosphotransferase

<400> 6
atgggagctt ggattgaaca agatggattg cacgcaggtt ctccggccgc ttgggtggag 60

aggctattcg gctatgactg ggcacaacag acaatcggct gctctgatgc cgccgtgttc 120

cggctgtcag cgcaggggcg cccggttctt tttgtcaaga ccgacctgtc cggtgccctg 180

aatgaacttc aggacgaggc agcgcggcta tcgtggctgg ccacgacggg cgttccttgc 240

gcagctgtgc tcgacgttgt cactgaagcg ggaagggact ggctgctatt gggcgaagtg 300

ccggggcagg atctcctgtc atctcacctt gctcctgccg agaaagtatc catcatggct 360

gatgcaatgc ggcggctgca tacgcttgat ccggctacct gcccattcga ccaccaagcg 420

aaacatcgca tcgagcgagc acgtactcgg atggaagccg gtcttgtcga tcaggatgat 480

ctggacgaag agcatcaggg gctcgcgcca gccgaactgt tcgccaggct caaggcgcgc 540

atgcccgacg gcgaggatct cgtcgtgact catggcgatg cctgcttgcc gaatatcatg 600

gtggaaaatg gccgcttttc tggattcatc gactgtggcc ggctgggtgt ggcggaccgc 660

tatcaggaca tagcgttggc tacccgtgat attgctgaag agcttggcgg cgaatgggct 720

gaccgcttcc tcgtgcttta cggtatcgcc gctcccgatt cgcagcgcat cgccttctat 780

cgccttcttg acgagttctt c 801

<210> 7
<211> 702
<212> DNA
<213> Artificial Sequence

<220>
<223> tdTomato, orange fluorescent protein

<400> 7
atggtgagca agggcgagga ggtcatcaaa gagttcatgc gcttcaaggt gcgcatggag 60

ggctccatga acggccacga gttcgagatc gagggcgagg gcgagggccg cccctacgag 120

ggcacccaga ccgccaagct gaaggtgacc aagggcggcc ccctgccctt cgcctgggac 180

atcctgtccc cccagttcat gtacggctcc aaggcgtacg tgaagcaccc cgccgacatc 240

cccgattaca agaagctgtc cttccccgag ggcttcaagt gggagcgcgt gatgaacttc 300

gaggacggcg gtctggtgac cgtgacccag gactcctccc tgcaggacgg cacgctgatc 360

tacaaggtga agatgcgcgg caccaacttc ccccccgacg gccccgtaat gcagaagaag 420

accatgggct gggaggcctc caccgagcgc ctgtaccccc gcgacggcgt gctgaagggc 480

gagatccacc aggccctgaa gctgaaggac ggcggccact acctggtgga gttcaagacc 540

atctacatgg ccaagaagcc cgtgcaactg cccggctact actacgtgga caccaagctg 600

gacatcacct cccacaacga ggactacacc atcgtggaac agtacgagcg ctccgagggc 660

cgccaccacc tgttcctgta cggcatggac gagctgtaca ag 702

<210> 8
<211> 691

<212>  DNA
<213>  Artificial Sequence

<220>
<223>  double 35s promoter

<400>  8
gttcagaaga ccagagggct attgagactt ttcaacaaag ggtaatatcg ggaaacctcc      60

tcggattcca ttgcccagct atctgtcact tcatcgaaag gacagtagaa aaggaagatg     120

gcttctacaa atgccatcat tgcgataaag gaaaggctat cgttcaagat gcctctaccg     180

acagtggtcc caaagatgga cccccaccca cgaggaacat cgtggaaaaa gaagacgttc     240

caaccacgtc ttcaaagcaa gtggattgat gtgatacatg gtggagcacg acactctcgt     300

ctactccaag aatatcaaag atacagtctc agaagaccag agggctattg agacttttca     360

acaaagggta atatcgggaa acctcctcgg attccattgc ccagctatct gtcacttcat     420

cgaaaggaca gtagaaaagg aagatggctt ctacaaatgc catcattgcg ataaaggaaa     480

ggctatcgtt caagatgcct ctaccgacag tggtcccaaa gatggacccc cacccacgag     540

gaacatcgtg gaaaaagaag acgttccaac cacgtcttca aagcaagtgg attgatgtga     600

tatctccact gacgtaaggg atgacgcaca atcccactat ccttcgcaag acccttcctc     660

tatataagga agttcatttc atttggagag g                                    691

<210>  9
<211>  698
<212>  DNA
<213>  Artificial Sequence

<220>
<223>  Col E1 origin of replication

<400>  9
aaaaggatct aggtgaagat cctttttgat aatctcatga ccaaaatccc ttaacgtgag      60

ttttcgttcc actgagcgtc agaccccgta gaaaagatca aaggatcttc ttgagatcct     120

ttttttctgc gcgtaatctg ctgcttgcaa acaaaaaaac caccgctacc agcggtggtt     180

tgtttgccgg atcaagagct accaactctt tttccgaagg taactggctt cagcagagcg     240

cagataccaa atactgttct tctagtgtag ccgtagttag gccaccactt caagaactct     300

gtagcaccgc ctacatacct cgctctgcta atcctgttac cagtggctgc tgccagtggc     360

gataagtcgt gtcttaccgg gttggactca agacgatagt taccggataa ggcgcagcgg     420

tcgggctgaa cggggggttc gtgcacacag cccagcttgg agcgaacgac ctacaccgaa     480

ctgagatacc tacagcgtga gctatgagaa agcgccacgc ttcccgaagg gagaaaggcg     540

gacaggtatc cggtaagcgg cagggtcgga acaggagagc gcacgaggga gcttccaggg     600

ggaaacgcct ggtatcttta tagtcctgtc gggtttcgcc acctctgact tgagcgtcga     660

tttttgtgat gctcgtcagg ggggcggagc ctatggaa 698

<210> 10
<211> 3706
<212> DNA
<213> Artificial Sequence

<220>
<223> pVS1 Replicon

<400> 10
cgacatgtcg cacaagtcct aagttacgcg acaggctgcc gccctgccct tttcctggcg 60

ttttcttgtc gcgtgtttta gtcgcataaa gtagaatact tgcgactaga accggagaca 120

ttacgccatg aacaagagcg ccgccgctgg cctgctgggc tatgcccgcg tcagcaccga 180

cgaccaggac ttgaccaacc aacgggccga actgcacgcg gccggctgca ccaagctgtt 240

ttccgagaag atcaccggca ccaggcgcga ccgcccggag ctggccagga tgcttgacca 300

cctacgccct ggcgacgttg tgacagtgac caggctagac cgcctggccc gcagcacccg 360

cgacctactg gacattgccg agcgcatcca ggaggccggc gcgggcctgc gtagcctggc 420

agagccgtgg gccgacacca ccacgccggc cggccgcatg gtgttgaccg tgttcgccgg 480

cattgccgag ttcgagcgtt ccctaatcat cgaccgcacc cggagcgggc gcgaggccgc 540

caaggcgcga ggcgtgaagt ttggcccccg ccctaccctc accccggcac agatcgcgca 600

cgcccgcgag ctgatcgacc aggaaggccg caccgtgaaa gaggcggctg cactgcttgg 660

cgtgcatcgc tcgaccctgt accgcgcact tgagcgcagc gaggaagtga cgcccaccga 720

ggccaggcgg cgcggtgcct tccgtgagga cgcattgacc gaggccgacg ccctggcggc 780

cgccgagaat gaacgccaag aggaacaagc atgaaaccgc accaggacgg ccaggacgaa 840

ccgtttttca ttaccgaaga gatcgaggcg gagatgatcg cggccgggta cgtgttcgag 900

ccgcccgcgc acgtctcaac cgtgcggctg catgaaatcc tggccggttt gtctgatgcc 960

aagctcgcgg cctggccggc gagcttggcc gctgaagaaa ccgagcgccg ccgtctaaaa 1020

aggtgatgtg tatttgagta aaacagcttg cgtcatgcgg tcgctgcgta tatgatgcga 1080

tgagtaaata aacaaatacg caaggggaac gcatgaaggt tatcgctgta cttaaccaga 1140

aaggcgggtc aggcaagacg accatcgcaa cccatctagc ccgcgccctg caactcgccg 1200

gggccgatgt tctgttagtc gattccgatc cccagggcag tgcccgcgat tgggcggccg 1260

tgcgggaaga tcaaccgcta accgttgtcg gcatcgaccg cccgacgatt gaccgcgacg 1320

tgaaggccat cggccggcgc gacttcgtag tgatcgacgg agcgccccag gcggcggact 1380

tggctgtgtc cgcgatcaag gcagccgact tcgtgctgat tccggtgcag ccaagccctt 1440

acgacatatg ggccaccgcc gacctggtgg agctggttaa gcagcgcatt gaggtcacgg 1500

atggaaggct acaagcggcc tttgtcgtgt cgcgggcgat caaaggcacg cgcatcggcg 1560

gtgaggttgc cgaggcgctg gccgggtacg agctgcccat tcttgagtcc cgtatcacgc 1620

agcgcgtgag ctacccaggc actgccgccg ccggcacaac cgttcttgaa tcagaacccg 1680

agggcgacgc tgcccgcgag gtccaggcgc tggccgctga aattaaatca aaactcattt 1740

gagttaatga ggtaaagaga aaatgagcaa aagcacaaac acgctaagtg ccggccgtcc 1800

gagcgcacgc agcagcaagg ctgcaacgtt ggccagcctg gcagacacgc cagccatgaa 1860

gcgggtcaac tttcagttgc cggcggagga tcacaccaag ctgaagatgt acgcggtacg 1920

ccaaggcaag accattaccg agctgctatc tgaatacatc gcgcagctac cagagtaaat 1980

gagcaaatga ataaatgagt agatgaattt tagcggctaa aggaggcggc atggaaaatc 2040

aagaacaacc aggcaccgac gccgtggaat gccccatgtg tggaggaacg ggcggttggc 2100

caggcgtaag cggctgggtt gtctgccggc cctgcaatgg cactggaacc cccaagcccg 2160

aggaatcggc gtgagcggtc gcaaaccatc cggcccggta caaatcggcg cggcgctggg 2220

tgatgacctg gtggagaagt tgaaggcggc gcaggccgcc cagcggcaac gcatcgaggc 2280

agaagcacgc cccggtgaat cgtggcaagc ggccgctgat cgaatccgca aagaatcccg 2340

gcaaccgccg gcagccggtg cgccgtcgat taggaagccg cccaagggcg acgagcaacc 2400

agattttttc gttccgatgc tctatgacgt gggcacccgc gatagtcgca gcatcatgga 2460

cgtggccgtt ttccgtctgt cgaagcgtga ccgacgagct ggcgaggtga tccgctacga 2520

gcttccagac gggcacgtag aggtttccgc agggccggcc ggcatggcga gtgtgtggga 2580

ttacgacctg gtactgatgg cggtttccca tctaaccgaa tccatgaacc gataccggga 2640

agggaaggga gacaagcccg gccgcgtgtt ccgtccacac gttgcggacg tactcaagtt 2700

ctgccggcga gccgatggcg gaaagcagaa agacgacctg gtagaaacct gcattcggtt 2760

aaacaccacg cacgttgcca tgcagcgtac gaagaaggcc aagaacggcc gcctggtgac 2820

ggtatccgag ggtgaagcct tgattagccg ctacaagatc gtaaagagcg aaaccgggcg 2880

gccggagtac atcgagatcg agctagctga ttggatgtac cgcgagatca cagaaggcaa 2940

gaacccggac gtgctgacgg ttcaccccga ttactttttg atcgatcccg gcatcggccg 3000

ttttctctac cgcctggcac gccgcgccgc aggcaaggca gaagccagat ggttgttcaa 3060

gacgatctac gaacgcagtg gcagcgccgg agagttcaag aagttctgtt tcaccgtgcg 3120

caagctgatc gggtcaaatg acctgccgga gtacgatttg aaggaggagg cggggcaggc 3180

tggcccgatc ctagtcatgc gctaccgcaa cctgatcgag ggcgaagcat ccgccggttc 3240

ctaatgtacg gagcagatgc tagggcaaat tgccctagca ggggaaaaag gtcgaaaagg 3300

tctctttcct gtggatagca cgtacattgg gaacccaaag ccgtacattg ggaaccggaa 3360

cccgtacatt gggaacccaa agccgtacat tgggaaccgg tcacacatgt aagtgactga 3420

tataaaagag aaaaaaggcg atttttccgc ctaaaactct ttaaaactta ttaaaactct 3480

taaaacccgc ctggcctgtg cataactgtc tggccagcgc acagccgaag agctgcaaaa 3540

agcgcctacc cttcggtcgc tgcgctccct acgccccgcc gcttcgcgtc ggcctatcgc 3600

ggccgctggc cgctcaaaaa tggctggcct acggccaggc aatctaccag ggcgcggaca 3660

agccgcgccg tcgccactcg accgccggcg cccacatcaa ggcacc 3706

<210> 11
<211> 1008
<212> DNA
<213> Escherichia coli

<400> 11
atgcgctcac gcaactggtc cagaaccttg accgaacgca gcggtggtaa cggcgcagtg 60

gcggttttca tggcttgtta tgactgtttt tttggggtac agtctatgcc tcgggcatcc 120

aagcagcaag cgcgttacgc cgtgggtcga tgtttgatgt tatggagcag caacgatgtt 180

acgcagcagg gcagtcgccc taaaacaaag ttaaacatca tgagggaagc ggtgatcgcc 240

gaagtatcga ctcaactatc agaggtagtt ggcgtcatcg agcgccatct cgaaccgacg 300

ttgctggccg tacatttgta cggctccgca gtggatggcg gcctgaagcc acacagtgat 360

attgatttgc tggttacggt gaccgtaagg cttgatgaaa caacgcggcg agctttgatc 420

aacgaccttt tggaaacttc ggcttcccct ggagagagcg agattctccg cgctgtagaa 480

gtcaccattg ttgtgcacga cgacatcatt ccgtggcgtt atccagctaa gcgcgaactg 540

caatttggag aatggcagcg caatgacatt cttgcaggta tcttcgagcc agccacgatc 600

gacattgatc tggctatctt gctgacaaaa gcaagagaac atagcgttgc cttggtaggt 660

ccagcggcgg aggaactctt tgatccggtt cctgaacagg atctatttga ggcgctaaat 720

gaaaccttaa cgctatggaa ctcgccgccc gactgggctg gcgatgagcg aaatgtagtg 780

cttacgttgt cccgcatttg gtacagcgca gtaaccggca aaatcgcgcc gaaggatgtc 840

gctgccgact gggcaatgga gcgcctgccg gcccagtatc agcccgtcat acttgaagct 900

agacaggctt atcttggaca agaagaagat cgcttggcct cgcgcgcaga tcagttggaa 960

gaatttgtcc actacgtgaa aggcgagatc accaaggtag tcggcaaa 1008

<210> 12
<211> 909
<212> DNA
<213> Artificial Sequence

<220>
<223> cDNA sequence of ZmWUS2

<400> 12
atggcggcca atgcgggcgg cggtggagcg ggaggaggca gcggcagcgg cagcgtggct 60

gcgccggcgg tgtgccgccc cagcggctcg cggtggacgc cgacgccgga gcagatcagg     120

atgctgaagg agctctacta cggctgcggc atccggtcgc ccagctcgga gcagatccag     180

cgcatcaccg ccatgctgcg gcagcacggc aagatcgagg gcaagaacgt cttctactgg     240

ttccagaacc acaaggcccg cgagcgccag aagcgccgcc tcaccagcct cgacgtcaac     300

gtgcccgccg ccggcgcggc cgacgccacc accagccaac tcggcgtcct ctcgctgtcg     360

tcgccgccgc cttcaggcgc ggcgcctccc tcgcccaccc tcggcttcta cgccgccggc     420

aatggcggcg gatcggctgt gctgctggac acgagttccg actggggcag cagcggcgct     480

gccatggcca ccgagacatg cttcctccag gactacatgg gcgtgacgga cacgggcagc     540

tcgtcgcagt ggccacgctt ctcgtcgtcg gacacgataa tggcggcggc cgcggcgcgg     600

gcggcgacga cgcgggcgcc cgagacgctc cctctcttcc cgacctgcgg cgacgacggc     660

ggcagcggta gcagcagcta cttgccgttc tggggtgccg cgtccacaac tgccggcgcc     720

acttcttccg ttgcgatcca gcagcaacac cagctgcagg agcagtacag cttttacagc     780

aacagcaaca gcacccagct ggccggcacc ggcaaccaag acgtatcggc aacagcagca     840

gcagccgccg ccctggagct gagcctcagc tcatggtgct ccccttaccc tgctgcaggg     900

agtatgtga                                                             909

<210>  13
<211>  302
<212>  PRT
<213>  Zea mays

<400>  13

Met Ala Ala Asn Ala Gly Gly Gly Gly Ala Gly Gly Gly Ser Gly Ser
1               5                   10                  15

Gly Ser Val Ala Ala Pro Ala Val Cys Arg Pro Ser Gly Ser Arg Trp
            20                  25                  30

Thr Pro Thr Pro Glu Gln Ile Arg Met Leu Lys Glu Leu Tyr Tyr Gly
        35                  40                  45

Cys Gly Ile Arg Ser Pro Ser Ser Glu Gln Ile Gln Arg Ile Thr Ala
    50                  55                  60

Met Leu Arg Gln His Gly Lys Ile Glu Gly Lys Asn Val Phe Tyr Trp
65                  70                  75                  80

Phe Gln Asn His Lys Ala Arg Glu Arg Gln Lys Arg Arg Leu Thr Ser
                85                  90                  95

Leu Asp Val Asn Val Pro Ala Ala Gly Ala Ala Asp Ala Thr Thr Ser

            100                 105                 110

Gln Leu Gly Val Leu Ser Leu Ser Ser Pro Pro Pro Ser Gly Ala Ala
        115                 120                 125

Pro Pro Ser Pro Thr Leu Gly Phe Tyr Ala Ala Gly Asn Gly Gly Gly
    130                 135                 140

Ser Ala Val Leu Leu Asp Thr Ser Ser Asp Trp Gly Ser Ser Gly Ala
145                 150                 155                 160

Ala Met Ala Thr Glu Thr Cys Phe Leu Gln Asp Tyr Met Gly Val Thr
                165                 170                 175

Asp Thr Gly Ser Ser Ser Gln Trp Pro Arg Phe Ser Ser Ser Asp Thr
            180                 185                 190

Ile Met Ala Ala Ala Ala Ala Arg Ala Ala Thr Thr Arg Ala Pro Glu
        195                 200                 205

Thr Leu Pro Leu Phe Pro Thr Cys Gly Asp Asp Gly Gly Ser Gly Ser
    210                 215                 220

Ser Ser Tyr Leu Pro Phe Trp Gly Ala Ala Ser Thr Thr Ala Gly Ala
225                 230                 235                 240

Thr Ser Ser Val Ala Ile Gln Gln Gln His Gln Leu Gln Glu Gln Tyr
                245                 250                 255

Ser Phe Tyr Ser Asn Ser Asn Ser Thr Gln Leu Ala Gly Thr Gly Asn
            260                 265                 270

Gln Asp Val Ser Ala Thr Ala Ala Ala Ala Ala Ala Leu Glu Leu Ser
        275                 280                 285

Leu Ser Ser Trp Cys Ser Pro Tyr Pro Ala Ala Gly Ser Met
    290                 295                 300

<210> 14
<211> 13362
<212> DNA
<213> Artificial Sequence

<220>
<223> IPR232-pS-01

<400> 14
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtgggccc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg gcggccggaa gacaaagata 1620

gcccggggcc tgcaggacca atgcattgga tctggagacc cgccggcggc agaagagatc 1680

caacaaaaca ttcacaatgg attatagaaa catttgttta ttcattataa tgagatctta 1740

cattcattta atattagaaa aagccacaaa ttcataacac aacaagccaa gaaaaaaaca 1800

caaacttaag cacacaagct ttttatttga cacaccaaat atttcatctt catcttcata 1860

tgagctcgtc gacggtaccc cctcaattag ttacatcacc actttgtaca agaaagctgg 1920

gtgtcatttt tgatgaaaca ggagcttttt gatatttcca tttgaatatt ttggtatctg 1980

attagtgatg atttcagcta acatctctgg gaactcaata ctcatggtct tatccaaaaa 2040

tgtctggaag cagtaggtaa ggagattctc aaccacctca tgcatggagt ccagaagctt 2100

tgtcagttgg taaaaccgtt gccagttctg actggagttc ccttcccttt tgacgatggc 2160

ttttcctagc tctttgatat aagtcattcg aatctcatca aataactctt ggctcttcag 2220

accttcctta ggaactgagg agagaagcag taaggttttc atacagagat actcttcata 2280

ggatacctgc aatctttgta attcagagga gacaaacagc atgtgtttac attggtcata 2340

catgcagggt agagacattc tctgctcatt aataatcaga tcaggagcaa agcagagcag 2400

gtttccactt gattgtctgt atgatctcca acccagggca aatgccatga gaaacatcca 2460

tgagtactgt agcagggtca tttggtcatc caggtgtaag tttctgaagc ctggtatcgc 2520

ctttgcccat ttcactgctg caatcacttg acgcccacct aacatgttga gtgtggtcat 2580

aattctccac gctgaatctg gaacagagct atcatatcct gcatacaaca cctcgggttc 2640

aatcacctcc agcagtgaca ccaaggtagg ggtgagctgt ggtaatgcag caggaactat 2700

tgttttgtta ggattttccg aagtgtcttg tgagactcct gcagtggctt gctgaatccc 2760

tttgattttt ttctttgtag atccattgtc gttccaaact gtaaatgttg gagcaacttc 2820

tccaccacct tccccatggt aatacatatt agagccaaca ttattcgtca ttgccgcggg 2880

aaaatctcca cctggcgact gctgggtctg ctgctgctga gcaaagtaat aatggtttct 2940

tgcgttataa tcaaactcac tagcagcgta ggcatcgcag ttaaccgggg ctgcaaatcc 3000

ttgataacca ccataaccaa caacatttcc acaaacagta accgaatcat ccgaaacaga 3060

actttgatga tcgatattag tcatgaggct ctgcgtgttg ctcaagaaat ggtgttgatc 3120

atcctcttgt ttgaaacaag ccctagcact ctccgaagac aagtttcctc cattgtaata 3180

ataaccattg tacctctctt gatgttggtg caataagctc aaatctactc cctgatgatg 3240

ctgaaccgca gcgttttgcc aaccgctagc actattctca ctctctgaaa cgttattact 3300

gatcatcatc atacttggaa ccggacggtt agcctccttg agacgttttg cggcgctacc 3360

aataggaaga ctagggcttt cgaggattgc tttaacgttg tatctgttca tgtcgaagtt 3420

agtcactgcg gttaatcctc tgaatttgat ggccgcaatg tcgtatgcct ctgcagcttc 3480

ttcttgtgtg ccaaaagttc ccaagtagag gtctttgtta ccggcgactc ttcctatcct 3540

agcttgccat cttccatgtt ggtgatgtct tgttactcca cgataaatcg atgcaccacg 3600

agagaaacca ctacttttcc tgcgcagtga ggcaacatac tcttgccttg tcatgtgctt 3660

catctcttct acctcttttt catattcgct catggggaag ttagtagtag tggtggttcc 3720

ccaatacttg agtgcggcta aatcataagc cctagctgct tttcttcttt tgtcataacc 3780

tcccaaataa acttgtcttc ctttgcgcgt ttggccttct cttttacaac tattatccca 3840

taaatgtgcc tcatatcttc ctgtccaccg atgccttgta acaccgcggt atatagacgt 3900

cctctgtcca aaactctcaa tagttttctt cggtgtagct tcaacgctat catcaatagt 3960

cttcccttgg gcaacaacgt tgttattgct gtcgttgttg ttatcacaag aagtagatga 4020

gttcattgag agggacaggc cttttgcagc attgccattt tcttgattat caacattatc 4080

cacgggttga tttctcagcc atgtctttat catcgaaagt cctagtgagc caccaccacc 4140

gtctcctcct ccataacagc cacttccact tccatctcca acgttttcgt tggtgttgta 4200

aatcgtggtg gtgcggccaa ggaaattctc aagctttggt ccatcttgct catcgttgtg 4260

gatgttattg catgcacaac cattgatgtc ccaatctcgg gagtgactat tgttgtctct 4320

ggtgaaagca tcgacgacga caccaaaggg agaaggaaac gatgtttgga tggctgaaga 4380

ctcatcggag gcagcggtcg gatcgtaaca gtactctccg gcgacatcta cgacggttgt 4440

ggtggtggaa gagtagacgt ccttacgatg gtgattttgt tcataaggag agagagaaaa 4500

gcctaaccag ttattatcca ttgagcctgc ttttttgtac aaacttgtga tcgggatctg 4560

taattgtaat gttgtttgtt gtttgttgtt gttggtaatt gtggatcctc tagagtcccc 4620

cgtgttctct ccaaatgaaa tgaacttcct tatatagagg aagggtcttg cgaaggatag 4680

tgggattgtg cgtcatccct tacgtcagtg gagatatcac atcaatccac ttgctttgaa 4740

gacgtggttg gaacgtcttc tttttccacg atgctcctcg tgggtggggg tccatctttg 4800

ggaccactgt cggcagaggc atcttcaacg atggcctttc ctttatcgca atgatggcat 4860

ttgtaggagc caccttcctt ttccactatc ttcacaataa agtgacagat agctgggcaa 4920

tggaatccga ggaggtttcc ggatattacc ctttgttgaa aagtctcaat tgccctttgg 4980

tcttctgaga ctgtatcttt gatatttttg gagtagacaa gtgtgtcgtg ctccaccatg 5040

ttgacgaaga ttttcttctt gtcattgagt cgtaagagac tctgtatgaa ctgttcgcca 5100

gtctttacgg cgagttctgt taggtcctct atttgaatct ttgactccat ggcctttgat 5160

tcagtgggaa ctaccttttt agagactcca atctctatta cttgcctcgc cggcgggtct 5220

cgggatcccc tgcaggacta gtggatttgt cttcaaggcc ttaagggcca gatcttgggc 5280

ccggtacccg atcagattgt cgtttcccgc cttcggttta aactatcagt gtttgacagg 5340

atatattggc gggtaaacct aagagaaaag agcgtttatt agaataatcg gatatttaaa 5400

agggcgtgaa aaggtttatc cgttcgtcca tttgtatgtg catgccaacc acagggttcc 5460

cctcgggagt gcttggcatt ccgtgcgata atgacttctg ttcaaccacc caaacgtcgg 5520

aaagcctgac gacggagcag cattccaaaa agatcccttg gctcgtctgg gtcggctaga 5580

aggtcgagtg ggctgctgtg gcttgatccc tcaacgcggt cgcggacgta gcgcagcgcc 5640

gaaaaatcct cgatcgcaaa tccgacgctg tcgaaaagcg tgatctgctt gtcgctcttt 5700

cggccgacgt cctggccagt catcacgcgc caaagttccg tcacaggatg atctggcgcg 5760

agttgctgga tctcgccttc aatccgggtc tgtggcggga actccacgaa aatatccgaa 5820

cgcagcaaga tatcgcggtg catctcggtc ttgcctgggc agtcgccgcc gacgccgttg 5880

atgtggacgc cgaaaaggat ctaggtgaag atcctttttg ataatctcat gaccaaaatc 5940

ccttaacgtg agttttcgtt ccactgagcg tcagaccccg tagaaaagat caaaggatct 6000

tcttgagatc ctttttttct gcgcgtaatc tgctgcttgc aaacaaaaaa accaccgcta 6060

ccagcggtgg tttgtttgcc ggatcaagag ctaccaactc tttttccgaa ggtaactggc 6120

ttcagcagag cgcagatacc aaatactgtt cttctagtgt agccgtagtt aggccaccac 6180

ttcaagaact ctgtagcacc gcctacatac ctcgctctgc taatcctgtt accagtggct 6240

gctgccagtg gcgataagtc gtgtcttacc gggttggact caagacgata gttaccggat 6300

aaggcgcagc ggtcgggctg aacggggggt tcgtgcacac agcccagctt ggagcgaacg 6360

acctacaccg aactgagata cctacagcgt gagctatgag aaagcgccac gcttcccgaa 6420

gggagaaagg cggacaggta tccggtaagc ggcagggtcg gaacaggaga gcgcacgagg 6480

gagcttccag ggggaaacgc ctggtatctt tatagtcctg tcgggtttcg ccacctctga 6540

cttgagcgtc gatttttgtg atgctcgtca ggggggcgga gcctatggaa aaacgccagc 6600

aacgcggcct ttttacggtt cctggccttt tgctggcctt ttgctcacat gttctttcct 6660

gcgttatccc ctgattctgt ggataaccga ttaccgcctt tgagtgagct gataccgctc 6720

gccgcagccg aacgaccgag cgcagcgagt cagtgagcga ggaagcggaa gagcgcctga 6780

tgcggtattt tctccttacg catctgtgcg gtatttcaca ccgcatatgg tgcactctca 6840

gtacaatctg ctctgatgcc gcatagttaa gccagtatac actccgctat cgctacgtga 6900

ctgggtcatg gctgcgcccc gacacccgcc aacacccgct gacgcgccct gacgggcttg 6960

tctgctcccg gcatccgctt acagacaagc tgtgaccgtc tccgggagct gcatgtgtca 7020

gaggttttca ccgtcatcac cgaaacgcgc gaggcagggg tacgtcgagg tcgatccaac 7080

ccctccgctg ctatagtgca gtcggcttct gacgttcagt gcagccgtct tctgaaaacg 7140

acatgtcgca caagtcctaa gttacgcgac aggctgccgc cctgcccttt tcctggcgtt 7200

ttcttgtcgc gtgttttagt cgcataaagt agaatacttg cgactagaac cggagacatt 7260

acgccatgaa caagagcgcc gccgctggcc tgctgggcta tgcccgcgtc agcaccgacg 7320

accaggactt gaccaaccaa cgggccgaac tgcacgcggc cggctgcacc aagctgtttt 7380

ccgagaagat caccggcacc aggcgcgacc gcccggagct ggccaggatg cttgaccacc 7440

tacgccctgg cgacgttgtg acagtgacca ggctagaccg cctggcccgc agcacccgcg 7500

acctactgga cattgccgag cacatccagg aggccggcgc gggcctgcgt agcctggcag 7560

agccgtgggc cgacaccacc acgccggccg gccgcatggt gttgaccgtg ttcgccggca 7620

ttgccgagtt cgagcgttcc ctaatcatcg accgcacccg gagcgggcgc gaggccgcca 7680

aggcgcgagg cgtgaagttt ggcccccgcc ctaccctcac cccggcacag atcgcgcacg 7740

cccgcgagct gatcgaccag gaaggccgca ccgtgaaaga ggcggctgca ctgcttggcg 7800

tgcatcgctc gaccctgtac cgcgcacttg agcgcagcga ggaagtgacg cccaccgagg 7860

ccaggcggcg cggtgccttc cgtgaggacg cattgaccga ggccgacgcc ctggcggccg 7920

ccgagaatga acgccaagag gaacaagcat gaaaccgcac caggacggcc aggacgaacc 7980

gtttttcatt accgaagaga tcgaggcgga gatgatcgcg gccgggtacg tgttcgagcc 8040

gcccgcgcac gtctcaaccg tgcggctgca tgaaatcctg gccggtttgt ctgatgccaa 8100

gctcgcggcc tggccggcga gcttggccgc tgaagaaacc gagcgccgcc gtctaaaaag 8160

gtgatgtgta tttgagtaaa acagcttgcg tcatgcggtc gctgcgtata tgatgcgatg 8220

agtaaataaa caaatacgca aggggaacgc atgaaggtta tcgctgtact taaccagaaa 8280

ggcgggtcag gcaagacgac catcgcaacc catctagccc gcgccctgca actcgccggg 8340

gccgatgttc tgttagtcga ttccgatccc cagggcagtg cccgcgattg ggcggccgtg 8400

cgggaagatc aaccgctaac cgttgtcggc atcgaccgcc cgacgattga ccgcgacgtg 8460

aaggccatcg gccggcgcga cttcgtagtg atcgacggag cgccccaggc ggcggacttg 8520

gctgtgtccg cgatcaaggc agccgacttc gtgctgattc cggtgcagcc aagcccttac 8580

gacatatggg ccaccgccga cctggtggag ctggttaagc agcgcattga ggtcacggat 8640

ggaaggctac aagcggcctt tgtcgtgtcg cgggcgatca aaggcacgcg catcggcggt 8700

gaggttgccg aggcgctggc cgggtacgag ctgcccattc ttgagtcccg tatcacgcag 8760

cgcgtgagct acccaggcac tgccgccgcc ggcacaaccg ttcttgaatc agaacccgag 8820

ggcgacgctg cccgcgaggt ccaggcgctg gccgctgaaa ttaaatcaaa actcatttga 8880

gttaatgagg taaagagaaa atgagcaaaa gcacaaacac gctaagtgcc ggccgtccga 8940

gcgcacgcag cagcaaggct gcaacgttgg ccagcctggc agacacgcca gccatgaagc 9000

gggtcaactt tcagttgccg gcggaggatc acaccaagct gaagatgtac gcggtacgcc 9060

aaggcaagac cattaccgag ctgctatctg aatacatcgc gcagctacca gagtaaatga 9120

gcaaatgaat aaatgagtag atgaatttta gcggctaaag gaggcggcat ggaaaatcaa 9180

gaacaaccag gcaccgacgc cgtggaatgc cccatgtgtg gaggaacggg cggttggcca 9240

ggcgtaagcg gctgggttgt ctgccggccc tgcaatggca ctggaacccc caagcccgag 9300

gaatcggcgt gagcggtcgc aaaccatccg gcccggtaca aatcggcgcg gcgctgggtg 9360

atgacctggt ggagaagttg aaggcggcgc aggccgccca gcggcaacgc atcgaggcag 9420

aagcacgccc cggtgaatcg tggcaagcgg ccgctgatcg aatccgcaaa gaatcccggc 9480

aaccgccggc agccggtgcg ccgtcgatta ggaagccgcc caagggcgac gagcaaccag 9540

attttttcgt tccgatgctc tatgacgtgg gcacccgcga tagtcgcagc atcatggacg 9600

tggccgtttt ccgtctgtcg aagcgtgacc gacgagctgg cgaggtgatc cgctacgagc 9660

ttccagacgg gcacgtagag gtttccgcag ggccggccgg catggcgagt gtgtgggatt 9720

acgacctggt actgatggcg gtttcccatc taaccgaatc catgaaccga taccgggaag 9780

ggaagggaga caagcccggc cgcgtgttcc gtccacacgt tgcggacgta ctcaagttct 9840

gccggcgagc cgatggcgga aagcagaaag acgacctggt agaaacctgc attcggttaa 9900

acaccacgca cgttgccatg cagcgtacga agaaggccaa gaacggccgc ctggtgacgg 9960

tatccgaggg tgaagccttg attagccgct acaagatcgt aaagagcgaa accgggcggc 10020

cggagtacat cgagatcgag ctagctgatt ggatgtaccg cgagatcaca gaaggcaaga 10080

acccggacgt gctgacggtt caccccgatt actttttgat cgatcccggc atcggccgtt 10140

ttctctaccg cctggcacgc cgcgccgcag gcaaggcaga agccagatgg ttgttcaaga 10200

cgatctacga acgcagtggc agcgccggag agttcaagaa gttctgtttc accgtgcgca 10260

agctgatcgg gtcaaatgac ctgccggagt acgatttgaa ggaggaggcg gggcaggctg 10320

gcccgatcct agtcatgcgc taccgcaacc tgatcgaagg cgaagcatcc gccggttcct 10380

aatgtacgga gcagatgcta gggcaaattg ccctagcagg ggaaaaaggt cgaaaaggtc 10440

tctttcctgt ggatagcacg tacattggga acccaaagcc gtacattggg aaccggaacc 10500

cgtacattgg gaacccaaag ccgtacattg ggaaccggtc acacatgtaa gtgactgata 10560

taaaagagaa aaaaggcgat ttttccgcct aaaactcttt aaaacttatt aaaactctta 10620

aaacccgcct ggcctgtgca taactgtctg gccagcgcac agccgaagag ctgcaaaaag 10680

cgcctaccct tcggtcgctg cgctccctac gccccgccgc ttcgcgtcgg cctatcgcgg 10740

ccgctggccg ctcaaaaatg gctggcctac ggccaggcaa tctaccaggg cgcggacaag 10800

ccgcgccgtc gccactcgac cgccggcgcc cacatcaagg caccggtggg tatgcctgac 10860

gatgcgtgga gaccgaaacc ttgcgctcgt tcgccagcca ggacagaaat gcctcgactt 10920

cgctgctgcc caaggttgcc gggtgacgca caccgtggaa acggatgaag gcacgaaccc 10980

agtggacata agcctgttcg gttcgtaagc tgtaatgcaa gtagcgtatg cgctcacgca 11040

actggtccag aaccttgacc gaacgcagcg gtggtaacgg cgcagtggcg gttttcatgg 11100

cttgttatga ctgttttttt ggggtacagt ctatgcctcg ggcatccaag cagcaagcgc 11160

gttacgccgt gggtcgatgt ttgatgttat ggagcagcaa cgatgttacg cagcagggca 11220

gtcgccctaa aacaaagtta aacatcatga gggaagcggt gatcgccgaa gtatcgactc 11280

aactatcaga ggtagttggc gtcatcgagc gccatctcga accgacgttg ctggccgtac 11340

atttgtacgg ctccgcagtg gatggcggcc tgaagccaca cagtgatatt gatttgctgg 11400

ttacggtgac cgtaaggctt gatgaaacaa cgcggcgagc tttgatcaac gaccttttgg 11460

aaacttcggc ttcccctgga gagagcgaga ttctccgcgc tgtagaagtc accattgttg 11520

tgcacgacga catcattccg tggcgttatc cagctaagcg cgaactgcaa tttggagaat 11580

ggcagcgcaa tgacattctt gcaggtatct tcgagccagc cacgatcgac attgatctgg 11640

ctatcttgct gacaaaagca agagaacata gcgttgcctt ggtaggtcca gcggcggagg 11700

aactctttga tccggttcct gaacaggatc tatttgaggc gctaaatgaa accttaacgc 11760

tatggaactc gccgcccgac tgggctggcg atgagcgaaa tgtagtgctt acgttgtccc 11820

gcatttggta cagcgcagta accggcaaaa tcgcgccgaa ggatgtcgct gccgactggg 11880

caatggagcg cctgccggcc cagtatcagc ccgtcatact tgaagctaga caggcttatc 11940

ttggacaaga agaagatcgc ttggcctcgc gcgcagatca gttggaagaa tttgtccact 12000

acgtgaaagg cgagatcacc aaggtagtcg gcaaataatg tctaacaatt cgttcaagcc 12060

gacgccgctt cgcggcgcgg cttaactcaa gcgttagatg cactaagcac ataattgctc 12120

acagccaaac tatcaggtca agtctgcttt tattattttt aagcgtgcat aataagccct 12180

acacaaattg ggagatatat catgaaaggc tggctttttc ttgttatcgc aatagttggc 12240

gaagtaatcg caacatagct tgcttggtcg ttccgcgtga acgtcggctc gattgtacct 12300

gcgttcaaat actttgcgat cgtgttgcgc gcctgcccgg tgcgtcggct gatctcacgg 12360

atcgactgct tctctcgcaa cgccatccga cggatgatgt ttaaaagtcc catgtggatc 12420

actccgttgc cccgtcgctc accgtgttgg ggggaaggtg cacatggctc agttctcaat 12480

ggaaattatc tgcctaaccg gctcagttct gcgtagaaac caacatgcaa gctccaccgg 12540

gtgcaaagcg gcagcggcgg caggatatat tcaattgtaa atggcttcat gtccgggaaa 12600

tctacatgga tcagcaatga gtatgatggt caatatggag aaaaagaaag agtaattacc 12660

aatttttttt caattcaaaa atgtagatgt ccgcagcgtt attataaaat gaaagtacat 12720

tttgataaaa cgacaaatta cgatccgtcg tatttatagg cgaaagcaat aaacaaatta 12780

ttctaattcg gaaatcttta tttcgacgtg tctacattca cgtccaaatg ggggcttaga 12840

tgagaaactt cacgatcggc tctagttcga gcgatctagt aacatagatg acaccgcgcg 12900

cgataattta tcctagtttg cgcgctatat tttgttttct atcgcgtatt aaatgtataa 12960

ttgcgggact ctaatcataa aaacccatct cataaataac gtcatgcatt acatgttaat 13020

tattacatgc ttaacgtaat tcaacagaaa ttatatgata atcatcgcaa gaccggcaac 13080

aggattcaat cttaagaaac tttattgcca aatgtttgaa cgatcgggga aattcgtttc 13140

tatgcttcaa ttctgctcta tagatgttct tctagtctta acttttagaa ggcacaacat 13200

ttgaaatgtt ggtaattttt ctgctgggta gtgaaccatt gtaatttgtg tttgcttata 13260

ttatccgttg ggtttctcta tgttatctgt ggggccatat cccagaactg gttgagtcgg 13320

tccaacacct gggtgccaat catgtcgatg gtggggtatg gt 13362

<210> 15
<211> 12734
<212> DNA
<213> Artificial Sequence

<220>
<223> IPR252-pS-01

<400> 15
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg gcggccggaa gacaaagata 1620

gcccggggcc tgcaggacca atgcattgga tctggagacc cgccggcggc agaagagatc 1680

caacaaaaca ttcacaatgg attatagaaa catttgttta ttcattataa tgagatctta 1740

cattcattta atattagaaa aagccacaaa ttcataacac aacaagccaa gaaaaaaaca 1800

caaacttaag cacacaagct ttttatttga cacaccaaat atttcatctt catcttcata 1860

tgagctcgtc gacggtaccc cctcaattag ttacatcacc actttgtaca agaaagctgg 1920

gtacaaatgt ttgaacgatc ggggaaattc gagctcggta ccgggccccc cctcgagttt 1980

tttttttttt ttttttttag tctgaaaatt tatccaacta atggaaacta aaaataagtc 2040

ttaagaaatt aaccaagaaa aagaaaaaga gaaagaaaaa aacataatca aagtatcaca 2100

ctgttcacca gcacacaaca acgcatataa ctaaagatct ttaactattt tttctaattg 2160

tcgttccaaa ctgtaaatgt tggagcaact tctccaccac cttccccatg gtaatacata 2220

ttagagccaa cattattcgt cattgccgcg ggaaaatctc cacctggcga ctgctgggtc 2280

tgctgctgct gagcaaagta ataatggttt cttgcgttat aatcaaactc actagcagcg 2340

taggcatcgc agttaaccgg ggctgcaaat ccttgataac caccataacc aacaacattt 2400

ccacaaacag taaccgaatc atccgaaaca gaactttgat gatcgatatt agtcatgagg 2460

ctctgcgtgt tgctcaagaa atggtgttga tcatcctctt gtttgaaaca agccctagca 2520

ctctccgaag acaagtttcc tccattgtaa taataaccat tgtacctctc ttgatgttgg 2580

tgcaataagc tcaaatctac tccctgatga tgctgaaccg cagcgttttg ccaaccgcta 2640

gcactattct cactctctga aacgttatta ctgatcatca tcatacttgg aaccggacgg 2700

ttagcctcct tgagacgttt tgcggcgcta ccaataggaa gactagggct ttcgaggatt 2760

gctttaacgt tgtatctgtt catgtcgaag ttagtcactg cggttaatcc tctgaatttg 2820

atggccgcaa tgtcgtatgc ctctgcagct tcttcttgtg tgccaaaagt tcccaagtag 2880

aggtctttgt taccggcgac tcttcctatc ctagcttgcc atcttccatg ttggtgatgt 2940

cttgttactc cacgataaat cgatgcacca cgagagaaac cactactttt cctgcgcagt 3000

gaggcaacat actcttgcct tgtcatgtgc ttcatctctt ctacctcttt ttcatattcg 3060

ctcatgggga agttagtagt agtggtggtt ccccaatact tgagtgcggc taaatcataa 3120

gccctagctg ctttttcttc tttgtcataa cctcccaaat aaacttgtct tcctttgcgc 3180

gtttggcctt ctcttttaca actattatcc cataaatgtg cctcatatct tcctgtccac 3240

cgatgccttg taacaccgcg gtatatagac gtcctctgtc caaaactctc aatagttttc 3300

ttcggtgtag cttcaacgct atcatcaata gtcttccctt gggcaacaac gttgttattg 3360

ctgtcgttgt tgttatcaca agaagtagat gagttcattg agagggacag gccttttgca 3420

gcattgccat tttcttgatt atcaacatta tccacgggtt gatttctcag ccatgtcttt 3480

atcatcgaaa gtcctagtga gccaccacca ccgtctcctc ctccataaca gccacttcca 3540

cttccatctc caacgttttc gttggtgttg taaatcgtgg tggtgcggcc aaggaaattc 3600

tcaagctttg gtccatcttg ctcatcgttg tggatgttat tgcatgcaca accattgatg 3660

tcccaatctc gggagtgact attgttgtct ctggtgaaag catcgacgac gacaccaaag 3720

ggagaaggaa acgatgtttg gatggctgaa gactcatcgg aggcagcggt cggatcgtaa 3780

cagtactctc cggcgacatc tacgacggtt gtggtggtgg aagagtagac gtccttacga 3840

tggtgatttt gttcataagg agagagagaa aagcctaacc agttattatc cattgagcct 3900

gcttttttgt acaaacttgt gatcgggatc tgtaattgta atgttgtttg ttgtttgttg 3960

ttgttggtaa ttgtggatcc tctagagtcc cccgtgtcct ctccaaatga aatgaacttc 4020

cttatataga ggaagggtct tgcgaaggat agtgggattg tgcgtcatcc cttacgtcag 4080

tggagatatc acatcaatcc acttgctttg aagacgtggt tggaacgtct tctttttcca 4140

cgatgctcct cgtgggtggg ggtccatctt tgggaccact gtcggcagag gcatcttcaa 4200

cgatggcctt tcctttatcg caatgatggc atttgtagga gccaccttcc ttttccacta 4260

tcttcacaat aaagtgacag atagctgggc aatggaatcc gaggaggttt ccggatatta 4320

ccctttgttg aaaagtctca attgcccttt ggtcttctga gactgtatct ttgatatttt 4380

tggagtagac aagtgtgtcg tgctccacca tgttgacgaa gattttcttc ttgtcattga 4440

gtcgtaagag actctgtatg aactgttcgc cagtctttac ggcgagttct gttaggtcct 4500

ctatttgaat ctttgactcc atggcctttg attcagtggg aactaccttt ttagagactc 4560

caatctctat tacttgcctc gccggcgggt ctcgggatcc cctgcaggac tagtggattt 4620

gtcttcaagg ccttaagggc cagatcttgg gcccggtacc cgatcagatt gtcgtttccc 4680

gccttcggtt taaactatca gtgtttgaca ggatatattg gcgggtaaac ctaagagaaa 4740

agagcgttta ttagaataat cggatattta aaagggcgtg aaaaggttta tccgttcgtc 4800

catttgtatg tgcatgccaa ccacagggtt cccctcggga gtgcttggca ttccgtgcga 4860

taatgacttc tgttcaacca cccaaacgtc ggaaagcctg acgacggagc agcattccaa 4920

aaagatccct tggctcgtct gggtcggcta gaaggtcgag tgggctgctg tggcttgatc 4980

cctcaacgcg gtcgcggacg tagcgcagcg ccgaaaaatc ctcgatcgca aatccgacgc 5040

tgtcgaaaag cgtgatctgc ttgtcgctct ttcggccgac gtcctggcca gtcatcacgc 5100

gccaaagttc cgtcacagga tgatctggcg cgagttgctg gatctcgcct tcaatccggg 5160

tctgtggcgg gaactccacg aaaatatccg aacgcagcaa gatatcgcgg tgcatctcgg 5220

tcttgcctgg gcagtcgccg ccgacgccgt tgatgtggac gccgaaaagg atctaggtga 5280

agatcctttt tgataatctc atgaccaaaa tcccttaacg tgagttttcg ttccactgag 5340

cgtcagaccc cgtagaaaag atcaaaggat cttcttgaga tccttttttt ctgcgcgtaa 5400

tctgctgctt gcaaacaaaa aaaccaccgc taccagcggt ggtttgtttg ccggatcaag 5460

agctaccaac tctttttccg aaggtaactg gcttcagcag agcgcagata ccaaatactg 5520

ttcttctagt gtagccgtag ttaggccacc acttcaagaa ctctgtagca ccgcctacat 5580

acctcgctct gctaatcctg ttaccagtgg ctgctgccag tggcgataag tcgtgtctta 5640

ccgggttgga ctcaagacga tagttaccgg ataaggcgca gcggtcgggc tgaacggggg 5700

gttcgtgcac acagcccagc ttggagcgaa cgacctacac cgaactgaga tacctacagc 5760

gtgagctatg agaaagcgcc acgcttcccg aagggagaaa ggcggacagg tatccggtaa 5820

gcggcagggt cggaacagga gagcgcacga gggagcttcc agggggaaac gcctggtatc 5880

tttatagtcc tgtcgggttt cgccacctct gacttgagcg tcgatttttg tgatgctcgt 5940

caggggggcg gagcctatgg aaaaacgcca gcaacgcggc ctttttacgg ttcctggcct 6000

tttgctggcc ttttgctcac atgttctttc ctgcgttatc ccctgattct gtggataacc 6060

gattaccgcc tttgagtgag ctgataccgc tcgccgcagc cgaacgaccg agcgcagcga 6120

gtcagtgagc gaggaagcgg aagagcgcct gatgcggtat tttctcctta cgcatctgtg 6180

cggtatttca caccgcatat ggtgcactct cagtacaatc tgctctgatg ccgcatagtt 6240

aagccagtat acactccgct atcgctacgt gactgggtca tggctgcgcc ccgacacccg 6300

ccaacacccg ctgacgcgcc ctgacgggct tgtctgctcc cggcatccgc ttacagacaa 6360

gctgtgaccg tctccgggag ctgcatgtgt cagaggtttt caccgtcatc accgaaacgc 6420

gcgaggcagg ggtacgtcga ggtcgatcca acccctccgc tgctatagtg cagtcggctt 6480

ctgacgttca gtgcagccgt cttctgaaaa cgacatgtcg cacaagtcct aagttacgcg 6540

acaggctgcc gccctgccct tttcctggcg ttttcttgtc gcgtgtttta gtcgcataaa 6600

gtagaatact tgcgactaga accggagaca ttacgccatg aacaagagcg ccgccgctgg 6660

cctgctgggc tatgcccgcg tcagcaccga cgaccaggac ttgaccaacc aacgggccga 6720

actgcacgcg gccggctgca ccaagctgtt ttccgagaag atcaccggca ccaggcgcga 6780

ccgcccggag ctggccagga tgcttgacca cctacgccct ggcgacgttg tgacagtgac 6840

caggctagac cgcctggccc gcagcacccg cgacctactg gacattgccg agcacatcca 6900

ggaggccggc gcgggcctgc gtagcctggc agagccgtgg gccgacacca ccacgccggc 6960

cggccgcatg gtgttgaccg tgttcgccgg cattgccgag ttcgagcgtt ccctaatcat 7020

cgaccgcacc cggagcgggc gcgaggccgc caaggcgcga ggcgtgaagt ttggcccccg 7080

ccctaccctc accccggcac agatcgcgca cgcccgcgag ctgatcgacc aggaaggccg 7140

caccgtgaaa gaggcggctg cactgcttgg cgtgcatcgc tcgaccctgt accgcgcact 7200

tgagcgcagc gaggaagtga cgcccaccga ggccaggcgg cgcggtgcct tccgtgagga 7260

cgcattgacc gaggccgacg ccctggcggc cgccgagaat gaacgccaag aggaacaagc 7320

atgaaaccgc accaggacgg ccaggacgaa ccgtttttca ttaccgaaga gatcgaggcg 7380

gagatgatcg cggccgggta cgtgttcgag ccgcccgcgc acgtctcaac cgtgcggctg 7440

catgaaatcc tggccggttt gtctgatgcc aagctcgcgg cctggccggc gagcttggcc 7500

gctgaagaaa ccgagcgccg ccgtctaaaa aggtgatgtg tatttgagta aaacagcttg 7560

cgtcatgcgg tcgctgcgta tatgatgcga tgagtaaata aacaaatacg caaggggaac 7620

gcatgaaggt tatcgctgta cttaaccaga aaggcgggtc aggcaagacg accatcgcaa 7680

cccatctagc ccgcgccctg caactcgccg gggccgatgt tctgttagtc gattccgatc 7740

cccagggcag tgcccgcgat tgggcggccg tgcgggaaga tcaaccgcta accgttgtcg 7800

gcatcgaccg cccgacgatt gaccgcgacg tgaaggccat cggccggcgc gacttcgtag 7860

tgatcgacgg agcgccccag gcggcggact tggctgtgtc cgcgatcaag gcagccgact 7920

tcgtgctgat tccggtgcag ccaagccctt acgacatatg ggccaccgcc gacctggtgg 7980

agctggttaa gcagcgcatt gaggtcacgg atggaaggct acaagcggcc tttgtcgtgt 8040

cgcgggcgat caaaggcacg cgcatcggcg gtgaggttgc cgaggcgctg gccgggtacg 8100

agctgcccat tcttgagtcc cgtatcacgc agcgcgtgag ctacccaggc actgccgccg 8160

ccggcacaac cgttcttgaa tcagaacccg agggcgacgc tgcccgcgag gtccaggcgc 8220

tggccgctga aattaaatca aaactcattt gagttaatga ggtaaagaga aaatgagcaa 8280

aagcacaaac acgctaagtg ccggccgtcc gagcgcacgc agcagcaagg ctgcaacgtt 8340

ggccagcctg gcagacacgc cagccatgaa gcgggtcaac tttcagttgc cggcggagga 8400

tcacaccaag ctgaagatgt acgcggtacg ccaaggcaag accattaccg agctgctatc 8460

tgaatacatc gcgcagctac cagagtaaat gagcaaatga ataaatgagt agatgaattt 8520

tagcggctaa aggaggcggc atggaaaatc aagaacaacc aggcaccgac gccgtggaat 8580

gccccatgtg tggaggaacg ggcggttggc caggcgtaag cggctgggtt gtctgccggc 8640

cctgcaatgg cactggaacc cccaagcccg aggaatcggc gtgagcggtc gcaaaccatc 8700

cggcccggta caaatcggcg cggcgctggg tgatgacctg gtggagaagt tgaaggcggc 8760

gcaggccgcc cagcggcaac gcatcgaggc agaagcacgc cccggtgaat cgtggcaagc 8820

ggccgctgat cgaatccgca aagaatcccg gcaaccgccg gcagccggtg cgccgtcgat 8880

taggaagccg cccaagggcg acgagcaacc agattttttc gttccgatgc tctatgacgt 8940

gggcacccgc gatagtcgca gcatcatgga cgtggccgtt ttccgtctgt cgaagcgtga 9000

ccgacgagct ggcgaggtga tccgctacga gcttccagac gggcacgtag aggtttccgc 9060

agggccggcc ggcatggcga gtgtgtggga ttacgacctg gtactgatgg cggtttccca 9120

tctaaccgaa tccatgaacc gataccggga agggaaggga gacaagcccg gccgcgtgtt 9180

ccgtccacac gttgcggacg tactcaagtt ctgccggcga gccgatggcg gaaagcagaa 9240

agacgacctg gtagaaacct gcattcggtt aaacaccacg cacgttgcca tgcagcgtac 9300

gaagaaggcc aagaacggcc gcctggtgac ggtatccgag ggtgaagcct tgattagccg 9360

ctacaagatc gtaaagagcg aaaccgggcg gccggagtac atcgagatcg agctagctga 9420

ttggatgtac cgcgagatca cagaaggcaa gaacccggac gtgctgacgg ttcaccccga 9480

ttactttttg atcgatcccg gcatcggccg ttttctctac cgcctggcac gccgcgccgc 9540

aggcaaggca gaagccagat ggttgttcaa gacgatctac gaacgcagtg gcagcgccgg 9600

agagttcaag aagttctgtt tcaccgtgcg caagctgatc gggtcaaatg acctgccgga 9660

gtacgatttg aaggaggagg cggggcaggc tggcccgatc ctagtcatgc gctaccgcaa 9720

cctgatcgaa ggcgaagcat ccgccggttc ctaatgtacg gagcagatgc tagggcaaat 9780

tgccctagca ggggaaaaag gtcgaaaagg tctctttcct gtggatagca cgtacattgg 9840

gaacccaaag ccgtacattg ggaaccggaa cccgtacatt gggaacccaa agccgtacat 9900

tgggaaccgg tcacacatgt aagtgactga tataaaagag aaaaaaggcg atttttccgc 9960

ctaaaactct ttaaaactta ttaaaactct taaaacccgc ctggcctgtg cataactgtc 10020

tggccagcgc acagccgaag agctgcaaaa agcgcctacc cttcggtcgc tgcgctccct 10080

acgccccgcc gcttcgcgtc ggcctatcgc ggccgctggc cgctcaaaaa tggctggcct 10140

acggccaggc aatctaccag ggcgcggaca agccgcgccg tcgccactcg accgccggcg 10200

cccacatcaa ggcaccggtg ggtatgcctg acgatgcgtg gagaccgaaa ccttgcgctc 10260

gttcgccagc caggacagaa atgcctcgac ttcgctgctg cccaaggttg ccgggtgacg 10320

cacaccgtgg aaacggatga aggcacgaac ccagtggaca taagcctgtt cggttcgtaa 10380

gctgtaatgc aagtagcgta tgcgctcacg caactggtcc agaaccttga ccgaacgcag 10440

cggtggtaac ggcgcagtgg cggttttcat ggcttgttat gactgttttt ttggggtaca 10500

gtctatgcct cgggcatcca agcagcaagc gcgttacgcc gtgggtcgat gtttgatgtt 10560

atggagcagc aacgatgtta cgcagcaggg cagtcgccct aaaacaaagt taaacatcat 10620

gagggaagcg gtgatcgccg aagtatcgac tcaactatca gaggtagttg gcgtcatcga 10680

gcgccatctc gaaccgacgt tgctggccgt acatttgtac ggctccgcag tggatggcgg 10740

cctgaagcca cacagtgata ttgatttgct ggttacggtg accgtaaggc ttgatgaaac 10800

aacgcggcga gctttgatca acgacctttt ggaaacttcg gcttcccctg gagagagcga 10860

gattctccgc gctgtagaag tcaccattgt tgtgcacgac gacatcattc cgtggcgtta 10920

tccagctaag cgcgaactgc aatttggaga atggcagcgc aatgacattc ttgcaggtat 10980

cttcgagcca gccacgatcg acattgatct ggctatcttg ctgacaaaag caagagaaca 11040

tagcgttgcc ttggtaggtc cagcggcgga ggaactcttt gatccggttc ctgaacagga 11100

tctatttgag gcgctaaatg aaaccttaac gctatggaac tcgccgcccg actgggctgg 11160

cgatgagcga aatgtagtgc ttacgttgtc ccgcatttgg tacagcgcag taaccggcaa 11220

aatcgcgccg aaggatgtcg ctgccgactg ggcaatggag cgcctgccgg cccagtatca 11280

gcccgtcata cttgaagcta gacaggctta tcttggacaa gaagaagatc gcttggcctc 11340

gcgcgcagat cagttggaag aatttgtcca ctacgtgaaa ggcgagatca ccaaggtagt 11400

cggcaaataa tgtctaacaa ttcgttcaag ccgacgccgc ttcgcggcgc ggcttaactc 11460

aagcgttaga tgcactaagc acataattgc tcacagccaa actatcaggt caagtctgct 11520

tttattattt ttaagcgtgc ataataagcc ctacacaaat tgggagatat atcatgaaag 11580

gctggctttt tcttgttatc gcaatagttg gcgaagtaat cgcaacatag cttgcttggt 11640

cgttccgcgt gaacgtcggc tcgattgtac ctgcgttcaa atactttgcg atcgtgttgc 11700

gcgcctgccc ggtgcgtcgg ctgatctcac ggatcgactg cttctctcgc aacgccatcc 11760

gacggatgat gtttaaaagt cccatgtgga tcactccgtt gccccgtcgc tcaccgtgtt 11820

ggggggaagg tgcacatggc tcagttctca atggaaatta tctgcctaac cggctcagtt 11880

ctgcgtagaa accaacatgc aagctccacc gggtgcaaag cggcagcggc ggcaggatat 11940

attcaattgt aaatggcttc atgtccggga aatctacatg gatcagcaat gagtatgatg 12000

gtcaatatgg agaaaaagaa agagtaatta ccaatttttt ttcaattcaa aaatgtagat 12060

gtccgcagcg ttattataaa atgaaagtac attttgataa aacgacaaat tacgatccgt 12120

cgtatttata ggcgaaagca ataaacaaat tattctaatt cggaaatctt tatttcgacg 12180

tgtctacatt cacgtccaaa tgggggctta gatgagaaac ttcacgatcg gctctagttc 12240

gagcgatcta gtaacataga tgacaccgcg cgcgataatt tatcctagtt tgcgcgctat 12300

attttgtttt ctatcgcgta ttaaatgtat aattgcggga ctctaatcat aaaaacccat 12360

ctcataaata acgtcatgca ttacatgtta attattacat gcttaacgta attcaacaga 12420

aattatatga taatcatcgc aagaccggca acaggattca atcttaagaa actttattgc 12480

caaatgtttg aacgatcggg gaaattcgtt tctatgcttc aattctgctc tatagatgtt 12540

cttctagtct taacttttag aaggcacaac atttgaaatg ttggtaattt ttctgctggg 12600

tagtgaacca ttgtaatttg tgtttgctta tattatccgt tgggtttctc tatgttatct 12660

gtggggccat atcccagaac tggttgagtc ggtccaacac ctgggtgcca atcatgtcga 12720

tggtggggta tggt 12734

<210> 16
<211> 5472

<212>  DNA
<213>  Artificial Sequence

<220>
<223>  IPR 252-pK-02

<400>  16
cggacgaccc tgcgcgctca gacgaaacac cgccgcatca gagcagccaa tggtctgctg      60

cgcccaatca tagccaaaca gacgttccac ccacgctgcc gggctacccg catgcaggcc     120

atcctgttca atcatactct tcctttttca atattattga agcatttatc agggttattg     180

tctcatgagc ggatacatat ttgaatgtat ttagaaaaat aaacaaatag gggttccgcg     240

cacatttccc cgaaaagtgc cacctaaatt gtaagcgtta atattttgtt aaaattcgcg     300

ttaaattttt gttaaatcag ctcatttttt aaccaatagg ccgaaatcgg caaaatccct     360

tataaatcaa aagaatagac cgagataggg ttgagtggcc gctacagggc gctcccattc     420

gccattcagg ctgcgcaact gttgggaagg gcgtttcggt gcgggcctct tcgctattac     480

gccagctggc acgacaggtt tcccgactgg aaagcgggca gtgagcgcaa cgcaattaat     540

gtgagttagc tcactcatta ggcaccccag gctttacact ttatgcttcc ggctcgtatg     600

ttgtgtggaa ttgtgagcgg ataacaattt cacacaggaa acagctatga ccatgattac     660

gccaagctcg aaattaaccc tcactaaagg gaacaaaagc tggactagag gcccttaagg     720

ccttgaagac aaatccacta gtcctgcagg ggatcccgag acccgccggc gaggcaagta     780

atagagattg gagtctctaa aaaggtagtt cccactgaat caaaggccat ggagtcaaag     840

attcaaatag aggacctaac agaactcgcc gtaaagactg gcgaacagtt catacagagt     900

ctcttacgac tcaatgacaa gaagaaaatc ttcgtcaaca tggtggagca cgacacactt     960

gtctactcca aaaatatcaa agatacagtc tcagaagacc aaagggcaat tgagactttt    1020

caacaaaggg taatatccgg aaacctcctc ggattccatt gcccagctat ctgtcacttt    1080

attgtgaaga tagtggaaaa ggaaggtggc tcctacaaat gccatcattg cgataaagga    1140

aaggccatcg ttgaagatgc ctctgccgac agtggtccca aagatggacc cccacccacg    1200

aggagcatcg tggaaaaaga agacgttcca accacgtctt caaagcaagt ggattgatgt    1260

gatatctcca ctgacgtaag ggatgacgca caatcccact atccttcgca agacccttcc    1320

tctatataag gaagttcatt tcatttggag aggacacggg ggactctaga ggatccacaa    1380

ttaccaacaa caacaaacaa caaacaacat tacaattaca gatcccgatc acaagtttgt    1440

acaaaaaagc aggctcaatg gataataact ggttaggctt ttctctctct ccttatgaac    1500

aaaatcacca tcgtaaggac gtctactctt ccaccaccac aaccgtcgta gatgtcgccg    1560

gagagtactg ttacgatccg accgctgcct ccgatgagtc ttcagccatc caaacatcgt    1620

ttccttctcc ctttggtgtc gtcgtcgatg ctttcaccag agacaacaat agtcactccc    1680

gagattggga catcaatggt tgtgcatgca ataacatcca caacgatgag caagatggac 1740

caaagcttga gaatttcctt ggccgcacca ccacgattta caacaccaac gaaaacgttg 1800

gagatggaag tggaagtggc tgttatggag gaggagacgg tggtggtggc tcactaggac 1860

tttcgatgat aaagacatgg ctgagaaatc aacccgtgga taatgttgat aatcaagaaa 1920

atggcaatgc tgcaaaaggc ctgtccctct caatgaactc atctacttct tgtgataaca 1980

acaacgacag caataacaac gttgttgccc aagggaagac tattgatgat agcgttgaag 2040

ctacaccgaa gaaaactatt gagagttttg gacagaggac gtctatatac cgcggtgtta 2100

caaggcatcg gtggacagga agatatgagg cacatttatg ggataatagt tgtaaaagag 2160

aaggccaaac gcgcaaagga agacaagttt atttgggagg ttatgacaaa gaagaaaaag 2220

cagctagggc ttatgattta gccgcactca agtattgggg aaccaccact actactaact 2280

tccccatgag cgaatatgaa aaagaggtag aagagatgaa gcacatgaca aggcaagagt 2340

atgttgcctc actgcgcagg aaaagtagtg gtttctctcg tggtgcatcg atttatcgtg 2400

gagtaacaag acatcaccaa catggaagat ggcaagctag gataggaaga gtcgccggta 2460

acaaagacct ctacttggga acttttggca cacaagaaga agctgcagag gcatacgaca 2520

ttgcggccat caaattcaga ggattaaccg cagtgactaa cttcgacatg aacagataca 2580

acgttaaagc aatcctcgaa agccctagtc ttcctattgg tagcgccgca aaacgtctca 2640

aggaggctaa ccgtccggtt ccaagtatga tgatgatcag taataacgtt tcagagagtg 2700

agaatagtgc tagcggttgg caaaacgctg cggttcagca tcatcaggga gtagatttga 2760

gcttattgca ccaacatcaa gagaggtaca atggttatta ttacaatgga ggaaacttgt 2820

cttcggagag tgctagggct tgtttcaaac aagaggatga tcaacaccat ttcttgagca 2880

acacgcagag cctcatgact aatatcgatc atcaaagttc tgtttcggat gattcggtta 2940

ctgtttgtgg aaatgttgtt ggttatggtg gttatcaagg atttgcagcc ccggttaact 3000

gcgatgccta cgctgctagt gagtttgatt ataacgcaag aaaccattat tactttgctc 3060

agcagcagca gacccagcag tcgccaggtg gagatttccc cgcggcaatg acgaataatg 3120

ttggctctaa tatgtattac catggggaag gtggtggaga agttgctcca acatttacag 3180

tttggaacga caattagaaa aaatagttaa agatctttag ttatatgcgt tgttgtgtgc 3240

tggtgaacag tgtgatactt tgattatgtt tttttctttc tctttttctt tttcttggtt 3300

aatttcttaa gacttatttt tagtttccat tagttggata aattttcaga ctaaaaaaaa 3360

aaaaaaaaaa aaactcgagg gggggcccgg taccgagctc gaatttcccc gatcgttcaa 3420

acatttgtac ccagctttct tgtacaaagt ggtgatgtaa ctaattgagg gggtaccgtc 3480

gacgagctca tatgaagatg aagatgaaat atttggtgtg tcaaataaaa agcttgtgtg 3540

cttaagtttg tgtttttttc ttggcttgtt gtgttatgaa tttgtggctt tttctaatat 3600

taaatgaatg taagatctca ttataatgaa taaacaaatg tttctataat ccattgtgaa 3660

tgttttgttg gatctcttct gccgccggcg ggtctccaga tccaatgcat tggtcctgca 3720

ggccccgggc tatctttgtc ttccggccgc catggccaga tcgtacccaa ttcgccctat 3780

agtgagtcgt attacaattc actggccgtc gttttacaac gtcgtgactg ggaaaaccct 3840

ggcgttaccc aacttaatcg ccttgcagca catccccctt tcgccagctg cattaacatg 3900

gtcatagctg tttccttgcg tattgggcgc tctccgcttc ctcgctcact gactcgctgc 3960

gctcggtcgt tcgggtaaag cctggggtgc ctaatgagca aaaggccagc aaaaggccag 4020

gaaccgtaaa aaggccgcgt tgctggcgtt tttccatagg ctccgccccc ctgacgagca 4080

tcacaaaaat cgacgctcaa gtcagaggtg gcgaaacccg acaggactat aaagatacca 4140

ggcgtttccc cctggaagct ccctcgtgcg ctctcctgtt ccgaccctgc cgcttaccgg 4200

atacctgtcc gcctttctcc cttcgggaag cgtggcgctt tctcatagct cacgctgtag 4260

gtatctcagt tcggtgtagg tcgttcgctc caagctgggc tgtgtgcacg aaccccccgt 4320

tcagcccgac cgctgcgcct tatccggtaa ctatcgtctt gagtccaacc cggtaagaca 4380

cgacttatcg ccactggcag cagccactgg taacaggatt agcagagcga ggtatgtagg 4440

cggtgctaca gagttcttga agtggtggcc taactacggc tacactagaa gaacagtatt 4500

tggtatctgc gctctgctga agccagttac cttcggaaaa agagttggta gctcttgatc 4560

cggcaaacaa accaccgctg gtagcggtgg tttttttgtt tgcaagcagc agattacgcg 4620

cagaaaaaaa ggatctcaag aagatccttt gatcttttct acggggtctg acgctcagtg 4680

gaacgaaaac tcacgttaag ggattttggt catgagatta tcaaaaagga tcttcaccta 4740

gatcctttta aattaaaaat gaagttttaa atcaatctaa agtatatatg agtaaacttg 4800

gtctgacagt tattagaaaa attcatccag cagacgataa aacgcaatac gctggctatc 4860

cggtgccgca atgccataca gcaccagaaa acgatccgcc cattcgccgc ccagttcttc 4920

cgcaatatca cgggtggcca gcgcaatatc ctgataacga tccgccacgc ccagacggcc 4980

gcaatcaata aagccgctaa aacggccatt ttccaccata atgttcggca ggcacgcatc 5040

accatgggtc accaccagat cttcgccatc cggcatgctc gctttcagac gcgcaaacag 5100

ctctgccggt gccaggccct gatgttcttc atccagatca tcctgatcca ccaggcccgc 5160

ttccatacgg gtacgcgcac gttcaatacg atgtttcgcc tgatgatcaa acggacaggt 5220

cgccgggtcc agggtatgca gacgacgcat ggcatccgcc ataatgctca ctttttctgc 5280

cggcgccaga tggctagaca gcagatcctg acccggcact tcgcccagca gcagccaatc 5340

acggcccgct tcggtcacca catccagcac cgccgcacac ggaacaccgg tggtggccag 5400

ccagctcaga cgcgccgctt catcctgcag ctcgttcagc gcaccgctca gatcggtttt 5460

cacaaacagc ac 5472